(19) Europäisches Patentamt / European Patent Office / Office européen des brevets

(11) **EP 1 899 914 B1**

(12) **FASCICULE DE BREVET EUROPEEN**

(45) Date de publication et mention
de la délivrance du brevet:
**30.11.2016 Bulletin 2016/48**

(51) Int Cl.:
**G06T 5/00** (2006.01)

(21) Numéro de dépôt: **06755496.4**

(86) Numéro de dépôt international:
**PCT/FR2006/001118**

(22) Date de dépôt: **18.05.2006**

(87) Numéro de publication internationale:
**WO 2006/128990 (07.12.2006 Gazette 2006/49)**

(54) **MÉTHODE DE SEGMENTATION D'UNE SEQUENCE D'IMAGES TRIDIMENSIONELLES, NOTAMMENT EN PHARMACO-IMAGERIE**

VERFAHREN ZUR SEGMENTIERUNG EINER SEQUENZ VON DREIDIMENSIONALEN BILDERN, INSBESONDERE IN PHARMACO-ABBILDUNGEN

METHOD FOR SEGMENTING A SEQUENCE OF THREE-DIMENSIONAL IMAGES, IN PARTICULAR IN PHARMACO-IMAGING

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.05.2005 FR 0505441**

(43) Date de publication de la demande:
**19.03.2008 Bulletin 2008/12**

(73) Titulaire: **Commissariat à l'Énergie Atomique et aux Énergies Alternatives 75015 Paris (FR)**

(72) Inventeurs:
- **MAROY, Renaud**
  **91360 Villemoisson Sur Orge (FR)**
- **TAVITIAN, Bertrand**
  **75015 Paris (FR)**
- **FROUIN, Vincent**
  **75015 Paris (FR)**

(74) Mandataire: **Gevers & Orès**
  **41 avenue de Friedland**
  **75008 Paris (FR)**

(56) Documents cités:
- **GUO H. ET A.: "Clustering Huge Data Sets for Parametric PET Imaging" BIOSYSTEMS, vol. 71, 2003, pages 81-92, XP002346999 cité dans la demande**
- **KIMURA Y., SENDA M., ALPERT N.M.: "Fast Formation of Statistically reliable FDG Parametric Images Based on Clustering and Principal Components" PHYSICS IN MEDICINE AND BIOLOGY, vol. 47, 2002, pages 455-468, XP002347000 cité dans la demande**
- **WONG K.-P. ET AL.: "Segmentation of Dynamic PET Images Using Cluster Analysis" IEEE TRANS. ON NUCLEAR SCIENCE, vol. 49, no. 1, février 2002 (2002-02), pages 200-207, XP002347001 cité dans la demande**
- **O'SULLIVAN F.: "Imaging Radiotracer Model Parameters in PET: a Mixture Analysis Approach" IEEE TRANS. ON MEDICAL IMAGING, vol. 12, no. 3, septembre 1993 (1993-09), pages 399-412, XP002347002**
- **BRANKOV J.G. ET AL.: "Segmentation of Dynamic PET or fMRI Images Based on Similarity Metric" IEEE TRANS. ON NUCLEAR SCIENCE, vol. 50, no. 5, octobre 2003 (2003-10), pages 1410-1414, XP002347003**
- **FROUIN F. ET AL.: "GLOBAL STRATEGY TO EXTRACT AUTOMATICALLY RELEVANT SUBDOMINANT PERFUSION INFORMATION: APPLICATION TO SKELETAL MUSCLE NMR IMAGING WITH ARTERIAL SPIN LABELING" BIOMEDICAL IMAGING, 2002. PROCEEDINGS. 2002 IEEE INTERNATIONAL SYMPOSIUM ON, 7 juillet 2002 (2002-07-07), - 10 juillet 2002 (2002-07-10) pages 569-572, XP002347004**

**Description**

La présente invention concerne une méthode de segmentation d'une image ou d'une séquence d'images tridimensionnelles en une image tridimensionnelle partitionnée en régions d'intérêt. L'invention s'applique d'une manière générale à tous types d'images, en particulier dans les domaines de la pharmaco-imagerie et de l'imagerie satellite. Elle s'applique également aux images bidimensionnelles, qui sont des cas particuliers d'images tridimensionnelles dans lesquelles la valeur d'une des dimensions est égale à l'unité.

Une évolution récente et remarquable dans le développement des médicaments est l'utilisation de la pharmaco-imagerie pour mesurer les paramètres pharmacocinétiques. De manière connue, la pharmacocinétique est la composante de la pharmacologie qui décrit la disposition des médicaments dans l'organisme, et précise de façon qualitative et quantitative les processus d'absorption, de distribution, de métabolisation et d'élimination d'un principe actif.

Le grand avantage de la pharmaco-imagerie par rapport aux techniques classiques, qui nécessitent le prélèvement répété d'échantillons biologiques, est sa capacité à mesurer la concentration du principe actif dans les organes du patient humain ou animal vivant. On évite ainsi de sacrifier des lots d'animaux, et l'on diminue de façon substantielle la dispersion des valeurs - toutes les mesures de la cinétique étant faites chez le même animal. De plus, on peut analyser la cinétique dans les organes profonds chez l'homme.

En contrepartie, la pharmaco-imagerie requiert une mise en oeuvre qui peut être technologiquement lourde : d'abord le marquage, radioactif ou autre, du principe actif à suivre ; ensuite, la preuve que la détection par imagerie donne bien une mesure quantitative de la concentration du principe actif ; enfin, le repérage de la localisation anatomique du signal lié au principe actif marqué.

D'une façon générale, mais non exclusive, une technique préférée de la pharmaco-imagerie est la tomographie d'émission de positons (TEP) qui, du fait du principe de détection sur lequel elle s'appuie, est parmi les techniques d'imagerie les mieux adaptées aux mesures quantitatives de concentrations moléculaires dans les organes de l'homme ou de l'animal.

En tout état de cause, la mesure de la concentration d'un principe actif marqué (appelé ci-après traceur) en un point de l'organisme étudié n'est interprétable sur le plan pharmacocinétique que si l'on peut assigner ce point à un organe défini, soit sur une base anatomique, soit sur une base physiologique : le coeur, le foie, le rein, une tumeur, une région du cerveau, etc. Or, l'image est uniquement représentative de la localisation de l'indicateur (émetteur de positons radioactif dans le cas de la TEP) lié au principe actif étudié, et ne contient a *priori* aucune information anatomique ou physiologique, mais uniquement une information pharmacocinétique. Il est donc nécessaire de superposer les images successives de localisation du traceur à une ou des images renseignant sur l'anatomie et/ou la physiologie. Cette superposition ou recalage peut se faire de plusieurs manières :

- Dans le cas le plus favorable et le moins fréquent, la distribution du traceur dévoile une anatomie reconnaissable. C'est le cas des traceurs se concentrant dans l'ensemble d'un système anatomique remarquable comme le squelette, qui ne nécessitent pas de recalage supplémentaire, toute l'information étant dans l'image. Inversement, c'est aussi le cas de certains traceurs qui diffusent très bien dans tous les organes (par exemple le fluorodésoxyglucose). Dans ce dernier cas, les organes sont délimités sur les images par un contraste dépendant du niveau de rétention du traceur, ce qui n'est pas forcément suffisant pour leur identification, par exemple dans le cas de deux organes accolés ayant le même niveau de rétention du traceur. D'une façon générale, ce cas où le recalage est peu ou pas utile ne concerne que les traceurs « généralistes » (ions, précurseurs métaboliques, etc.) et pas l'imagerie des principes actifs.
- Dans de nombreux autres cas, les organes sont repérés par « plaquage » d'une image anatomique issue des connaissances de l'examinateur sur les pharmaco-images. Un biologiste peut souvent deviner la localisation des organes anatomiques, tels que le foie ou le coeur, sur les images dont l'échelle de contraste suit la dynamique de concentration du traceur. La précision de la localisation obtenue dépend alors de la capacité à identifier l'organe, donc de la distribution du traceur qui délimite plus ou moins bien les contours d'un organe, et de l'habileté ou de l'habitude de l'examinateur. On comprend que cette méthode sans image anatomique est inapplicable au cas d'un traceur qui ne serait présent qu'en un point.

- De plus en plus souvent, l'examinateur fait appel à une deuxième image qu'il superpose à la pharmaco-image, via un deuxième mode d'imagerie dans lequel le contraste n'est pas fondé sur la localisation du traceur mais sur l'anatomie (par exemple la tomodensitométrie, ou l'imagerie par résonance magnétique ou IRM) qui est appliquée au même individu, de préférence lors de la même session d'imagerie. En superposant les deux images, on recale la pharmaco-image sur une image anatomique qui identifie les organes. Cette double modalité est de plus en plus largement utilisée pour l'imagerie clinique avec l'avènement récent du système « PET-CT » associant caméra TEP et tomodensitomètre X. Toutefois, la double modalité n'est pas totalement satisfaisante pour la pharmaco-imagerie pour des raisons à la fois fondamentales et pratiques :

- Sur le plan fondamental, l'image anatomique ne fait que plaquer une information statique sur l'information pharmaco-cinétique donnée par la pharmaco-image. Le lien, autre que le sujet, entre l'image anatomique et la pharmaco-image, ne peut donc être établi que si la localisation pharmacocinétique du traceur suit exactement les contours des organes décrits par l'image anatomique. Par conséquent, l'information supplémentaire apportée par l'image anatomique est limitée à la résolution de la méthode d'imagerie anatomique utilisée, qui est loin d'être toujours suffisante pour identifier la nature de tous les pharmaco-organes. Par exemple, la tomodensitométrie apporte peu de contraste dans les tissus mous et le cerveau, l'IRM distingue mal les masses pulmonaires, etc.
- Sur le plan pratique :

    - Les caméras couplées de type « PET-CT » ne sont actuellement disponibles que pour l'homme, donc avec une grande ouverture et une résolution moindre que les caméras TEP dédiées aux petits animaux. Ces caméras sont nécessairement plus chères que les caméras TEP seules.
    - Dans le cas d'une imagerie anatomique par tomodensitométrie, la dose d'irradiation nécessaire pour obtenir une image chez le petit rongeur est loin d'être négligeable. Ceci pose des problèmes de toxicité et/ou d'interprétation de résultats dans le cas de l'imagerie des tumeurs.
    - La qualité du recalage obtenue est tributaire de l'immobilité totale du sujet pendant toute la durée de la pharmacocinétique explorée, ce qui n'est pas le cas lors du transport d'un animal d'une caméra à une autre.

Par ailleurs, l'interprétation pharmacocinétique des images TEP nécessite généralement la délimitation de régions d'intérêt (« ROI ») représentatives des organes. Kimura et coll. [Kimura, 2002] et Zhou [Zhou, 2000] ont montré qu'un regroupement adéquat des éléments de volume (voxels) améliore la qualité de la quantification pharmacocinétique. En effet, le tracé des régions d'intérêt suppose que chaque organe ou partie d'organe présente un comportement homogène pour un traceur donné, qui peut être caractérisé par une cinétique physiologique. Par conséquent, quelle que soit la méthode de segmentation, les limites des organes tracés doivent être visibles pour éviter que les régions d'intérêt n'englobent deux organes ou parties d'organes aux fonctions différentes (appelés pharmaco-organes ci-après). La qualité de la segmentation de l'image en régions d'intérêt cohérentes sur le plan pharmacocinétique est donc cruciale. Or, les régions d'intérêt sont en général tracées manuellement, ce qui est un travail long, fastidieux, dépendant de l'opérateur et nécessitant un degré d'expertise certain.

Un certain nombre de travaux, dont le but n'était pas limité à l'analyse pharmacocinétique d'images, ont proposé des méthodes de segmentation automatique permettant de s'affranchir de l'opérateur pour la segmentation des régions d'intérêt. Les images TEP souffrent d'un faible rapport signal sur bruit. Comme elles souffrent également d'une faible résolution spatiale, l'activité mesurée en un élément de volume donné de l'image est polluée par l'activité contenue dans les éléments de volumes voisins. En particulier, la mesure de la concentration du traceur dans les organes de petite taille peut être soit sous-estimée, soit surestimée selon que la concentration radioactive dans les structures environnantes est moins élevée ou plus élevée. Cet effet, dit de « volume partiel » peut en effet être modélisé comme un lissage de l'image [Frouin, 2002] et rend donc les contours des organes intrinsèquement flous. En conséquence, les parties des organes proches des frontières de ces derniers ne contiendront pas la cinétique d'un unique pharmaco-organe, mais une combinaison linéaire des cinétiques de tous les pharmaco-organes proches. Ainsi, la cinétique de pharmaco-organe la plus représentée au sein d'un élément de volume n'est pas forcément celle du ou des pharmaco-organe(s) réellement contenu(s) dans cet élément de volume.

En outre, les images TEP dynamiques représentent de manière connue une grande masse de donnée (un demi-million de cinétiques), dont le traitement est prohibitif en termes de temps de calcul et de mémoire informatique requise [Guo, 2003]. Dans les images, l'organisme étudié représente entre 20% et 40% des données. La partie restante, extérieure à l'organisme, contient principalement du bruit.

On sait par ailleurs que la cinétique au sein d'un élément de volume (ce terme désigne l'unité volumique élémentaire de l'image) n'a de sens qu'à condition que le pharmaco-organe étudié soit parfaitement immobile au cours du temps d'acquisition de la séquence d'images. Tout mouvement du pharmaco-organe imagé brise le lien entre la cinétique d'un élément de volume et les cinétiques de ce pharmaco-organe. Les mouvements physiologiques, extrêmement difficiles à corriger, sont de deux sortes.

Certains mouvements sont périodiques, de période en général inférieure à la durée d'acquisition d'une image de la séquence. Les battements cardiaques et la respiration imposent un déplacement des organes voisins, générant un effet de flou non négligeable, mais à peu près constant d'une acquisition à la suivante.

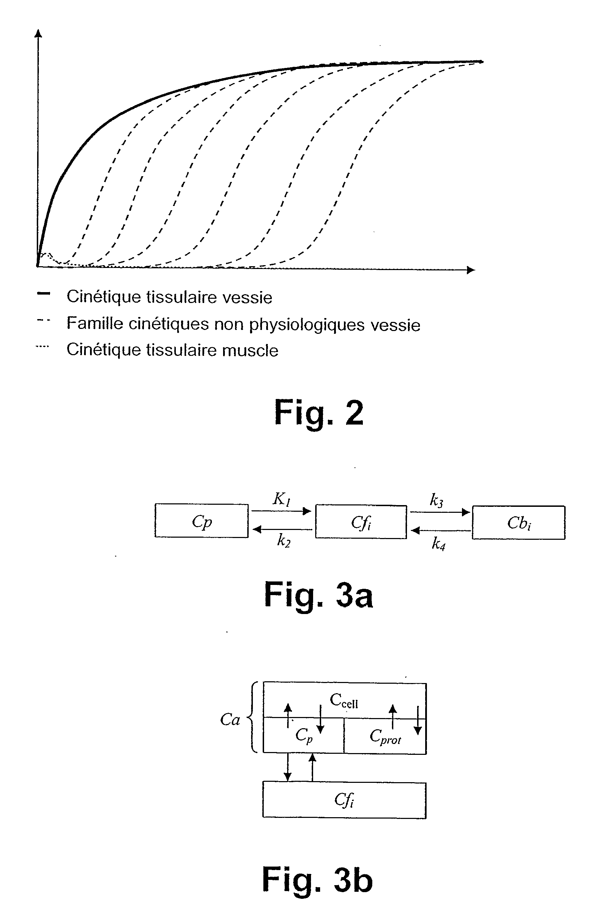

D'autres mouvements non périodiques sont imprévisibles, comme le déplacement des viscères lors de la digestion et sous l'effet de la respiration, ou le remplissage de la vessie au cours de l'examen : cette dernière peut voir son volume apparent décupler entre le début et la fin de l'examen. Pour les traceurs excrétés dans les urines, la concentration du traceur et des métabolites dans les urines devient très élevée. Un voxel correspondant à une région viscérale ou musculaire en début d'examen peut donc contenir une cinétique caractéristique de la vessie en fin d'examen. Comme le montre la figure 2 jointe, le remplissage de la vessie génère une famille de cinétiques composées pour les premiers temps d'acquisition de la cinétique d'un pharmaco-organe proche de la vessie, et pour les derniers temps de la cinétique de la vessie. L'hypothèse d'un nombre fixe de cinétiques contenues dans une image TEP, au bruit et à l'effet de volume partiel près, ne s'applique donc pas dans le cas d'un organisme imagé sujet à des mouvements physiologiques non périodiques.

Enfin, il est connu que le bruit dans les images TEP diffère selon les méthodes de reconstruction d'images utilisées.

Au sein d'une image issue d'une reconstruction analytique par rétroprojection filtrée, le bruit est souvent considéré comme additif gaussien stationnaire dans l'image, non corrélé avec le signal. En revanche, le bruit d'une image de la séquence dépend de la durée d'acquisition de cette image. Cette dépendance peut être considérée comme linéaire [Guo, 2003] par rapport à l'inverse de la durée d'acquisition de l'image.

Dans les images issues de méthodes statistiques itératives, comme « OSEM » (« Ordered Subset Expectation Maximization », méthode reconstruisant l'image en maximisant la vraisemblance de sa projection selon diverse incidences) ou « AWOSEM » (« Attenuation Weighted Ordered Subset Expectation Maximization », méthode opérant de façon similaire à « OSEM » en prenant en compte le phénomène d'atténuation des photons par l'organisme), on ne peut plus supposer un bruit stationnaire dans l'espace. Le bruit dépend du nombre d'itérations utilisées pour la reconstruction, phénomène stationnaire, mais est aussi corrélé au signal. On peut écrire : $\sigma^2 = \alpha^2 \times S$, où $\sigma$ représente la variance locale du bruit à un instant donné, $\alpha$ une constante indépendante de la position et du temps, mais dépendant de la méthode de reconstruction utilisée ainsi que du nombre d'itérations choisi, et S le signal au point considéré. Dans ce cas de figure, le rapport signal sur bruit s'exprime comme $S/\sigma = \alpha \times S^{1/2}$. La séparation du bruit et du signal en un point quelconque de l'image permet le calcul de $\alpha$ et permet de caractériser finement le bruit. Comme dans le cas d'une reconstruction par méthode analytique, il conviendra de corriger les données de l'influence de la durée d'acquisition de chaque image de la séquence.

Plusieurs méthodes de segmentation des images TEP en agrégats regroupant des zones à cinétiques homogènes, sans connaissance anatomique a *priori,* ont récemment été proposées pour tenter de résoudre les problèmes précités.

Ashburner [Ashburner, 1996] suppose qu'une image TEP contient un nombre connu de cinétiques - une par agrégat -, et décrit donc toute cinétique d'un voxel de l'image comme la cinétique d'un agrégat, multipliée par un facteur d'échelle, à laquelle s'ajoute un bruit gaussien multinormal - de loi normale pour chaque image acquise - identique pour chaque agrégat.

Wong et coll. [Wong, 2002] supposent les cinétiques homogènes au sein d'un même agrégat, mais dissimilaires entre agrégats différents. Ils proposent une approche d'agrégation par la méthode des k-moyennes, qui maximise la variance des cinétiques entre les classes, tout en minimisant la variance des cinétiques au sein d'une même classe. La cinétique d'un agrégat est alors estimée comme la moyenne des cinétiques des individus le composant.

Frouin F. et coll. [Frouin, 2001] utilisent aussi la méthode des k-moyennes afin de réaliser une segmentation du coeur sur des images de perfusion. Cependant, l'agrégation ne s'opère pas sur les cinétiques elles-mêmes, mais sur des facteurs extraits des cinétiques par analyse factorielle, assurant une meilleure robustesse de la segmentation. Cependant, l'interprétation des facteurs d'une analyse factorielle devient difficile au-delà de 4 ou 5 facteurs, ce qui exclut de l'utiliser directement sur un corps entier. On notera que la méthode décrite par Frouin F. et coll ne segmente pas un organisme en pharmaco-organes, mais un pharmaco-organe en zones de prééminence de facteurs tels que les cinétiques artérielle, veineuse et tissulaire.

Acton [Acton, 1999] utilise la méthode des c-moyennes floues, proche des k-moyennes, mais permettant de mieux prendre en compte la nature intrinsèquement floue des données acquises en tomographie par simples photons, tout en assurant une meilleure robustesse.

Kimura et coll. [Kimura, 2002] proposent une méthode d'agrégation sur les projections des cinétiques sur les vecteurs propres associés aux deux plus grandes valeurs propres d'une analyse en composantes principales opérée sur l'ensemble des cinétiques de l'organisme, afin d'extraire des paramètres de modélisation compartimentale.

Brankov et coll. [Brankov, 2003] proposent d'utiliser une mesure de similarité définie comme le cosinus de l'angle formé entre deux vecteurs représentés dans l'espace des cinétiques, plutôt qu'une norme euclidienne ou une norme de variance totale. Cette mesure de similarité présente une forte sensibilité au bruit dans les zones de faible rapport signal sur bruit. Brankov et coll. présentent deux algorithmes de type attente-maximisation (« EM » en anglais), l'un flou et l'autre binaire, qui déterminent des agrégats à l'intérieur desquels les individus - cinétiques - présentent une forte similarité. La méthodologie « EM » permet d'estimer de façon itérative une variable cachée dont l'image est une réalisation particulière conformément à un modèle choisi. Chaque itération comprend une première phase d'attente où l'on calcule la vraisemblance attendue des données complètes à partir de la distribution jointe des données observées et cachées, et une seconde phase de maximisation où l'on estime les paramètres du modèle qui maximise cette vraisemblance du modèle attendue. Le processus est répété jusqu'à convergence de l'algorithme.

Brankov compare sa méthode notamment à une application des mélanges d'analyseurs en composantes principales probabilistes. Cette méthode, introduite par Tipping et Bishop [Tipping, 1999], modélise le signal au sein de la zone à segmenter par un mélange de projections sur des sous-espaces de l'espace des cinétiques.

Un inconvénient majeur de toutes ces méthodes est qu'elles sont initialisées aléatoirement. A chaque lancement, l'algorithme converge vers l'un de ses minima locaux. Or, la solution recherchée correspond *a priori* au minimum global de cette énergie. Plusieurs lancements du programme avec des initialisations à chaque fois différentes sont donc nécessaires pour approcher de la meilleure solution.

Guo [Guo, 2003] propose une agrégation par classification ascendante hiérarchique permettant d'obtenir un nombre d'agrégats défini a *posteriori,* mais aussi de conserver les agrégats de petite taille. Pour le calcul de la distance entre cinétiques, la valeur aux éléments de volume considérés est pondérée à chaque instant de la cinétique par le rapport signal sur bruit attendu pour l'image correspondante. Ce rapport, pour une image donnée de la séquence, est supposé dépendre essentiellement de la durée d'acquisition de cette image. L'approche hiérarchique assure la convergence de l'algorithme vers un minimum optimal, mais au prix d'un temps de calcul ne permettant pas d'examiner tout le volume de données. De plus, toute fusion de voxels obtenue par cet algorithme est définitive. En conséquence, une affectation erronée d'un voxel lors des premières itérations, par exemple du fait du bruit, ne peut être corrigée lors des itérations successives.

Guo et coll. [Guo, 2003] proposent une pré-segmentation par histogramme, afin d'obtenir des premiers regroupements accélérant une classification ascendante hiérarchique. Cette dernière opère par une succession de fusions d'individus dans un ordre optimal selon un critère choisi. Les deux individus - typiquement des voxels de l'image - les plus proches en terme d'une distance choisie sont agrégés, puis les agrégats ou individus les plus proches sont de nouveaux agrégés, et ainsi de suite jusqu'à ce qu'un critère d'arrêt soit vérifié ou bien qu'il n'existe plus qu'un unique agrégat regroupant tous les individus. L'histogramme utilisé par Guo et coll. peut être décrit comme une comptabilisation du nombre de voxels ayant une intensité donnée. Cette classification par histogramme se base sur les valeurs de la dernière image au sens temporel de la série acquise après administration du traceur. On suppose que les premières fusions ont peu d'incidence sur les agrégats finaux et on regroupe les voxels correspondant au même intervalle de l'histogramme de la dernière image de la séquence. La variation de la distribution spatiale est supposée minimale pour cette dernière image parmi toutes celles contenues dans l'intervalle de temps considéré, le traceur ayant eu le maximum de temps pour se distribuer dans les organes suivant son affinité. Toutefois, dans le cas des isotopes à courte période radioactive utilisés en TEP, du fait de la décroissance exponentielle de la radioactivité au cours du temps, ceci entraîne l'inconvénient d'un bruit accru du fait que la dernière série présente aussi le plus faible rapport signal sur bruit de toutes les images de la série.

Parmi toutes ces méthodes, seule celle de F. Frouin est validée sur des organes en mouvement de type mouvements périodiques de faible période. Cependant, de par son principe, elle n'est applicable qu'à des zones de l'organisme comprenant très peu de pharmaco-organes. Toutes les autres méthodes précitées n'ont pas été validées dans le cas d'une problématique corps entier, et ne sont pas adaptées aux mouvements physiologiques propres à cette problématique.

Le document de Brevet EP-A-1 365 356 présente une méthode de segmentation semi-automatique d'images acquises par TEP, qui requiert notamment la délinéation préalable d'une région d'intérêt et de voxels-modèles à extraire dans les images. On notera que la méthode présentée dans ce dernier document est limitée au domaine de l'oncologie et qu'elle ne permet pas de segmenter les images en autant de régions d'intérêt que de pharmaco-organes, mais seulement en deux parties dont une seule contient une tumeur.

Un but de la présente invention est de proposer une méthode de segmentation d'une image ou d'une séquence d'images tridimensionnelles de départ à base de voxels pour l'obtention d'une image tridimensionnelle de segmentation comportant une partition en régions d'intérêt, ladite image ou séquence d'images comprenant des mesures, pour chaque voxel et au cours de n intervalles de temps ($n \geq 1$), de l'évolution réelle d'un signal représentatif d'au moins une variable de type physique, chimique ou biologique de ladite image ou séquence, qui permette de remédier à ces inconvénients.

A cet effet, la méthode de segmentation selon l'invention comprend essentiellement les étapes suivantes :

a) une modélisation du signal comprenant la définition d'un modèle paramétrique d'évolution spatio-temporelle dudit signal, ce modèle comprenant des jeux de paramètres de sorte que lesdits jeux soient respectivement propres à des structures correspondant auxdites régions d'intérêts et que chaque jeu de paramètres soit indépendant des coordonnées spatiales dans la structure correspondante (ces paramètres seront dits « homogènes » ci-après) ;
b) une extraction d'échantillons de voxels de sorte que lesdits échantillons soient respectivement inclus dans lesdites structures, cette extraction comprenant :

(i) un calcul, pour chaque voxel de ladite image ou séquence d'images de départ ou bien d'une zone d'intérêt de celle-ci, d'un critère de validité d'une hypothèse selon laquelle ledit modèle d'évolution de ladite variable au sein du voisinage de ce voxel est propre à une et une seule desdites structures,
(ii) une extraction de voxels-modèles qui sont définis comme réalisant les maxima locaux dudit critère,
(iii) une définition, pour chaque voxel-modèle, de l'un desdits échantillons de voxels inclus dans ladite structure correspondante, de manière à ce que cet échantillon présente une évolution de ladite variable qui soit conforme à celle du modèle de la structure à laquelle appartient ledit voxel-modèle, puis

(iv) une estimation, sur chaque échantillon ainsi défini, des paramètres dudit modèle d'évolution de la structure dans laquelle ledit échantillon est inclus, puis

c) une fusion desdits échantillons regroupant les échantillons dont le modèle d'évolution est propre à la même structure, ladite fusion suivant, précédant ou incluant une classification de tous les voxels de ladite image ou séquence d'images ou d'une zone d'intérêt de celle-ci par agrégation à un groupe d'échantillons, de manière qu'une similarité maximale existe entre l'évolution de ladite variable pour ces voxels et l'évolution dudit modèle caractérisant ce groupe d'échantillons.

Cette invention s'applique aussi bien aux images bidimensionnelles, qui peuvent être des cas particuliers d'images tridimensionnelles dans lesquelles une des trois dimensions adopte la valeur un. Comme indiqué ci-dessus, nous utilisons le terme « tridimensionnel » pour désigner les images bidimensionnelles, qui sont un cas particulier d'images tridimensionnelles, aussi bien que les images tridimensionnelles.

Il est essentiel de noter que la méthode selon l'invention permet une segmentation automatique en structures ou régions d'intérêt (e.g. des pharmaco-organes), qui est uniquement basée sur l'information (e.g. pharmacocinétique) présente dans une série séquentielle d'images, notamment grâce à l'initialisation non aléatoire qu'elle comprend à l'étape b) précitée où l'on extrait les paramètres des modèles des structures d'intérêt dans des zones positionnées automatiquement au coeur de ceux-ci. Cette initialisation est adaptée aux données à traiter. Cette méthode permet ainsi d'obtenir systématiquement le même résultat sur un même jeu de données avec les mêmes paramètres, du fait de son caractère déterministe.

Par « voxel », on entend dans la présente description un élément unitaire de l'image volumique « 3D », qui est généralement choisi cubique. C'est le plus petit volume spatial dont on dispose au sein de l'image. Les pixels sont des éléments d'images bidimensionnelles et peuvent être considérés comme des cas particuliers de voxels. Par la suite, nous utiliserons le terme « voxel » pour englober les termes « voxel » et « pixel ».

Par « région », on entend une zone connexe de l'image, i.e. en un seul morceau. Deux régions seront dites connexes si elles se touchent.

De préférence, ladite image ou séquence d'images de départ est obtenue par une technique d'imagerie choisie dans le groupe constitué par la tomographie par émission de positons (TEP), l'imagerie par résonance magnétique (IRM), la tomographie d'émission de photons (e.g. « SPECT »), les imageries optiques, le scanner aux rayons X, l'imagerie histologique, l'imagerie autoradiographique, l'imagerie satellitaire et l'imagerie photographique.

D'une manière générale, on notera que la technique d'imagerie utilisée peut être aussi bien de type « 2D » que « 3D ».

A titre encore plus préférentiel, ladite séquence d'images est obtenue par la technique TEP. Dans ce cas, on met avantageusement en oeuvre à l'étape b) des analyses en composantes principales locales dans un espace à n dimensions, soit au voisinage d'un voxel, soit en un échantillon, pour, d'une part, calculer le critère de validité de l'hypothèse selon laquelle ledit modèle d'évolution au sein du voisinage dudit voxel est propre à une unique structure et, d'autre part, estimer à partir d'un échantillon les paramètres du modèle d'évolution de la structure dans laquelle cet échantillon est inclus.

Une analyse en composantes principales (ACP) opérée sur un tel ensemble de mesures multidimensionnelles comprend un changement de repère, tel qu'un minimum d'axes rend compte d'un maximum de la variance du signal. On distingue les vecteurs propres ou vecteurs directeurs des axes dégagés par l'ACP, et les valeurs propres qui sont respectivement associées à ces axes et qui représentent chacune la variance du signal le long de l'axe correspondant. En général, on trie les axes ou vecteurs propres par valeur propre décroissante.

La reconstruction d'une mesure est représentée par les k premiers vecteurs propres de l'ACP, et on réalise la projection de cette mesure sur le sous-espace déterminé par les k vecteurs propres associés aux k plus grandes valeurs propres de l'ACP. Le résidu de la projection est égal à la mesure elle-même à laquelle est soustraite la projection de cette mesure sur l'ACP, et ce résidu, représentatif du bruit relatif aux mesures, correspond à une projection sur les axes de faible variance.

La variance du signal non reconstruit au sein de l'ensemble de mesures est égale à la moyenne des plus petites valeurs propres de l'ACP non prises en compte pour la reconstruction du signal. Si le nombre de valeurs propres retenues est juste suffisant pour reconstruire le signal au sein de l'ensemble, la variance de signal non reconstruit représente la variance du bruit. Si ce nombre de valeurs propres est trop petit, la variance de signal non reconstruit contiendra à la fois le bruit et du signal.

On définit en outre comme suit la variance de signal non reconstruit relative au signal. Si l'on divise l'écart type du signal non reconstruit par la norme de la moyenne du signal au sein de l'ensemble, on obtient le rapport signal sur bruit lorsque k est juste assez grand pour que les k premiers vecteurs propres de l'ACP reconstruisent le signal. Dans une zone sans signal, alors que la variance de signal non reconstruit sera identique à ce qu'elle serait dans une zone où le signal est bien reconstruit, la variance de signal non reconstruit relative au signal sera très forte.

On définit également la proportion de signal non reconstruit comme étant égale à la norme du résidu de reconstruction de la mesure par l'ACP, divisé par la norme de cette même mesure.

Dans son application particulièrement avantageuse à la pharmaco-imagerie, on notera que la méthode selon l'invention répond aux exigences de l'imagerie corps entier. En effet, lesdites images de départ peuvent être avantageusement des images d'un organisme entier humain ou animal, l'image de segmentation segmentant ledit corps en une partition de pharmaco-organes dont les contours respectifs délimitent les régions d'intérêt.

Dans cette application, on notera également que la méthode selon l'invention répond en outre aux exigences de l'imagerie corps entier en mouvement, dans la mesure où cette méthode est applicable à un organisme entier animé de mouvements physiologiques de type périodiques tels que la respiration et les battements du coeur, dont la période est réduite en comparaison de la durée d'acquisition de l'une des images de départ de ladite séquence, ou bien à des mouvements physiologiques non périodiques.

Ainsi, une cinétique de concentrations pourra être constituée d'un mélange des pharmaco-organes qui auront traversé l'élément de volume où cette cinétique est lue. La cinétique lue à un instant donné contiendra le même ratio de chaque pharmaco-organe que la même cinétique à un autre instant. De plus, le modèle choisi à l'étape a) permet de prendre en compte ce type de mouvements périodiques lors de l'étape d'extraction des voxels-modèles, excluant les zones de l'image traversées par plusieurs pharmaco-organes au cours du mouvement périodique.

Quant aux mouvements non périodiques ou de période de l'ordre de la durée d'acquisition d'une image de la séquence (e.g. digestion, dilatation de la vessie, mouvement globaux du corps), aucun voxel-modèle ne pourra être extrait dans les zones affectées par de tels mouvements, du fait que les cinétiques d'aucune de ces zones ne pourront satisfaire localement le modèle à un seul organe. Ces zones seront donc exclues de la phase critique de détermination des modèles des pharmaco-organes (voir la figure 2 qui illustre des cinétiques relatives à des mouvements non périodiques de vessies de rats).

On notera que la présente invention permet de s'affranchir de la limitation qu'impose une définition purement anatomique des organes, tout en restant compatible avec les techniques d'imagerie bimodales.

Par « pharmaco-organe », on entend de manière connue dans la présente description une parcelle de l'organisme présentant une réponse identique à des traceurs, de sorte que les pharmaco-organes ne sont pas totalement identifiables aux organes. Par exemple, l'organe rein pourra être dissocié en au moins deux pharmaco-organes : le cortex du rein et le bassinet, dans lesquels les traceurs présentent des cinétiques du fait du temps nécessaire à la filtration du sang par le cortex du rein pour former l'urine dans le bassinet.

Par « traceur », on entend de manière connue dans la présente description une molécule entrant dans les mécanismes biologiques étudiés et marquée de façon à pouvoir être suivie dans l'organisme. Cette molécule est injectée par voie intraveineuse à dose « traceuse », c'est-à-dire suffisamment importante pour être repérée, mais suffisamment faible pour ne pas perturber le fonctionnement de l'organisme. Un tel traceur, alors marqué par un isotope radioactif, est notamment utilisé dans les techniques d'imagerie moléculaire, notamment la tomographie d'émission de positions (TEP), la gammatomographie (« Single-Photon Emission Computed Tomography », « SPECT » en abrégé), l'imagerie optique, l'imagerie par IRM et l'imagerie par ultrasons.

A titre d'exemple de variable de type physique, chimique ou biologique caractérisant ladite séquence, on utilise avantageusement la concentration radioactive en un instant donné $t_0$ à $t_n$ d'au moins un principe actif marqué et injecté dans ledit organisme, l'ensemble des voxels à l'intérieur de chaque pharmaco-organe présentant des cinétiques biochimiques de répartition dudit principe actif qui sont similaires.

Selon d'autres caractéristiques de la méthode de l'invention :

- on admet qu'il existe dans chaque image de départ de ladite séquence une partition de l'espace en un nombre fini desdites structures, lesquelles sont chacune connexes et présentent chacune en leur sein un comportement homogène en réponse au phénomène étudié dont l'évolution de ladite variable est représentative; et
- on détermine le nombre desdites structures a *posteriori.*

Selon d'autres caractéristiques avantageuses de l'étape a) de modélisation selon l'invention :

- ledit modèle comprend en outre des paramètres hétérogènes dépendant des coordonnées spatiales des voxels au sein d'une même structure, et l'on admet que lesdits paramètres homogènes et hétérogènes peuvent être estimés sur un ou plusieurs éléments de volume inclus dans cette même structure ;
- on admet en outre que lesdites structures présentent entre elles des réponses différentes audit phénomène étudié, à moins qu'elles ne soient pas connexes ;
- on admet en outre que le bruit, qui est inhérent auxdites mesures et qui est dû au mode d'acquisition de ladite séquence d'images de départ et de segmentation, est additif ;

- on se fixe en outre les deux contraintes suivantes, pour prendre en compte des mélanges locaux de différentes évolutions temporelles dudit signal :

  (i) si la totalité des voxels qui sont voisins d'un voxel et qui contribuent à l'évolution de ladite variable relative à ce voxel est incluse dans la même structure, alors ledit signal correspondant est une réalisation du modèle de cette structure, et
  (ii) ledit jeu de paramètres homogènes pour ces voxels voisins est le même que celui dudit modèle propre à cette structure ; et

- dans le cas d'une technique d'imagerie avec injection de traceur, telle que la TEP ou la « SPECT », l'imagerie optique, l'imagerie par IRM et l'imagerie par ultrasons, ledit modèle peut être un modèle compartimental de type à un ou plusieurs traceurs indépendants et à plusieurs compartiments, tels que des compartiments biologiques (on suppose que les traceurs se répartissent dans les compartiments, et ce modèle étudie la concentration du traceur dans chaque compartiment via des vitesses de transfert entre les compartiments).

Selon un exemple préférentiel de réalisation de l'invention, le modèle choisi est un modèle compartimental à quatre paramètres, mais la présente méthode peut être étendue sans modification à un modèle compartimental à six paramètres. Cette modélisation suppose les organes sains. En cas d'organes présentant des zones pathologiques, ces dernières feront l'objet d'un modèle séparé, et seront donc segmentées séparément de la partie saine de l'organe. Les zones pathologiques des organes pourront donc être mises en relief par la présente méthode.

On notera que la modélisation du signal qui est mise en oeuvre à cette étape a) passe par une étude des mécanismes essentiels du phénomène en jeu, par exemple en introduisant des paramètres physiologiques comme la concentration de traceur dans le plasma ou le tissu, ou encore la fraction de volume sanguin, dans le cas de la pharmaco-imagerie (par « phénomène », on entend un processus caché dont les effets sont mesurables indirectement, e.g. le phénomène « activation de neurone » qui est corrélé à sa consommation de glucose dans le cas de l'imagerie TEP).

Ledit modèle utilisé à l'étape a) est notamment conçu pour permettre de calculer des expressions analytiques des mesures attendues, conformément au phénomène étudié. La confrontation de ces expressions analytiques avec les mesures des effets du phénomène permet d'en estimer les paramètres (e.g. pour le phénomène « activation de neurone », la consommation de glucose peut être mesurée indirectement et partiellement via la technique TEP, par injection puis acquisition de molécules de fluoro déoxy glucose marquées par un isotope émetteur de position).

On notera que ces mélanges locaux de différentes évolutions temporelles du signal traduisent un effet dit de « volume partiel », dû à la faible résolution intrinsèque de la caméra utilisée. Le signal mesuré en un voxel donné contient alors un mélange des cinétiques physiologiques des régions d'intérêt voisines (e.g. des pharmaco-organes voisins).

On notera également que la méthode selon l'invention permet de déterminer de façon fiable, à l'effet de volume partiel près, les modèles d'évolution correspondant à chaque structure d'intérêt, tels que des modèles de cinétiques d'un pharmaco-organe. Ces modèles sont en effet estimés dans les zones de l'image où le modèle de cinétique à un seul organe est le plus valable. Il est donc inutile d'affiner les paramètres des modèles des pharmaco-organes au cours de multiples itérations : une simple agrégation de chaque élément de volume au modèle qui représente le mieux sa cinétique suffit.

On choisit dans la présente méthode un modèle de bruit additif gaussien non stationnaire dans le temps. Le bruit est estimé sur l'ensemble des échantillons extraits lors de la segmentation, et il est corrigé de l'influence du signal dans le cas d'une séquence d'image reconstruite par une méthode itérative. La méthode selon l'invention prend en compte :

- la non-stationnarité spatiale du bruit au sein des images reconstruites par une méthode itérative, en corrigeant les données de l'influence du signal lors de chaque étape de notre algorithme, et
- la non-stationnarité temporelle du bruit, ce qui permet de donner aux différentes images de la séquence des pondérations différentes rendant compte de la variabilité réelle des données autour du modèle.

Selon d'autres caractéristiques de l'étape b) d'extraction de voxels-modèles selon l'invention :

- cette étape b) comprend une sélection préalable, dans un espace à n dimensions ($n \geq 1$) correspondant à ladite séquence d'images de départ (par exemple n=38 pour une séquence d'images TEP données), de voxels-modèles pour que chacune desdites structures d'intérêt contienne en son coeur au moins un voxel-modèle ainsi que le voisinage de celui-ci ;
- cette étape b) comprend, suite à ladite sélection préalable, la définition d'une métrique prévue pour définir les distances dans ledit espace et ledit critère d'extraction local ; et
- on admet à l'étape b) que pour toute structure à segmenter, il existe un voxel dont le voisinage est compris au sein de la région d'intérêt correspondante.

Selon une autre caractéristique de l'invention, cette étape b) comprend :

- une détermination par exemple itérative, pour chaque structure d'intérêt, d'un échantillon de voxels (i.e. une liste de voxels) appartenant à cette structure, cette appartenance étant déterminée par une similitude de l'évolution de ladite variable avec le modèle relatif à cette structure, puis
- une estimation, pour chaque échantillon, des paramètres homogènes du modèle qui correspondent à la structure.

On entend par « distance » dans la présente description la distance entre deux mesures de l'évolution temporelle dudit signal (e.g. mesures de cinétiques dans le cas de mesures de concentrations radioactives par la technique TEP, notamment). En général on utilise une distance pondérée de Minkowski, dont les distances euclidiennes, de Manhattan et distance maximum sont des cas particuliers. Le facteur de pondération, qui est choisi pour compenser une éventuelle non-stationnarité du signal, est en général pris égal à 1 pour chaque valeur de mesure (e.g. un instant déterminé de la cinétique des concentrations).

On entend par « seuillage » d'une image l'opération qui consiste à sélectionner les voxels dont la mesure dans l'image est supérieure à un seuil et, par extension, l'opération où l'on sélectionne les voxels dont la mesure dans l'image est comprise entre deux seuils : un seuil bas et un seuil haut. Un seuillage peut donc consister à sélectionner les voxels dont la mesure dans l'image est égale à une valeur donnée.

Selon une autre caractéristique de l'invention, on notera que la bordure de chaque structure est exclue de l'extraction desdits échantillons qui est mise en oeuvre à l'étape b) précitée selon l'invention.

Selon une autre caractéristique de l'invention, l'étape c) de fusion et de classification comprend une fusion desdits échantillons correspondant à une même structure d'intérêt, avant ou après ladite classification des voxels. Cette fusion est mise en oeuvre par exemple par une classification ascendante hiérarchique. Quant à la classification des voxels qui est également comprise dans l'étape c), elle est par exemple mise en oeuvre par une analyse discriminante, étant entendu que toute méthode de classement pourrait convenir pour cette classification.

A l'issue de cette étape c) de fusion et de classification, on obtient une image de label indiquant à quel groupe d'échantillons un voxel donné est apparenté. Bien que l'on puisse se satisfaire de cette image, il est nécessaire, pour vérifier la définition d'une image de segmentation, de séparer chaque composante connexe pour chaque label.

Selon une autre caractéristique de l'invention, ladite méthode de segmentation comprend en outre, suite à l'étape c), une étape de séparation de composantes connexes qui est mise en oeuvre pour chacun desdits labels.

On notera que cette étape de séparation des composantes connexes permet de séparer entre elles des structures non connexes qui présentent des évolutions similaires de ladite variable, et que cette étape de séparation est nécessaire pour passer d'une image de classification à une image de segmentation en régions d'intérêt.

Selon une autre caractéristique avantageuse de l'invention, ladite méthode comprend en outre une étape optionnelle de pré-segmentation qui est mise en oeuvre avant ou après l'étape a) et qui consiste à séparer lesdites images de départ en une première partie contenant lesdites structures d'intérêt et en une seconde partie correspondant à un fond d'image exclu de la segmentation.

On notera que cette étape de pré-segmentation permet non seulement d'accélérer la mise en oeuvre de la méthode selon l'invention, mais encore d'éviter la pollution de la segmentation de la zone d'intérêt de l'image par des cinétiques non significatives de structures.

Selon une autre caractéristique avantageuse de l'invention et indépendante de la caractéristique optionnelle précédente (i.e. non conditionnée par celle-ci), ladite méthode comprend en outre une autre étape optionnelle d'optimisation des échantillons qui est mise en oeuvre suite à l'étape b) et avant l'étape c) et qui consiste à rechercher par un algorithme par exemple itératif, pour chaque échantillon, une forme par exemple géométrique, paramétrique ou libre qui minimise en son sein le signal d'autres structures que celle où le voxel-modèle a été extrait.

On utilisera par exemple pour cette étape d'optimisation des échantillons une « croissance de région », i.e. une agrégation des voxels voisins des agrégats à ces derniers, par ordre décroissant de similitude avec ledit modèle.

On propose, par exemple, l'algorithme suivant permettant d'extraire un échantillon $\psi_i$ minimisant en son sein le signal provenant d'autres structures que $i$.

A la première itération, l'échantillon $\psi_i$ recherché est égal à l'ensemble des cinétiques contenues dans $V_j$.

A chaque itération, le modèle de la structure $i$ est estimé au sein de la forme $\psi_i$. Ensuite, une croissance de région est opérée à partir de l'élément de volume $j$, en maximisant à chaque nouvelle agrégation un critère de similarité entre la cinétique de l'élément de volume considéré et le modèle estimé de la structure $i$. La croissance de région cesse lorsque le nombre d'éléments de volume inclus dans la région réalisée devient suffisant pour estimer les paramètres $\{\theta_u(i)\}_{1 \leq u \leq U}$ du modèle $M$ pour la structure $i$ - par exemple égal à celui contenu dans $V_j$. $\psi_i$ est alors calculée comme l'ensemble des cinétiques des éléments de volume de la région réalisée.

Le processus itératif cesse *lorsque* $\psi_i$ cesse d'évoluer d'une itération à l'autre.

On notera que cette étape optionnelle d'optimisation des échantillons améliore la qualité et la robustesse de la segmentation obtenue de chaque structure détectée, car elle assure que l'échantillon est bien inclus dans la structure correspondante.

Selon une autre caractéristique avantageuse de l'invention et indépendante de chacune des deux caractéristiques optionnelles précédentes, ladite méthode comprend en outre une étape optionnelle de représentation hiérarchique desdites régions d'intérêt obtenues qui est mise en oeuvre suite à l'étape c) et avant ladite étape de séparation des structures non connexes.

On notera que cette étape optionnelle de représentation hiérarchique des organes permet à l'utilisateur de choisir le niveau de fusion optimal pour une structure donnée. Un niveau de fusion trop élevé ne permettrait pas de détecter la structure, qui serait fusionné avec une autre, tandis qu'un niveau de fusion trop bas scinderait la partie correspondante de l'image en deux zones représentant la même structure. Cette représentation hiérarchique permet, de plus, de choisir a *posteriori* le nombre de structures au sein de l'image.

La présente méthode propose de conserver toutes les images de segmentation pour chaque niveau de fusion et de laisser le choix à l'utilisateur, e.g. pour un pharmaco-organe donné, du niveau de fusion où il est le mieux segmenté.

On notera que cette méthode fusionne non pas les éléments de volume, mais les échantillons via les modèles d'évolution (e.g. de cinétiques de pharmaco-organes), dont le nombre est avantageusement plus faible de quatre ordres de grandeur. La « navigation » entre les divers niveaux de fusion est donc possible et peut être interprétée comme une recherche du niveau de fusion qui est tel que tous les modèles correspondant au même pharmaco-organe soient fusionnés. L'identification entre agrégat et région d'intérêt (e.g. pharmaco-organe) devient donc totale.

D'une manière générale, on notera que la méthode selon l'invention est applicable à la segmentation de tous types d'images ou de séquences d'images de départ, e.g. des images satellites.

D'une manière générale, on notera que la méthode selon l'invention permet de traiter une grande masse de données.

En effet, le temps de calcul reste compatible avec les besoins des utilisateurs, même dans le cas d'acquisition de données par une caméra de type TEP à haute résolution spatiale. La complexité des étapes critiques de l'algorithme - extraction des voxels-modèles et segmentation - est en effet proportionnelle au nombre d'individus à classer. Plusieurs millions d'individus sont ainsi classés sans que l'augmentation de ce nombre d'individus ne nécessite d'effectuer d'approximations lors de la segmentation. De plus, la présente méthode ne nécessite pas d'hypothèses sur la prévalence de certaines images de la séquence sur d'autres en termes de stabilité des phénomènes physiologiques.

On notera également que la méthode selon l'invention bénéficie d'un coût en temps de calcul qui est fixe et est notamment compatible avec le temps de traitement de données issues de la technique TEP.

On notera en outre que la présente méthode permet de segmenter des régions de tailles très différentes, en ne faisant aucune hypothèse sur la taille des structures à segmenter, telles que des pharmaco-organes.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec les dessins joints, parmi lesquels :

la figure 1 est un diagramme à blocs illustrant les différentes étapes obligatoires ou optionnelles de la méthode de segmentation selon l'invention,

la figure 2 est un graphique illustrant notamment les familles de cinétiques non physiologiques qui sont générées par le déploiement de vessies de rats, dans une image acquise par la technique TEP,

les figures 3a et 3b sont des schémas illustrant un exemple de modélisation compartimentale à un seul traceur d'un processus physiologique d'interaction entre le traceur et un organe, via trois compartiments et quatre paramètres,

la figure 4 est un histogramme illustrant, dans le cas d'une pré-segmentation selon l'invention, le nombre réduit de valeurs négatives d'une cinétique obtenue par la technique TEP qui est relative à un élément de volume inclus dans un organisme de rat,

la figure 5 est un histogramme illustrant, dans ce cas optionnel de pré-segmentation, le nombre plus élevé de valeurs négatives d'une cinétique relative à un élément de volume extérieur à cet organisme de rat,

les figures 6a et 6b illustrent deux analyses d'histogrammes de pré-segmentation illustrant l'extraction de l'indice du minimum d'un histogramme situé entre les deux pics correspondant respectivement à l'organisme de rat de la figure 4 et au fond d'image de la figure 5, cet indice correspondant à une valeur de seuillage permettant de séparer les éléments de volume de l'organisme de rat de ceux du fond,

les figures 7a et 7b sont deux images masquées obtenues par pré-segmentation à partir d'oligo-nucléotides injectés à ce rat, ces deux images correspondant respectivement aux figures 6a et 6b,

les figures 8a et 8b sont deux images de coupes coronales d'organismes de rats obtenues lors de l'étape d'extraction de voxels-modèles selon l'invention, l'image 8a illustrant une coupe coronale passant par le coeur et les reins et l'image 8b illustrant une coupe coronale passant par la vessie,

les figures 9a et 9b sont deux images de coupes coronales qui dérivent respectivement des figures 8a et 8b et qui illustrent les minima locaux des variances de données non reconstruites ayant été obtenus au coeur des organes par analyse en composantes principales,

les figures 10a et 10b sont deux images de coupes coronales qui dérivent respectivement des figures 9a et 9b et qui ont été obtenues suite à l'étape d'extraction par une étape d'optimisation des échantillons selon l'invention mise en oeuvre par croissance de région,

les figures 11 a et 11 b sont deux images de coupes coronales qui dérivent respectivement des figures 9a et 9b et qui ont été obtenues suite à une étape de classement des cinétiques selon l'invention en cent classes,

la figure 12 illustre un exemple d'arbre de fusion obtenu par une étape de fusion des échantillons selon l'invention qui fait suite à l'étape de classement des cinétiques et qui est mise en oeuvre par une classification ascendante hiérarchique,

les figures 13a et 13b sont des images de coupes coronales qui dérivent respectivement des figures 11a et 11b et qui ont été obtenues par fusion des échantillons via une classification ascendante hiérarchique, où dix-huit classes ont été conservées,

les figures 13c et 13d sont des images de coupes coronales qui dérivent respectivement des figures 13a et 13b et qui ont été obtenues par fusion des labels de l'image de classement des cinétiques via une classification ascendante hiérarchique, où dix-huit classes ont été conservées,

les figures 14a, 14b et 14c sont des images « 3D » de segmentation respectivement obtenues par cette étape de fusion selon des vues coronale, sagittale et oblique, et

la figure 15 est un diagramme à blocs illustrant une représentation hiérarchique des pharmaco-organes selon l'invention qui peut être mise en oeuvre suite à cette étape de fusion des échantillons.

Dans l'exposé suivant de la méthode selon l'invention, on a utilisé les notations suivantes :

- ♦ $i$ : un organe, un label ou un échantillon et $I$ : le nombre d'échantillons.
- ♦ $o$ : un organe.
- ♦ $t$ et $t'$ : des instants ou des indices d'images de la séquence.
- ♦ $\Delta_t$ : la durée d'acquisition de l'image $t$ de la séquence.
- ♦ $j$ et $k$ : des éléments de volume (voxels).
- ♦ $s$ : un traceur injecté et S le nombre de traceurs injectés.
- ♦ $r$ : un rayon.
- ♦ $C_j$ : la variation de la concentration au sein de l'élément de volume $j$ au cours du temps, que l'on nommera par la suite cinétique.
- ♦ $V_j$ : le voisinage de l'élément de volume $j$.
- ♦ $\psi_j$ : un échantillon de cinétiques représentatives du modèle de la structure $i$.
- ♦ $Cp(t)$ : la cinétique plasmatique, constante dans l'organisme.
- ♦ $Ca(t)$ : la cinétique artérielle, constante dans l'organisme.
- ♦ $Cf_i(t)$ : la cinétique du compartiment libre.
- ♦ $Cb_i(t)$ : la cinétique du compartiment lié.
- ♦ $Ct_i(t)$ : la cinétique au sein du tissu : $Ct_i(t) = Cf_i(t) + Cb_i(t)$.
- ♦ $M_{i,j}(t)$ : la cinétique prédite par modèle à un seul organe, l'organe $i$ au sein de l'élément de volume $j$.
- ♦ $\varepsilon_j(t)$ : le bruit de détection et de reconstruction à l'élément de volume $j$.
- ♦ $\sigma$ : l'écart type du bruit.
- ♦ $K_1$, $K_2$, $K_3$ et $K_4$ : les paramètres d'un modèle à trois compartiments.
- ♦ $Vb_{i,j}$ : le ratio de volume plasmatique au sein de l'élément de volume $j$ de l'organe $i$.
- ♦ $\mu_i(t)$ : la cinétique moyenne du modèle $M_{i,j}(t)$ au sein de l'organe $i$.
- ♦ $\beta_{j,k}$ : le coefficient de contamination de la cinétique de l'élément de volume $j$ par celle de l'élément de volume $k$, et inversement.
- ♦ $i(k)$ : la notation signifiant que $i$ dépend de $k$. Il s'agit ici de l'organe $i$ physiologiquement présent dans l'élément de volume $k$.
- ♦ $n_j$ : le nombre de valeurs nulles ou négatives de la cinétique $C_j(t)$ à l'élément de volume $j$.
- ♦ $Mode_{fond}$ : le pic de l'histogramme correspondant au fond.
- ♦ $Mode_{org}$ : le pic de l'histogramme correspondant à l'organisme.
- ♦ $nmin$ : m'indice du minimum de l'histogramme situé entre $Mode_{org}$ et $Mode_{fond}$, correspondant à une valeur de seuil permettant de séparer les éléments de volume de l'organisme imagé de ceux du fond.
- ♦ $B_{j,r}$ : la boule de rayon $r$ centrée en $j$.
- ♦ $\mu_j(t)$ : la moyenne des cinétiques $C_k$ au sein de la boule $B_j,r$.
- ♦ $e_{j,l}(t)$ : le $i^{\text{ème}}$ vecteur propre et $\lambda_{j,l}$ : la $i^{\text{ème}}$ valeur propre de l'analyse en composantes principales calculée sur les cinétiques des éléments de volumes inclus dans la boule $B_{j,r}$.

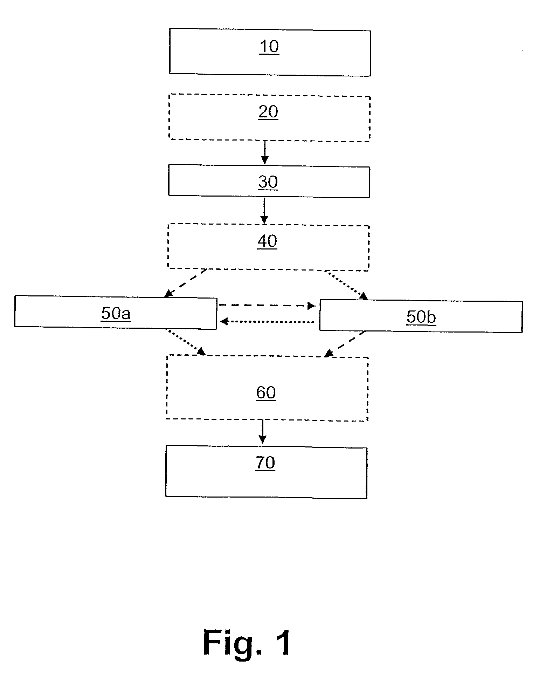

La méthode selon l'invention, dont les principales étapes obligatoires ou optionnelles sont illustrées à la figure 1, est préférentiellement mise en oeuvre via la technique d'imagerie TEP. On notera toutefois que cette méthode de l'invention n'est nullement limitée à cette technique.

Le diagramme de la figure 1 illustre les huit étapes de cette méthode, cinq de ces étapes 10, 30, 50a, 50b et 70 étant obligatoires (symbolisées par des cases en traits pleins) et trois autres étapes 20, 40 et 60 étant facultatives (symbolisées par des cases en pointillés).

Une première étape 10, essentielle dans la méthode selon l'invention, consiste en une modélisation du signal au sein de l'image, via la définition d'un modèle adapté au phénomène étudié. Ce modèle est défini une fois pour toutes pour un phénomène donné, et n'a donc pas à être redéfini pour une nouvelle image rendant compte de ce phénomène. La donnée de ce modèle permettra par la suite non seulement de déterminer si un volume donné contient les cinétiques provenant d'une unique structure, mais aussi de définir une distance entre une cinétique et un échantillon de cinétiques supposé représentatif d'une structure donnée.

Si nécessaire, une seconde étape optionnelle 20 de pré-segmentation de l'image séparera cette dernière en deux parties, l'une de ces parties correspondant à la zone étudiée, telle qu'un organisme avec la technique TEP, et l'autre partie correspondant au fond de l'image, jugé sans intérêt.

Une troisième étape 30, également essentielle dans la méthode selon l'invention, consiste en une extraction de voxels-modèles au coeur de chaque structure d'intérêt, rendue possible par la mesure de la présence d'un unique modèle de cinétique de structure au sein du voisinage de chaque voxel de l'image. Une même structure peut alors contenir plusieurs voxels-modèles.

Il est possible de conserver les voisinages définis ci-dessus des voxels-modèles extraits comme échantillons de points situés dans les organes. Cependant, il peut être judicieux, pour un voxel-modèle donné, de mettre en oeuvre une quatrième étape optionnelle 40 d'optimisation des échantillons dans laquelle on recherche un échantillon dont la forme est plus adaptée à celle de la structure au sein de laquelle le voxel-modèle a été extrait. Ce nouvel échantillon peut par exemple être extrait par une méthode connue de croissance de région.

On met ensuite en oeuvre deux étapes 50a et 50b toutes deux obligatoires, selon l'un ou l'autre des deux modes de réalisation selon l'invention décrits ci-après.

Selon un premier mode de réalisation, on met en oeuvre l'étape 50a de classement des cinétiques suite à l'étape 30 ou 40, dans laquelle chaque élément de volume de l'image est agrégé à l'échantillon dont le modèle est le plus proche de celui de cet élément. Puis on met en oeuvre l'étape 50b de fusion des échantillons, où les échantillons appartenant à un même organe sont fusionnés.

Selon un second mode de réalisation, on met en oeuvre l'étape 50b de fusion des échantillons suite à l'étape 30 ou 40, puis on met en oeuvre l'étape 50a de classement des cinétiques.

Si l'étape 50b de fusion utilise une méthode hiérarchique, cette hiérarchie de fusion peut être utilisée pour décrire les structures dans une étape optionnelle 60 de représentation hiérarchique des organes, une structure donnée apparaissant sur une plage de niveaux de fusion donnés. Dans un cas de figure idéal, l'intersection de toutes les plages d'apparition des structures est non vide et peut être extraite automatiquement. Dans un cas contraire, le choix peut être laissé à l'utilisateur, pour chacune des structures, de la plage de niveaux de fusion pour lequel elle apparaît le mieux.

On obtient à cette étape 60 une image d'agrégation des éléments de volumes aux fusions d'échantillons dont ils sont les plus semblables.

Cependant, comme deux structures peuvent présenter des réponses identiques au phénomène étudié tout en étant disjointes, on met en oeuvre préférentiellement une étape optionnelle 70 de séparation des composantes connexes, qui permet de séparer de telles structures.

Au terme de la mise en oeuvre de cette méthode selon l'invention, on dispose d'une segmentation de l'image conforme au modèle de signal choisi.

Plus précisément, la méthode de segmentation selon l'invention peut être décomposée comme suit.

On étudie un phénomène dont les effets sont mesurables en chaque point de l'espace discrétisé - image - et dont la mesure comporte $T$ valeurs ($T > 1$) - ces valeurs pourront par exemple être les mesures du phénomène à des moments successifs. Dans la suite, on appellera « cinétique » la suite ordonnée des $T$ valeurs de la mesure.

Appelons S la mesure sans bruit de ce phénomène en chaque point de l'espace. Appelons C la mesure réelle, soumise aux limitations du mode de détection utilisé.

On va détailler ci-après les principales hypothèses et contraintes sur lesquelles se base cette méthode selon l'invention :

Hypothèse H1 : il existe une partition de l'espace en un nombre fini de structures présentant chacune en son sein un comportement homogène et telle que chacune de ces structures soit connexe.

La présente méthode cherche donc à réaliser une telle partition au sein de l'image en se basant sur l'information contenue dans les mesures réelles $C$. Pour ce faire, nous commencerons par définir un modèle de signal basé sur la modélisation du phénomène lui-même. Ce modèle nous permettra d'effectuer une mesure de la présence de plusieurs structures dans un petit volume. Cette mesure est nécessaire à l'étape d'extraction des voxels-modèles. D'autre part, la probabilité d'appartenance d'un élément de volume à une structure sera déterminée par la similarité de la cinétique de cet élément de volume avec le modèle de la structure.

### ➢ **Etape 10 de modélisation du signal :**

Définissons un modèle paramétrique $M(\theta_u(i), \varphi_v(j))$ du phénomène, dont certains paramètres $\{\theta_u(i)\}_{1 \leq u \leq U}$ ne dépendent que de la structure étudiée $i$ et sont par conséquent homogènes au sein de cette structure. Les autres paramètres $\{\varphi_v(j)\}_{1 \leq v \leq V}$ du modèle M dépendent de la position $j$ dans l'espace. Les paramètres $\{\varphi_v(j)\}_{1 \leq v \leq V}$ sont donc variables au sein d'une même structure. On notera par la suite $M_{i,j}$ la réalisation du modèle de cinétique pour la structure $i$ à l'élément de volume j. Le modèle sera d'autant plus contraint que le nombre V de paramètres $\varphi_v(j)$ sera plus faible. On choisira M tel qu'en tout point j de $i$, on puisse supposer $S_j = M_{i,j}$.

La réponse d'une structure au phénomène étudié est caractérisée par l'ensemble des paramètres $\{\theta_u(i)\}_{1 \leq u \leq U}$, dont la valeur est propre à la structure $i$ et homogène au sein de celle-ci. M sera donc choisi de telle sorte que deux structures aux comportements différents présentent chacune un jeu de paramètres $\{\theta_u(i)\}_{1 \leq u \leq U}$ différent.

Hypothèse H2 : les paramètres de $M_{i,j}$ peuvent être estimés sur un échantillon de mesure inclus dans $i$ et de taille suffisamment grande.

Hypothèse H3 : les structures dont on cherchera à délimiter les contours présentent entre elles des réponses différentes au phénomène étudié, à moins qu'elles ne soient pas connexes.

Deux structures connexes présentant la même réponse au phénomène seront donc considérées comme une seule et même structure.

Hypothèse H4 : Le bruit de mesure dû au mode d'acquisition et de reconstruction de l'image est additif.

On en conclut que la mesure $C_j$ au sein de l'élément de volume $j$ peut s'exprimer comme :

$C_j = S_j + \varepsilon_j$ où le bruit $\varepsilon_j$ peut être non stationnaire tant d'un point de vue spatial que temporel.

Par conséquent, si $j$ est inclus dans $i$, $C_j$ s'écrit :

$$C_j(t) = M_{i,j}(t) + \varepsilon_j(t)$$

Une fois estimés les paramètres $\{\theta_u(i)\}_{1 \leq u \leq U}$ du modèle de la structure $i$, il devient possible de calculer pour tout élément de volume $j$ de l'image l'erreur de reconstruction $\varepsilon_j(t) = C_j(t) - M_{i,j}(t)$.

Cette erreur de reconstruction est un indicateur de la présence de la structure $i$ au voxel $j$.

On va examiner à présent le problème des mélanges locaux de cinétiques.

Dans le cas où $S_j$ n'est pas égal $M_{i,j}$, mais à une fonction $f_j$ connue des réalisations $M_{i(vk)+vk}$ des modèles des structures $i(V_k)$ présents aux éléments de volume $V_k$, les $\{V_k\}_{1 \leq k \leq K}$ étant les voisins de $j$. Le signal au sein d'un élément de volume $j$ donné s'écrit $S_j = f_j(\{M_{i(vk),vk}\}_{1 \leq k \leq K})$.

Cependant, si tous les voisins $\{V_k\}_{1 \leq k \leq K}$ sont inclus dans $i$, $S_j = f_j(\{M_{i,vk}\}_{1 \leq k \leq K}) = N_{i,j}$.

Contrainte C1 : M doit satisfaire à deux conditions :

(i) si tous les voisins $\{V_k\}_{1 \leq k \leq K}$ sont inclus dans $i$, alors $N$ doit toujours être une réalisation du modèle paramétrique $M$ ; et

(ii) le jeu de paramètre $\{\theta_u(i)\}_{1 \leq u \leq U}$ de N doit être le même que celui de M.

Dans le cas où tous les voisins $\{V_k\}_{1 \leq k \leq K}$ sont inclus dans i, on peut alors définir une fonction $h$ de $j$ telle que :

$$S_j = M_{i,h(j)} \text{ et } C_j(t) = M_{i,h(j)}(t) + \varepsilon_j(t)$$

Dans le cas d'une fonction $f_j$ linéaire, $M_i$ doit dépendre linéairement de $j$.

Dans le cas où $f_j$ est un barycentre à coefficients positifs, $M_i$ doit être une fonction convexe de $j$.

### ➢ **Etape 20 de pré-segmentation** :

Dans certains cas, l'image à segmenter contient un grand nombre d'éléments de volume dont la mesure n dimensionnelle correspond à une absence de signal. Ces éléments de volumes peuvent alors être regroupés en une même région. Le modèle de cinétique n'a pas de sens dans de telles régions, et les modèles estimés ne correspondront à aucun phénomène, si ce n'est le bruit. Toute analyse faite dans de telles régions est une perte de temps, qui peut être considérable lorsque leur taille devient non négligeable par rapport à la taille totale de l'image.

Du fait de la nature aléatoire de la mesure C au sein de telles régions, on peut s'attendre à y extraire un grand nombre de voxels-modèles lors de l'étape 30 d'extraction. Les modèles extraits étant eux-mêmes aléatoires, ils seront fort différents les uns des autres et la probabilité pour qu'ils soient tous fusionnés dans une même région est faible. L'application de l'algorithme aux zones de l'image ne présentant pas de signal le ralentira et perturberont l'étape d'extraction 30 et l'étape 50b de fusion des échantillons.

Il est donc avantageux d'isoler, lors de cette étape 20, les zones ne présentant pas de signal, que nous nommerons « extérieur », des zones présentant un signal, que nous nommerons « intérieur ».

On notera que nombre d'opérations permettent d'effectuer une telle séparation, comme par exemple un seuillage.

➢ **Etape 30 d'extractîon des voxels-modèles :**

Soit $H0_{vj}$ l'hypothèse selon laquelle le voisinage $V$ de l'élément de volume $j$ ne contient que des cinétiques provenant du modèle d'une seule structure.

Cette étape cruciale de la méthode selon l'invention va extraire des points dont le voisinage maximise un critère d'inclusion dans une structure. L'étape 10 de modélisation du signal présent dans l'image permet de définir ce critère comme la mesure de la validité de l'hypothèse $H0_{vj}$. Si un modèle à une seule structure est incapable de capter le signal au sein du voisinage considéré, alors il est probable que ce dernier contient les modèles de cinétiques de plusieurs structures.

Cette étape nécessite cependant une hypothèse supplémentaire, qui assure d'extraire des points à l'intérieur de chaque structure :

Hypothèse H5 : Pour toute structure, il existe un élément de volume $j$ dont le voisinage $V_j$ est compris au sein de l'organe.

Selon cette hypothèse, toute structure $i$ à segmenter contient une forme identique à celle de $V$ connue et déterminée a *priori*. Le non-respect de cette condition engendrerait une incertitude sur la détection de cette structure.

Dans le cas où le signal présent au sein d'un élément de volume $j$ se calcule comme une fonction $f_j$ des modèles des structures aux éléments de volume voisins, l'hypothèse H5 devient :

Hypothèse H5' : Pour toute structure, il existe un élément de volume dont le voisinage est compris au sein de l'organe, et dont et tel que pour tout k appartenant à ce voisinage, le domaine d'application de $f_k$ est lui aussi inclus dans la structure.

**Indicateur de l'intérieur des organes :**

Soit une mesure $\Gamma_{vj}$ de la validité de l'hypothèse $H0_{vj}$ au sein du voisinage $V_j$ de $j$ comparable entre deux points de l'espace. L'éventuelle non-stationnarité du bruit - tant spatiale que temporelle - devra donc être prise en compte dans le calcul de cette mesure. Cette mesure $\Gamma_{vj}$ est calculée pour chaque élément de volume de l'image - ou de la zone d'intérêt extraite lors de l'étape 20.

**Extraction :**

Les fortes valeurs $\Gamma_{vj}$ correspondent à des éléments de volume $j$ dont chaque point du voisinage $V_j$ - selon l'hypothèse H5 - ou dont le domaine d'application de $f_k$ pour tout $k$ appartenant à $V_j$ - selon l'hypothèse H5' - est inclus dans la même structure. En effet, l'hypothèse $H0_{vj}$ devient non valide au sein de $B_{j,r}$ lorsque $B_{j,r}$ est à cheval entre deux structures $i$ et $o$ caractérisées par des réponses différentes au phénomène étudié.

On extrait donc les maxima locaux de $\Gamma_{vj}$, la mesure de validité de $H0_{vj}$ calculée pour chaque élément de volume $j$ de la zone d'intérêt de l'image (un maximum local $j$ d'une mesure $\Gamma$ est tel que, pour tout $k$ voisin de $j$, on a $\Gamma_{vj} \geq \Gamma_{vk}$).

Cette étape 30 de la méthode selon l'invention détectera des points dans le voisinage duquel seul un modèle de structure est réalisé. Dans le cas d'un mélange local de cinétique par une fonction $f$, les points pour lesquels l'hypothèse H5' n'est pas vérifiée, bien que le voisinage $V_j$ soit inclus dans l'organe, n'ont donc pas la certitude d'être détectés.

A fortiori, cela est vrai pour les structures dont la taille est de l'ordre de la résolution de l'image (et non du dispositif d'acquisition), ainsi que pour les structures fines et allongées ne présentant aucun renflement. Cependant, l'extraction de maxima locaux du critère permet d'extraire des points au sein de régions de taille ou d'épaisseur inférieure - mais non négligeable - à la taille du voisinage considéré. On notera toutefois que l'estimation du modèle dans de telles régions sera polluée par les modèles de cinétiques des structures voisines.

➢ **Etape 40 d'optimisation des échantillons :**

Pour une géométrie de voisinage $V_j$ donnée et conformément au modèle M du phénomène étudié, les cinétiques contenues dans $V_j$ constituent un échantillon localement optimal de cinétiques de la structure $i$ contenant j. Les cinétiques aux éléments de volume contenus dans $V_j$ peuvent donc être choisies comme échantillons de cinétique de la structure $i$.

Cependant, certaines structures peuvent avoir une géométrie très différente de celle de $V$ et ne pas disposer de renflements suffisants pour qu'il existe un voisinage $V_j$ vérifiant H5 ou H5'. Le voisinage $V_j$ entourant un voxel j extrait lors de l'étape 30 pourra donc servir d'initialisation à un algorithme itératif cherchant une forme qui minimise en son sein le signal d'autres structures que $i$. Cette forme pourra être géométrique (ellipse, cylindre), paramétrique (beta-splines) ou encore de forme libre.

Selon la présente invention, un algorithme permettant d'extraire un échantillon $\psi_i$ minimisant en son sein le signal provenant d'autres structures que $i$ peut être le suivant.

A la première itération, l'échantillon $\psi_i$ recherché est égal à l'ensemble des cinétiques contenues dans $V_j$.

A chaque itération, le modèle de la structure $i$ est estimé au sein de la forme $\psi_i$. Ensuite, une croissance de région est opérée à partir de l'élément de volume $j$, en maximisant à chaque nouvelle agrégation un critère de similarité entre la cinétique de l'élément de volume considéré et le modèle estimé de la structure $i$. La croissance de région cesse lorsque le nombre d'éléments de volume inclus dans la région réalisée devient suffisant pour estimer les paramètres $\{\theta_u(i)\}_{1 \leq u \leq U}$ du modèle $M$ pour la structure $i$ - par exemple égal à celui contenu dans $V_j$.

$\psi_i$ est alors calculée comme l'ensemble des cinétiques des éléments de volume de la région réalisée.

Le processus itératif cesse lorsque $\psi_i$ cesse d'évoluer d'une itération à l'autre.

On a ainsi déterminé un nouvel échantillon $\psi_i$ de cinétiques de la structure $i$, dont la forme optimise localement l'estimation du modèle $M$ du phénomène étudié pour la structure $i$.

➢ **Etape 50a de classement des cinétiques :**

La définition des échantillons donne directement accès aux modèles de cinétiques des structures $i$ qui composent l'image. La classification des cinétiques - processus généralement itératif au cours duquel les paramètres du modèle sont estimés - se réduit ici à un simple classement : chaque élément de volume de l'image est agrégé à la structure dont le modèle rend le mieux compte de sa cinétique.

En cas de bruit non stationnaire d'un point de vue spatial, le bruit pourra être « blanchi », à moins que la variance du bruit ne soit estimée au sein des échantillons. Elle peut encore être calculée analytiquement si la dépendance spatiale de la variance du bruit suit une loi connue a *priori.*

Notons que le bruit pourra être estimé au sein des échantillons par la relation :

$$\sigma_i^2(t) = \sum_{k \in \psi_i} \left( C_k(t) - M_{i,k}(t) \right)^2 \cdot$$

➢ **Etape 50b de fusion des échantillons :**

L'étape d'extraction 30 peut extraire plusieurs voxels-modèles au sein d'une même structure. Aussi, l'image d'agrégation résultante est peu exploitable en tant que telle, car un élément de volume appartenant à une structure est assigné de façon aléatoire à l'un des échantillons associés à cette même structure.

On dispose de $I$ échantillons, dont plusieurs peuvent se trouver au sein de la même structure. Les échantillons inclus dans la même structure doivent donc être fusionnés, afin que chaque classe fusionnée obtenue corresponde à une structure. Il convient de déterminer une mesure de similarité entre les modèles $M_i$ et $M_{i'}$ estimés sur les échantillons $i$ et $i'$. Deux échantillons $i$ et $o$ contenus dans la même structure seront caractérisés par des valeurs de paramètres $\{\theta_u(i)\}_{1 \geq u \leq U}$ et $\{\theta_u(i')\}_{1 \leq u \leq U}$ proches, car ces derniers sont propres à la structure qu'ils représentent. La similarité entre les échantillons $i$ et $i'$ pourra être déterminée soit directement à partir des paramètres $\{\theta_u\}_{1 \leq u \leq U}$ définissant le modèle, soit en tant qu'erreur de reconstruction de l'échantillon $i$ (respectivement $i'$) par le modèle de l'échantillon $i'$ (respectivement $i$), soit par des primitives extraites des cinétiques reconstruites par les modèles (maximum, moment de réalisation du maximum, etc.), soit encore par une fonction incorporant ces différents éléments.

La fusion réunira prioritairement les échantillons dont la similarité entre leurs modèles respectifs est maximale. Une fusion d'échantillons sera considérée comme un nouvel échantillon, ce qui permettra de réitérer l'étape de fusion.

Les paramètres $\{\theta_u(i)\}_{1 \leq u \leq U}$ et $\{\theta_u(O)\}_{1 \leq u \leq U}$ des modèles de deux structures $i$ et $o$ étant différents en termes de paramètres, ils seront fusionnés après les fusions des échantillons représentant la même structure. Il existe donc théoriquement une étape de fusion après laquelle à chaque structure détectée correspond exactement à un groupe d'échantillons fusionnés. Cette étape de fusion particulière peut par exemple coïncider avec une soudaine diminution de la mesure de similarité entre les modèles des deux échantillons fusionnés. Cette étape optimale de fusion peut donc être déterminée par une étude de la mesure de similarité ou encore de ses dérivées par rapport à l'étape de fusion.

➤ **Etape 60 de représentation hiérarchique des organes :**

L'attribution d'un même label aux régions fusionnées par l'étape de fusion 50b permet théoriquement d'obtenir une image de classement dans laquelle chaque structure correspond exactement à une fusion d'échantillons, et ce pour une étape de fusion identique pour chaque structure étudiée.

Toutefois, les réponses de certaines structures au phénomène peuvent être très proches sans être identiques, ce qui n'assurerait pas que les échantillons correspondant à des structures différentes soient fusionnés après ceux qui correspondent à la même structure. Il n'existera alors pas de « niveau de fusion » tel que chaque structure - ou chaque groupe de structures présentant exactement la même réponse au phénomène observé - corresponde exactement à un échantillon.

Il est aussi envisageable que ce niveau de fusion optimal existe, mais qu'il soit impossible à déterminer à partir de l'étude de la mesure de similarité entre modèles de cinétiques d'échantillons à fusionner. Afin de pallier ces défauts, la présente méthode propose de représenter l'image sous la forme d'un arbre dont chaque feuille représente un agrégat obtenu lors de l'étape 50a de classement des cinétiques. Chaque noeud de l'arbre représente une fusion ou un agrégat issue d'une fusion, et les feuilles de fils représentent les agrégats qui composent cette dernière.

Par une reconnaissance de structures utilisant un algorithme automatique ou bien encore par une reconnaissance manuelle, cette étape 60 se propose de déterminer le noeud de l'arbre correspondant exactement à une structure donnée. L'identification de chaque structure dans l'arbre permet d'obtenir une image de classement telle qu'à une structure corresponde une fusion d'échantillons, mais à une étape de fusion dépendant de la structure étudiée.

➤ **Etape 70 de séparation des composantes connexes :**

D'après l'hypothèse H3 précitée, il est possible que deux structures aient la même réponse au phénomène étudié, mais soient non connexes. Les étapes 50a de classement et 50b de fusion, voire l'étape 60 de représentation hiérarchique, peuvent donc réunir sous un même label de telles régions.

Une simple séparation des composantes connexes, classe par classe, permet de séparer les structures qui présentent la même réponse au phénomène mais qui sont non connexes, et permet d'obtenir une image de segmentation, résultat final de la méthode selon l'invention.

**Exemple de mise en oeuvre de la méthode selon l'invention pour la segmentation d'images issues d'une Tomographie par Emission de Positions (TEP)**

Bien que la problématique corps entier du petit animal, tel qu'un rat, soit abordée dans cet exemple de réalisation, ce dernier pourrait tout aussi bien être appliqué à des images de zones spécifiques, comme le cerveau, et chez d'autres animaux ou chez l'homme. En effet, la segmentation du corps entier est particulièrement problématique du fait des importants mouvements physiologiques affectant l'image à traiter. On a choisi une segmentation de type corps entier et chez un petit animal pour montrer le bon fonctionnement de la méthode malgré la petite taille des organes à segmenter et malgré l'occurrence de mouvements physiologiques.

On montrera ici que le modèle de cinétique choisi vérifie les hypothèses de travail du principe général, et l'on décrira la méthode suivie comme une application directe du principe général énoncé ci-dessus.

**a) Etape 10 de modélisation du signal :**

➤ *Signal en TEP :*

La Tomographie par Emission de Positons permet de suivre le devenir d'une ou plusieurs molécule(s) présentant des propriétés biologiques d'intérêt, que l'on aura marquée au préalable par un isotope radioactif émetteur de positons. Ce traceur est injecté par voie intra-veineuse au sujet imagé installé dans le champ de vue de la caméra. Lors de la désintégration de l'isotope, un positon est émis qui, après avoir perdu son énergie cinétique par chocs successifs avec des électrons, s'annihile avec un électron pour donner deux photons partant dans des directions diamétralement opposées. Un système de détection composé de couronnes de détecteurs permet de détecter les photons émis et de reconstituer la ligne sur laquelle s'est produite la désintégration. A partir de ces événements, un algorithme reconstruit une image rendant compte, en chaque endroit de l'espace discrétisé, du nombre de désintégrations ayant eu lieu dans cet élément de volume.

Un pharmaco-organe peut être défini comme une structure de l'organisme dont tous les éléments présentent une réponse identique au traceur. Cela ne signifie pas pour autant que la cinétique mesurée au sein de tous les éléments de volumes d'un même organe soit forcément identique. On peut définir un modèle de cinétique $M_{i,j}$ permettant de rendre compte de la cinétique physiologique de chacun des éléments de volume du pharmaco-organe. Par cinétique physiologique, on entend le signal émis par les molécules marquées et non le signal mesuré. Ainsi, si l'on suppose le bruit additif et si l'on ignore l'effet de volume partiel, on peut décrire la cinétique $C_j$, pour un élément de *volume j* au sein d'un organe *i* comme :

$$C_j = M_{i,j} + \varepsilon_j$$

Si l'on prend en compte l'effet de volume partiel, qui mélange au voxel *j* les cinétiques physiologiques des pharmaco-organes *i(k)* au sein des voxels *k* voisins *de j*, $C_j$ s'écrit :

$$C_j = \sum_{k \in Vois(j)} \left( \beta_{j,k} \times M_{i(k),k} \right) + \varepsilon_j, \ où$$

$\beta_{j,k}$ est un coefficient de mélange déterminé par la fonction de réponse de l'imageur TEP.

Le modèle de cinétique physiologique à un seul organe devra être choisi de façon à ce que, pour deux pharmaco-organes *i* et *o* présentant des réponses différentes au traceur, le nombre d'éléments de volume *j* tels que $M_{i,j}(t) = M_{o,j}(t)$ soit faible.

> ➢ *Modélisation compartimentale à un seul traceur :*

Modélisons les processus physiologiques d'interaction entre le traceur et un organe *i* donné par trois compartiments et quatre paramètres. Ce traceur traverse la barrière cellulaire avec une constante de vitesse $K_1$. Il peut de la même façon repasser la barrière pour retourner dans le sang avec une constante de vitesse $K_2$. Il est transformé dans les cellules avec une constante de vitesse $K_3$. Si cette transformation est réversible la transformation inverse est caractérisée par une constante de vitesse $K_4$. Les trois compartiments étudiés sont $Cp$ le compartiment plasma-sérum, $Cf_i$ le compartiment libre de l'organe *i* et $Cb_i$ le compartiment lié de l'organe *i*. Soit $Cp(t)$, $Cf_i(t)$ et $Cb_i(t)$ les concentrations du traceur dans chacun de ces trois compartiments. Ce processus peut être schématisé comme illustré à la figure 3a.

Dans ce modèle, la variation de concentration des composés dans les différents compartiments s'écrit :

$$\frac{dCf_i(t)}{dt} = K_1 Cp(t) - (k_2 + k_3) Cf_i(t) + k_4 Cb_i(t)$$

$$\frac{dCb_i(t)}{dt} = k_3 Cf_i(t) - k_4 Cb_i(t)$$

Dans un premier temps, plaçons-nous dans le cas où un seul traceur est injecté à l'organisme. Soit $Ct_i(t)$ la concentration d'un traceur dans le tissu dans l'organe *i*, $Ct_i(t)$ s'écrit :

$$Ct_i(t) = Cf_i(t) + Cb_i(t)$$

$Cp(t)$, la concentration du traceur dans le sérum plasmatique, n'est pas accessible directement. En effet, le compartiment du sérum plasmatique est inclus dans un autre compartiment, le compartiment artériel $Ca(t)$. Soit donc $Ca(t)$ la concentration du traceur dans le compartiment artériel. Comme illustré à la figure 3b, le compartiment comprend un compartiment $C_{cell}$ contenant les cellules du sang, ainsi que le compartiment plasmatique $C_{plasma}$, comprenant lui-même le compartiment du sérum plasmatique $Cp$ - entrant dans le modèle compartimental choisi - et les protéines du sang $C_{prot}$.

Le modèle $M_{i,j}(t)$ cinétique du signal TEP au sein d'un élément de volume j donné compris dans l'organe i s'écrit :

$$M_{i,j}(t) = \left(\left(1 - Vb_{i,j}\right) \times Ct_i(t) + Vb_{i,j} \times Ca(t)\right)$$

où $Vb_{i,j}$ est le rapport de volume artériel au sein de l'élément de volume j de l'organe i.

$Ca(t)$ est homogène dans l'organisme entier et $Ct(t)$ est homogène au sein d'un pharmaco-organe sain donné. Dans le cas d'un pharmaco-organe au fonctionnement pathologique, celui-ci peut être séparé en une zone saine et une zone pathologique. Ces zones se comportant différemment par rapport au traceur, elles sont pharmaco-cinétiquement différentes et seront considérées comme deux pharmaco-organes différents.

$Ca(t)$ et $Ct(t)$ étant donc homogènes au sein d'un pharmaco-organe donnés, $M_{i,j}(t)$ appartient au segment $Ca(t)Ct_i(t)$, et donc par extension à la droite $Ca(t)Ct_i(t)$, lorsque j varie tout en restant au sein de l'organe i.

On peut donc modéliser la cinétique physiologique au sein d'un organe par une droite $D_i$ dans l'espace des cinétiques passant par $Ca(t)$ et $Ct_i(t)$. La connaissance des modèles $M_{i,j}(t)$ de cinétiques physiologiques pour quelques organes i devrait donc permettre de calculer Ca(t), comme l'intersection de toutes les droites $D_i$. En revanche, $Ct_i(t)$ ne peut être explicitement calculée.

➢ *Modélisation compartimentale à plusieurs traceurs :*

On montre aussi (voir l'annexe A joint), que lorsque plusieurs traceurs s - au nombre de S - sont injectés à l'organisme, le signal résultant $S_{i,s,j}(t)$ dans l'élément de volume j décrit toujours une droite dans l'espace des cinétiques, cette fois la droite passant par $\sum_{s=1}^{S} Ct_{i,s}(t)$ et $\sum_{s=1}^{S} Ca_s(t)$, à condition que ces différents traceurs n'interagissent pas entre eux.

Le modèle de cinétique reste donc valide dans le cadre du modèle compartimental à quatre paramètres, quel que soit le nombre de traceurs « indépendants » injectés.

➢ *Mélanges locaux de cinétiques :*

**- Impact de l'effet de volume partiel sur le signal :**

L'effet de volume partiel peut être modélisé [Frouin, 2002] comme un lissage de l'image. Le signal $S_j$ mesurée dans l'image au sein d'un élément de volume j est donc une combinaison linéaire des cinétiques $S_k$ des éléments de volume k voisins. Lorsque plusieurs traceurs sont injectés :

$$S_j(t) = \sum_{s=1}^{S} \left[ \sum_{k \in Voisinage(j)} \left(\beta_{j,k} \times M_{i(k),s,k}(t)\right) \right]$$

où $\beta_{j,k}$ vérifie $\sum_{k \in Voisinage(j)} \beta_{j,k} = 1$.

$S_j(t)$ appartient toujours à une droite de l'espace des cinétiques, passant par $\sum_{s=1}^{S} Ct_{i,s}(t)$ et $\sum_{s=1}^{S} Ca_s(t)$ (voir Annexe A) lorsque le voisinage $V_j$ de j dont les cinétiques physiologiques contribuent au signal $S_j$ est inclus dans l'organe i. Lorsque ce voisinage contient plusieurs organes modélisés par des droites différentes, $S_j(t)$ appartient à un sous espace de l'espace des cinétiques centré en Ca(t) et de dimension strictement supérieure à 1.

**- Impact des mouvements physiologiques sur le signal :**

Les mouvements physiologiques non périodiques ou dont la période ne peut être négligée par rapport à la durée d'acquisition d'une image de la série génèrent des cinétiques non interprétables. En effet, à chaque *instant t, j* ne contient pas les mêmes sites physiologiques.

Les cinétiques des régions subissant un mouvement physiologique périodique de période négligeable par rapport à la durée d'acquisition d'une image de la série, comme la respiration ou les battements du coeur, redeviennent interprétables (voir annexe A).

**- Conclusion :**

On a un modèle de cinétique à un unique pharmaco-organe.

Soit un volume A fixe dans le repère de la caméra. Soit un volume *B* fixe dans le repère d'un l'organe, placé au sein du pharmaco-organe *i*, de façon à ce que le voisinage de *B* (*Vois(B)*) sur lequel s'étend l'effet de volume partiel soit lui aussi inclus dans le pharmaco-organe *i*. Supposons A et *B* superposés au temps $t_o$ : A ne contient en $t_0$ que du signal issu de l'organe *i*.

Soit *Vois(A)* le voisinage de A sur lequel s'étend l'effet de volume partiel. Lors d'un mouvement physiologique périodique de faible période, *B* se déplace par rapport à A, si bien que les deux volumes ne sont plus superposés. Cependant, le modèle $M_{i,j}(t)$ à un unique organe *i* est valide au sein de A, fixe dans le référentiel de la caméra, si et seulement si *Vois(A)* reste inclus dans l'organe *i* lors de la période du mouvement physiologique (voir Annexe A).

➢ *Vérificafion des hypothèses du principe général :*

**- Partition de l'espace en pharmaco-organes :**

L'organisme peut être séparé en un nombre fini de pharmaco-organes, correspondant chacun à un organe, à un groupe d'organes connexes ou à une sous-partie d'un organe. Par définition du modèle compartimental, chaque pharmaco-organe est caractérisé par une cinétique artérielle et une cinétique tissulaire toutes deux homogènes dans le pharmaco-organe. L'hypothèse H1 précitée est donc vérifiée.

**- Estimation des paramètres du modèle :**

Le modèle $M_{i,j}$ est une droite dans l'espace des cinétiques. Il peut être estimé au moyen d'une analyse en composantes principales opérée sur les cinétiques des éléments de volume de l'échantillon et centrée sur la moyenne du signal au sein de l'échantillon. L'hypothèse H2 précitée est donc vérifiée.

**- Réponses différentes des pharmaco-organes au traceur:**

Un pharmaco-organe est défini par des cinétiques tissulaire et artérielle homogènes en son sein. En revanche, rien n'empêche deux pharmaco-organes voisins *i* et *o* de montrer une réponse identique au traceur, ou bien telle que $Ct_o$ appartienne à la droite $CaCt_i$. Rien n'assure donc que l'hypothèse H3 précitée soit vérifiée.

Considérons cependant comme un seul et unique pharmaco-organe deux pharmaco-organes voisins présentant des réponses identiques. L'hypothèse H3 est alors vérifiée. La méthode sera toutefois incapable de séparer les deux parties de ce pharmaco-organe.

**- Bruit de mesure et de reconstruction**

En TEP, pour des méthodes de reconstruction itératives ou analytiques, le bruit peut être considéré comme additif gaussien, conformément à l'hypothèse H4 précitée.

**- Mélange local de cinétiques :**

Comme nous venons de le voir, l'effet de volume partiel et les mouvements physiologiques génèrent au sein d'un élément de volume donné un mélange local de cinétiques de pharmaco-organes voisins. Dans le cas de l'effet de volume partiel, nous venons de voir que le mélange de cinétique, opéré au sein d'un élément de volume *j* donné, est un barycentre des modèles de cinétiques physiologiques aux éléments de volume voisin. De même, les mouvements physiologiques périodiques de faible période opèrent au sein du voxel *j* un barycentre des modèles de cinétiques des pharmaco-organes qui ont traversé *j* au cours du mouvement. Plaçons-nous dans le cas où tous les voisins de *j* appartiennent au pharmaco-organe *i*. $M_i$ étant une droite $D_i$ dans l'espace des cinétiques, tout barycentre de cinétiques appartenant à la droite $D_i$ appartient à la droite $D_i$. La contrainte C1 précitée est donc vérifiée.

**b) Etape 20 de pré-segmentation :**

Comme indiqué ci-dessus dans l'exposé général de la méthode selon l'invention, les zones présentant une absence de signal nuisent à la rapidité de l'algorithme et à la qualité des résultats. Ces zones sans signal correspondent en TEP à l'extérieur de l'organisme : le fond.

L'image TEP peut donc être découpée en deux zones, l'une représentant l'organisme et l'autre le fond. Les cinétiques du fond sont caractérisées par un faible signal, engendrant un grand nombre de valeurs nulles ou négatives, qui, étant non physiologiques, ne peuvent représenter une cinétique organique. Ces dernières, caractérisées par une activité en générale importante par rapport au bruit, s'annulent rarement et ont une moyenne plus forte que les cinétiques du fond.

L'analyse d'histogramme est une méthode rapide permettant de déterminer des seuils pour séparer plusieurs populations d'éléments de volume. L'histogramme représente le nombre d'éléments de volume ayant une activité donnée, ou plus généralement dont l'activité appartenant à un intervalle donné. Les intervalles sont jointifs et souvent pris de même largeur.

> *Méthodes usuelles :*

En TEP, un histogramme de l'image de transmission permettra de segmenter aisément l'organisme, mais dissociera difficilement ce dernier des objets présents dans le champ de vue : lit, dispositif pour rongeurs, éventuel respirateur, etc.

L'image usuellement utilisée pour réaliser un histogramme à partir de l'émission est la séquence d'images elle-même, moyennée sur le temps ou bien dont une image particulière a été extraite. Sur un tel histogramme, le premier pic représente le fond ou une partie de celui-ci.

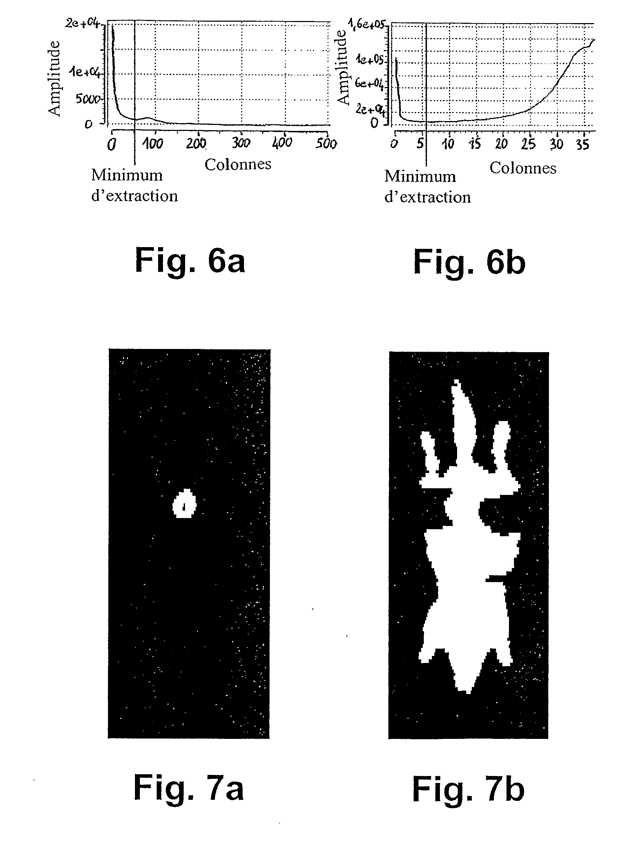

Du fait de la grande disparité des valeurs au sein de l'image, une analyse par histogramme des valeurs de l'image, si elle permet de détecter un pic correspondant au fond, n'assure pas la détermination d'un seuil séparant ce fond du reste de l'image (voir figures 6a, 6b et 7a, 7b).

> *Histogramme du nombre de valeurs négatives des cinétiques :*

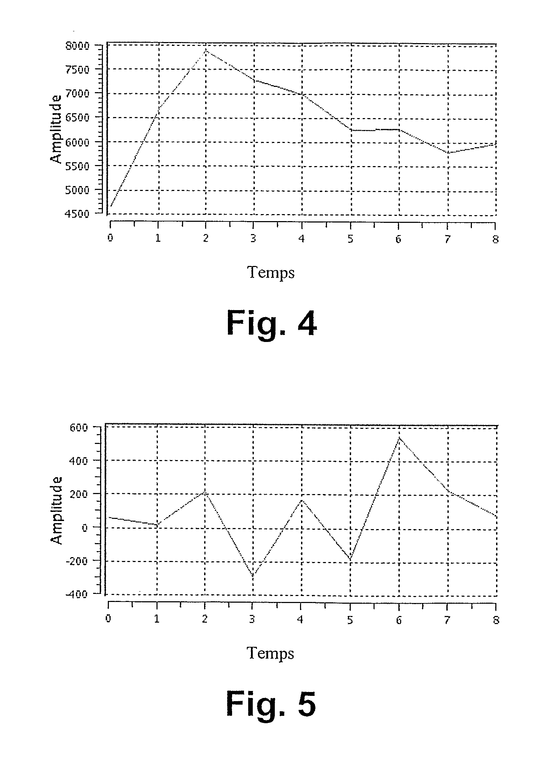

En revanche, l'histogramme du nombre de valeurs négatives ou nulles de la cinétique présente deux pics - appelés modes - distincts, l'un pour l'organisme, qui présente une radioactivité non négligeable par rapport au bruit, et l'autre pour l'air entourant l'organisme, qui présente une radioactivité négligeable par rapport au bruit. La cinétique d'un élément de volume inclus dans l'organisme présentera un faible nombre de valeurs nulles ou négatives, tandis que la cinétique d'un élément de volume du fond présentera de nombreuses valeurs négatives, comme le montrent les graphiques des figures 4 et 5 (respectivement des exemples de cinétiques d'un élément de volume inclus dans l'organisme et d'un élément de volume extérieur à l'organisme).

Soit $n_j$ le nombre de valeurs négatives prises par une cinétique $C_j$ donnée. On s'attend à ce que $n_j$ soit grand dans le fond et petit dans l'organisme. L'histogramme des valeurs prises par $n_j$ dans l'image présente deux modes, l'un correspondant au fond ($Mode_{fond}$) et l'autre à l'organisme ($Mode_{org}$). Celui-ci présentant généralement peu d'involutions (corps entier, crâne), sa surface représente un nombre d'éléments de volume négligeable par rapport à son volume et au volume du fond. Cet histogramme présente donc au moins un minimum d'indice *nmin* situé entre $Mode_{org}$ et $Mode_{fond}$. Une analyse multi-échelle de l'histogramme [Mangin, 1998] assure l'extraction du minimum d'indice *nmin* de l'histogramme entre les deux modes $Mode_{org}$ et $Mode_{fond}$. Tous les éléments de *volume j* de l'image qui sont tels que $n_j <$ *nmin* seront considérés par la suite dans l'organisme, et les autres hors de l'organisme. Une simple ouverture morphologique sur le masque obtenu et une extraction de la plus grande composante connexe permettent l'élimination de points du fond attribués à tort à l'organisme.

A l'intérieur de l'organisme, certains organes, comme les poumons, peuvent présenter une très faible activité, et être exclus de la zone d'intérêt. Afin de pallier cet inconvénient, on ajoute à la zone d'intérêt toute zone de fond non connexe avec les bords de l'image.

**c) Etape 30 d'extraction des voxels-modèles :**

Dans la suite nous prendrons comme voisinage $V_j$ d'un élément de volume $j$ la boule $B_{j,r}$ de rayon $r$. De plus, nous ne détaillerons l'exemple de réalisation que dans le cas d'une image reconstruite par une méthode analytique, pour laquelle le bruit au sein de l'image peut être supposé stationnaire dans l'espace. La variation de cet exemple de réalisation dans le cas d'une image reconstruite par une méthode itérative, pour laquelle la variance du bruit est liée au signal, est donné dans l'annexe C jointe.

Selon le modèle $M_{i,j}(t)$ choisi précédemment pour rendre compte de la cinétique physiologique au sein du pharmaco-organe $i$ et pour tous les éléments de *volume j* inclus dans $i$, $M_{i,j}(t)$ appartient à une droite dans l'espace des cinétiques. Plaçons-nous dans le cas d'une reconstruction analytique, pour laquelle le bruit est stationnaire dans l'espace.

Supposons l'hypothèse H5 vérifiée et que le bruit au sein d'une image donnée de la séquence, non corrigée de la décroissance radioactive, dépende essentiellement de la durée d'acquisition de cette image. Supposons cette

dépendance linéaire :

$\sigma(t) = \xi / \Delta_t$, où $\xi$ est une quantité stationnaire dans le temps et l'espace et $\Delta_t$ est la durée d'acquisition de l'image $t$ de la séquence.

Soit $C_k^{'}(t) = C_k(t) \times \Delta_t$ la cinétique à l'élément de volume k, dont la valeur au temps $t$ a été pondérée par la durée d'acquisition de l'image $t$ de la séquence. L'écart-type du bruit de l'ensemble des cinétiques $C_k^{'}(t)$ ainsi pondérées, est égal à $\xi$. L'étude des cinétiques $C_k^{'}(t)$ permet de s'affranchir de la non stationnarité temporelle du bruit.

➢ *Indicateur de l'intérieur des organes :*

L'hypothèse $H0_{vj}$ précitée est équivalente, dans le cas présent, à une hypothèse selon laquelle le signal au sein de la boule $B_{j,r}$ centrée au voxel $j$ et de rayon $r$ peut être modélisé par une droite dans l'espace des cinétiques, plus du bruit additif.

Considérons non plus les cinétiques, mais les cinétiques $C_k^{'}(t) = C_k(t) \times \Delta_t$, le bruit pouvant être de plus supposé gaussien. Soit une analyse en composante principale centrée (ACP) réalisée sur les cinétiques $C_k^{'}(t)$ au sein de la boule $B_{j,r}$. Soit $\{\lambda_{j,l}\}_{1 \leq l \leq T}$ les valeurs propres de cette ACP classées par ordre décroissant et $\{e_{j,l}\}_{1 \leq l \leq T}$ les vecteurs propres associés à ces valeurs propres. Soit enfin $\mu_j^{'}$ la moyenne des cinétiques au sein de $B_{j,r}$ sur laquelle est centrée l'ACP. Nous utiliserons comme indicateur de validité de l'hypothèse $H0_{vj}$ la variance $\Gamma_{vj}$ des données non reconstruites par le premier vecteur propre de l'ACP relativement au signal :

$$\Gamma_{vj} = \lambda_j{}^2 / \left\| \mu'_j \right\|^2,$$

où

$$\lambda_j^2 = \frac{1}{T-1} \sum_{l=2}^{T} \lambda_{j,l}^2.$$

$\lambda_j^2$ est la variance du signal non reconstruit au sein de $B_{j,r}$ par l'ACP (voir l'Annexe B jointe pour la justification).
<u>Discussion</u> : La mesure $\Gamma_{vj}$ de validité de l'hypothèse $H0_{vj}$ est une approximation de la moyenne des proportions de signal non reconstruit au sein de $B_{j,r}$. Cette approximation présente l'avantage d'être peu bruitée, d'être un bon indicateur de l'inclusion de $B_{j,r}$ dans un pharmaco-organe, mais aussi de détecter les points dont le voisinage est, en l'absence de points complètement inclus dans un organe donné, au moins majoritairement inclus dans ce dernier. Ainsi, des pharmaco-organes ne présentant aucune cinétique non polluée par les signaux physiologiques des organes voisins peuvent néanmoins être détectés.

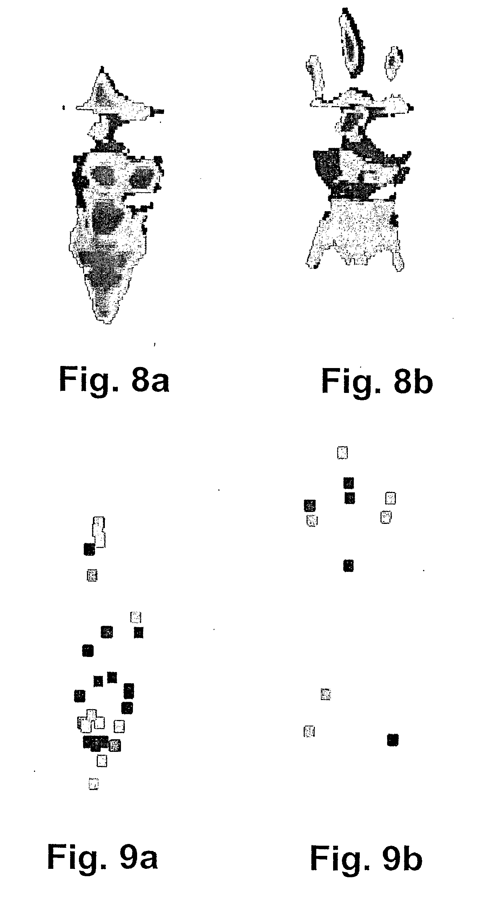

Les figures 8a et 8b illustrent des cartes des IR'$_j$ dans le cas d'une image reconstruite par une méthode itérative (la figure 8a étant une coupe coronale passant par le coeur et les reins du rat, et la figure 8b une coupe coronale passant par la vessie du rat).

➢ *Extraction des voxels-modèles :*

Le rayon r de la boule $B_{j,r}$ est choisi de façon à ce que le nombre de cinétiques qu'elle contient suffise au calcul de l'ACP. Il sera pris par exemple égal à n x 3.
Nous calculons donc la carte des $\Gamma_{vj}$ dont les minima locaux correspondent à des points situés au coeur des organes. Le voisinage de ces points est peu affecté par l'effet de volume partiel. Ces minima locaux, qui peuvent être multiples au sein du même organe, sont extraits automatiquement. Afin d'éliminer les maxima locaux dus au bruit, l'image des $\Gamma_{vj}$ est filtrée par un filtre gaussien dont la variance, choisie par un algorithme itératif, est juste suffisante pour que le nombre de minima locaux extrait soit inférieur à un seuil fixé a *priori*. Ce seuil sera fixé relativement haut pour éviter qu'un trop fort lissage ne déplace ou ne fasse disparaître des minima locaux correspondant à l'inclusion de la boule $B_{j,r}$ *dans* l'organe.
Les figures 9a et 9b illustrent les minima locaux de la carte des $\Gamma_{vj}$ par extraction des voxels-modèles (la figure 9a étant une coupe coronale passant par le coeur et les reins du rat, et la figure 9b une coupe coronale passant par la vessie du rat).

**d) Etape 40 d'optimisation des échantillons :**

La géométrie choisie en forme de boule $B_{j,r}$ peut ne pas être appropriée à certains pharmaco-organes aux formes allongées, plates ou en forme de « pelure d'oignon ». Nous allons donc définir un échantillon de points connexes à $j$ minimisant un critère de non-reconstruction par le modèle à un organe calculé sur les cinétiques des éléments de volume inclus dans $B_{j,r}$ (deux points connexes sont tels qu'ils appartiennent à la même région et qu'il existe un chemin inclus dans cette région que les relie).

La cinétique corrigée $C_k^{'}(t) = C_k(t) \times \Delta_t$ à l'élément de volume $k$ s'écrit :

$$C_k^{'}(t) = \left(S_k(t) + \varepsilon_k(t)\right) \times \Delta_t .$$

Nous utiliserons ici la méthode par croissance de région proposée dans le principe général afin d'optimiser la forme des échantillons $\psi_i$. Cette croissance de région, dont l'échantillon initial est formé des cinétiques des éléments de volume inclus dans $B_{j,r}$ utilisera comme mesure de similarité entre une cinétique $C_k^{'}(t)$ et l'échantillon $\psi_i$ l'inverse de la proportion de signal de $C_k^{'}(t)$ non reconstruit par le vecteur propre associé à la plus grande valeur propre de l'ACP centrée calculée sur $\psi_i$. Soit p'$_{j,k}$ cette proportion de signal non reconstruite :

$$p'_{j,k}(t) = \frac{\sum_{t=1}^{T}\left(\varepsilon_{j,k}(t) \times \dfrac{\Delta_t}{\max\limits_{1 \le t' \le T}(\Delta_{t'})}\right)^2}{\left\|C_k^{'}(t)\right\|^2} = \frac{\left\|\varepsilon_{j,k}^{'}(t)\right\|^2}{\left\|C_k^{'}(t)\right\|^2}$$

est de l'ordre de $\dfrac{\xi^2}{\left\|C_k^{'}(t)\right\|^2} \ll 1$ au sein de l'organe, et de l'ordre de 1 en dehors de l'organe, où $\varepsilon_{j,k}(t)$ contient encore du signal non reconstruit. Elle est aussi grande en bordure de l'organisme, car $\left\|C_k^{'}(t)\right\|^2$ y décroît fortement alors que $\varepsilon_{j,k}(t)$ y reste de l'ordre de $\xi$. Elle permet donc non seulement d'écarter les cinétiques trop éloignées du modèle, mais encore d'éviter, dans le cas d'un organe $i$ présentant une forte activité polluée par les activités d'organes voisins, de préférer une cinétique faible à une cinétique de l'organe $i$.

A chaque étape de raffinement de l'échantillon, une ACP est donc calculée sur $\psi_i$, puis une croissance de région par propagation de front est opérée à partir de l'élément de volume $j$, seul constituant de la région au départ. A chaque étape, l'élément de volume $k$ du front qui minimise p'$_{j,k}(t)$ est agrégé à l'échantillon $i$. L'introduction d'un modèle d'Ising permet de régulariser les contours des échantillons obtenus. La taille finale de chaque échantillon est prise égale au nombre d'éléments de volume inclus dans la boule $B_{i,r}$.

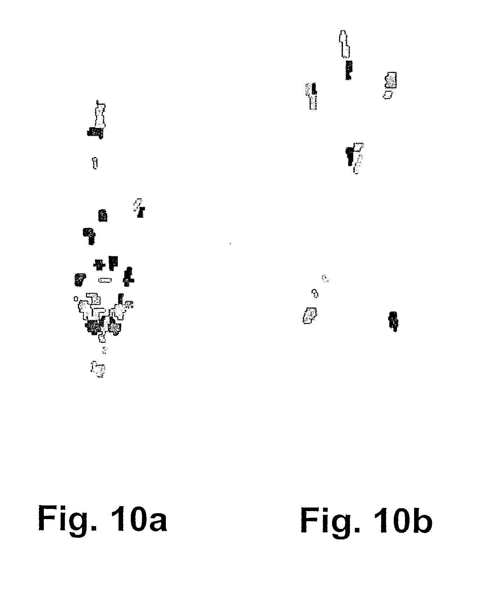

Les figures 10a et 10b illustrent le résultat de la croissance de région sur les échantillons (la figure 10a étant une coupe coronale passant par le coeur et les reins du rat, et la figure 10b une coupe coronale passant par la vessie du rat).

**e) Etape 50a de classement des cinétiques :**

Le bruit des cinétiques $C_k$ est considéré comme stationnaire dans l'espace, et le bruit des cinétiques $C_k^{'}$ est considéré comme stationnaire dans l'espace et le temps. La probabilité a posteriori de la donnée $C_k^{'}$ connaissant le modèle $M_{i,k}^{'}$ s'écrit :

$$p(C_k^{'} / M_{i,k}^{'}) = \gamma \times e^{-\frac{\left\|C_k^{'} - M_{i,k}^{'}\right\|^2}{2 \times T \times \xi^2}},$$

où $\gamma$ est un scalaire indépendant de l'organe $i$ comme de l'élément de volume $k$ considéré.

Une fois les modèles $M_{i,k}^{'}$ calculés à partir des cinétiques $C_k^{'}$ au sein de chaque échantillon par une analyse en composantes principales dont on ne conservera que le vecteur propre associé à la plus grande valeur propre, la maximisation de $p(C_k^{'} / M_{i,k}^{'})$ revient à trouver l'échantillon $i$ qui minimise $\left\|C_k^{'} - M_{i,k}^{'}\right\|^2$.

On attribue donc à l'élément de volume $k$ le label $o$ tel que :

$$o = \arg\min_{i} \left\|C_k^{'} - M_{i,k}^{'}\right\|^2.$$

### *Régularisation du classement des cinétiques :*

Afin de limiter les chances d'une agrégation erronée de $k$ du fait du bruit, on définit le processus de segmentation de l'élément de volume $k$ non plus comme :

$$o = \arg\min_{i} \left\|C_k^{'} - M_{i,k}^{'}\right\|^2,$$

mais comme

$$o = \arg\max_{i} \left( \sum_{j \in Vois(k)} \left[ \beta_{j,k} \times e^{-\frac{\left\|C_k^{'} - M_{i,k}^{'}\right\|^2}{2 \times T \times \xi^2}} \right] \right),$$

où *Vois(k)* désigne l'ensemble des éléments de volumes voisins de l'élément de volume $k$, et $\beta_{j,k}$ un facteur de pondération dépendant de la distance entre les éléments de volume $j$ et $k$.

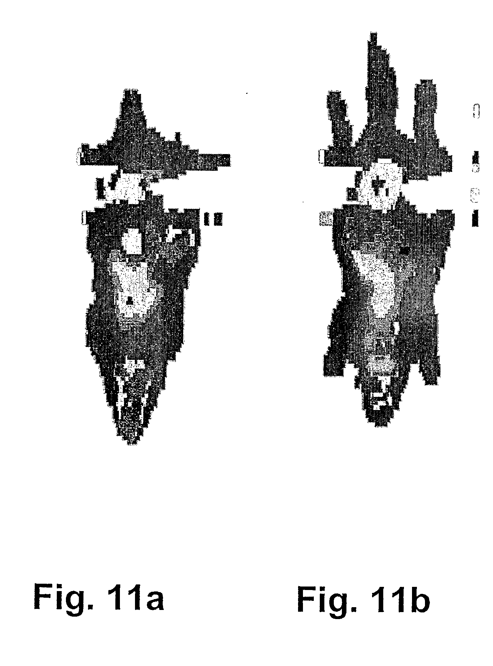

Les figures 11a et 11b illustrent le classement des cinétiques en 100 classes (la figure 11 a étant une coupe coronale passant par le coeur et les reins du rat, et la figure 11b une coupe coronale passant par la vessie du rat).

### f) <u>Etape 50b de fusion des échantillons</u> :

L'étape d'extraction des voxels-modèles peut extraire plusieurs voxels-modèles au sein d'un organe donné. Aussi l'image de segmentation résultante est peu exploitable en tant que telle, car un élément de volume appartenant à un pharmaco-organe est assigné de façon aléatoire à l'un des échantillons associés à ce même pharmaco-organe.

Nous disposons de I échantillons, dont plusieurs peuvent se trouver au sein du même organe. Une classification ascendante hiérarchique (HL) [Jain, 1988] permet de fusionner les échantillons.

Deux échantillons ont de bonne chance de représenter le même organe si les cinétiques de l'un sont bien reconstruites par le modèle à un seul organe estimé sur l'autre, et si les cinétiques moyennes de chacun d'entre eux présentent des pics d'activité similaires (en position et en intensité).

Dans le cas d'une reconstruction analytique, la variance du bruit associé aux cinétiques $C_k^{'}(t) = C_k(t) \times \Delta_t$ est égal à $\xi^2$, stationnaire dans l'espace et le temps. Soit $\lambda_i^{'2} = \frac{1}{T-1} \sum_{l=2}^{T} \lambda_{i,l}^{'2}$ la moyenne des T-1 plus petites valeurs propres d'une ACP calculée sur les cinétiques $C_k^{'}(t)$ au sein de l'échantillon $i$. $\xi^2$ peut être estimée comme la moyenne des $\lambda_i^{'2}$ sur les échantillons : $\xi^2 = \frac{1}{I} \sum_{i=2}^{I} \lambda_i^{'2}$.

La fonction de coût de fusion de deux échantillons a et b peut s'écrire ainsi :

$$C_{a,b} = \beta \times \max\left[\frac{1}{N} \times \sum_{k \in a} \frac{\left\|\varepsilon'_{b,k}\right\|^2}{\xi^2}, \frac{1}{N} \times \sum_{k \in b} \frac{\left\|\varepsilon'_{a,k}\right\|^2}{\xi^2}\right]$$

$$+ \delta \times \left(1 - \min\left[\left\|\mu_a\right\|_\infty / \left\|\mu_b\right\|_\infty, \left\|\mu_b\right\|_\infty / \left\|\mu_a\right\|_\infty\right]\right) + \gamma \times \left(1 - \min\left[\frac{t\max_b}{t\max_a}, \frac{t\max_a}{t\max_b}\right]\right),$$

$t\max_a$ étant l'instant où $\mu_a$ est maximum. On notera que les écarts de phase des cinétiques sont plus fortement pénalisés dans les temps précoces, caractérisés par des cinétiques rapides, qu'aux temps tardifs, caractérisés par des cinétiques lentes. Ce dernier critère permet de dissocier deux pharmaco-organes aux cinétiques proches, mais dont les modèles de cinétiques présentent un léger déphasage.

Les poids $\beta$, $\delta$ et $\gamma$ sont fixés de façon heuristique à $\beta$ = 0,5, $\delta$ = 0,25 et $\gamma$ = 0,5.

Entre deux groupes d'échantillons $G_a$ et $G_b$ pouvant contenir chacun plusieurs échantillons, on définit la fonction de coût suivante $C_{Ga,Gb} = \max\limits_{a \in ga, b \in gb}\left(C_{a,b}\right)$, dont le calcul est extrêmement rapide, une fois $C_{a,b}$ calculé pour tout couple (a, b).

♦ *Initialisation*

Initialement, chaque échantillon forme un groupe au sein duquel il se trouve seul. Le coût de fusion de chaque couple d'échantillon est calculé.

♦ *Etape de fusion*

A chaque étape de la classification ascendante hiérarchique, les deux groupes d'échantillons $G_a$ et $G_b$ pour lequel le coût de fusion $C_{Ga, Gb}$ est minimum sont fusionnés en un nouveau groupe $G_c$.

♦ *Fin de l'algorithme*

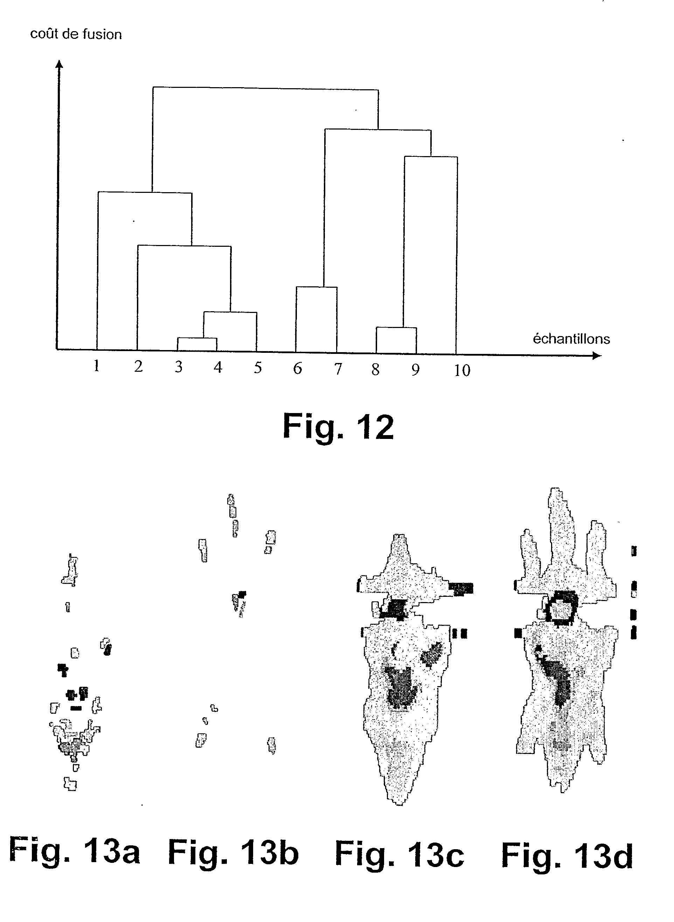

Le processus cesse lorsque tous les échantillons ont été fusionnés. La séquence des fusions entre échantillons est conservée sous la forme d'un arbre illustré à la figure 12.

Le niveau $E$ de fusion correspond à une image de classement des cinétiques entre $E$ échantillons. Afin d'obtenir une image de segmentation associée, il est nécessaire de dissocier les zones rattachées au même échantillon, mais non connexes entre elles. Cette opération est réalisée par une extraction des composantes connexes pour chaque label. On ne conservera que les composantes connexes présentant un volume supérieur au plus petit volume attendu pour un pharmaco-organe. L'affectation d'un label différent pour chacune de ces composantes connexes permet d'obtenir une partition de l'image en régions, c'est-à-dire une segmentation.

Les figures 13a et 13b résultent d'une fusion des échantillons par classification ascendante hiérarchique, et les figures 13c et 13d résultent d'une fusion des labels de l'image de classement des cinétiques par classification ascendante hiérarchique, 18 classes ayant été conservées pour chacune de ces figures (les figures 13a et 13c étant des coupes coronales passant par le coeur et les reins du rat, et les figures 13b et 13d des coupes coronales passant par la vessie du rat).

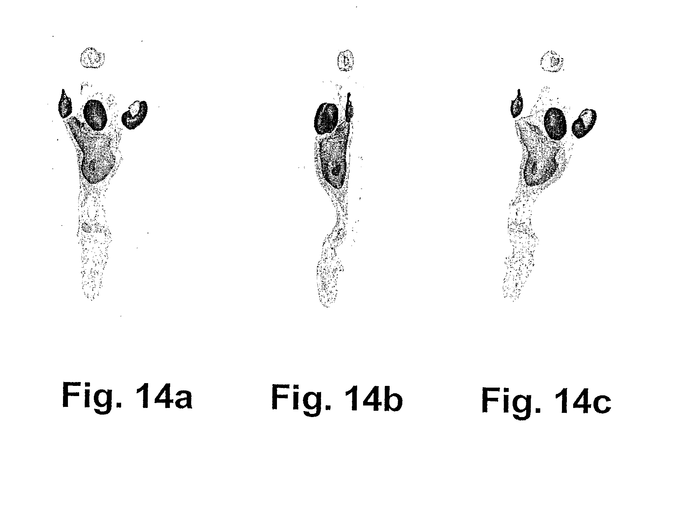

Les figures 14a, 14b et 14c sont des vues « 3D » de segmentation après fusion, respectivement coronale, sagittale et oblique.

**g) Etape 60 de représentation hiérarchique des organes** :

L'arbre de fusion des régions permet de représenter l'image segmentée non pas sous forme d'une image de label, mais d'un arbre d'images de label, dont chaque noeud ou feuille correspond à un groupe de pharmaco-organes à une étape de la fusion. Il est donc possible de suivre, moyennant un logiciel de visualisation, la fusion des diverses zones de label d'un même organe. Nous laissons le choix à l'opérateur du niveau de fusion qu'il estime optimal pour la reconnaissance d'un pharmaco-organe donné. En effet, ce niveau de fusion optimal peut être différent pour deux pharmaco-organes différents.

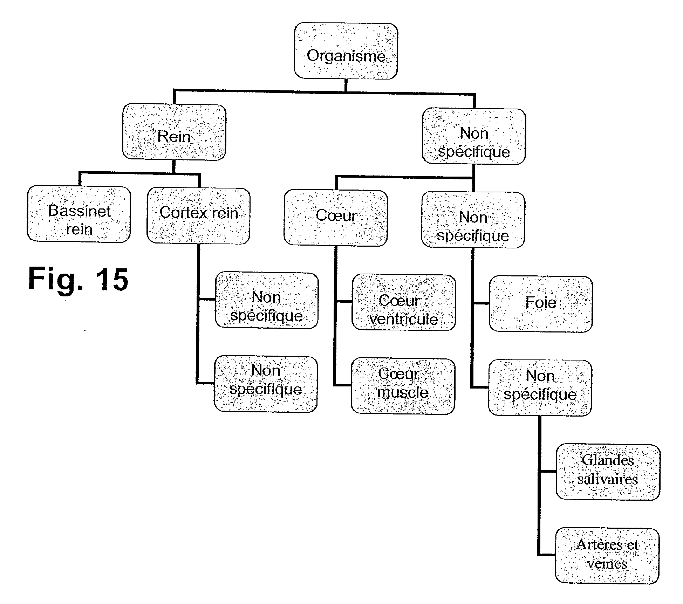

La figure 15 illustre un exemple de représentation hiérarchique des organes des rats examinés.

**h) Etape 70 de séparation des composantes connexes** :

Les composantes connexes de chaque agrégat obtenu par les étapes précédentes sont séparées par une méthode de fusion de labels. A chaque élément de volume est attribué un numéro. Les numéros de deux éléments de volumes voisins appartenant au même agrégat sont fusionnés en seul numéro, jusqu'à ce que cette opération ne soit plus possible. On obtient alors un numéro identifiant chaque composante connexe de chaque classe, ce qui constitue l'image de segmentation finale.

**Applications de la méthode selon l'invention**

**1) Applications réalisées** :

**a) Fantôme de rat** :

➢ *Fantôme numérique « MCAT» :*

Afin de valider la méthode, un fantôme numérique de rat a été réalisé à partir du fantôme « MCAT » [Segars, 2004] et simulé sur une caméra Siemens de type « HR+ ». Les organes de ce fantôme, segmentés sur une image de scanner X, sont modélisés par des surfaces « NURBS ». Outre un lissage des contours des organes, cette modélisation permet un calcul rapide de déformation. Le fantôme de rat prend donc en compte les mouvements dus à la respiration et aux battements du coeur.

Ces mouvements périodiques à haute fréquence génèrent un mélange de cinétiques des organes en mouvements. Un élément de volume de l'image, fixe dans le repère de la caméra simulée, contient donc un mélange des cinétiques des organes qui l'ont traversé lors de ces deux mouvements physiologiques.

Le remplissage de la vessie lors de l'examen, mouvement physiologique handicapant la segmentation bien plus que les mouvements périodiques, comme nous l'avons vu précédemment, n'est pas pris en compte par le fantôme « MCAT ». Nous l'avons simulé par une dilatation progressive de la zone de l'image correspondant à la vessie le long de l'examen simulé. Celui-ci comprend 37 images successives acquises sur une durée d'une minute chacune.

➢ *Détermination des cinétiques physiologiques :*

Un calcul préalable, pour chaque trame, de la probabilité d'appartenance de chaque élément de volume aux différents organes permet de recomposer rapidement une image TEP non bruitée, à partir de l'équation suivante [Kamasak, 2003] :

$$C_j(t) = \sum_{i=1}^{I} \left( p_{i,j} \times \left[ Vb_{i,j} \times Ca(t) + \left( 1 - Vb_{i,j} \right) \times Ct_i(t) \right] \right),$$

où $p_{i,j}$ est la probabilité d'appartenance de l'élément de volume $j$ à l'organe $i$, $Ca(t)$ est la cinétique vasculaire, identique pour tous les éléments de volume $j$ et tous les pharmaco-organes $i$, $Ct_i(t)$ est la cinétique tissulaire de l'organe $i$, identique pour tous les éléments de *volume j et Vb$_{i,j}$* est un scalaire représentant le ratio de volume sanguin dans l'élément de *volume j* de l'organe $i$.

Une fois la cinétique artérielle $Ca(t)$ déterminée, $Ct_i(t)$ est calculée comme une convolution de la cinétique artérielle par une somme pondérée d'exponentielles décroissantes [Kamasak, 2003].

$$Ct_i(t) = Ca(t) * \left( \sum_{w=1}^{W} \left[ a_{i,w} \times e^{-t/b_{i,w}} \right] \right)$$

Les paramètres $a_{i,w}$ et $b_{i,w}$ sont déterminés aléatoirement pour chaque fonction exponentielle $w$ et chaque pharmaco-organe $i$.

> *Projection et reconstruction :*

Cette image « 4D » est ensuite projetée analytiquement, bruitée et reconstruite par un algorithme de reconstruction soit analytique, soit itératif. Les différents phénomènes de détection ou de reconstruction tels que les coïncidences fortuites, la diffusion Compton et le « spill-over » sont aussi simulés.

> *Segmentation et critère de qualité de segmentation :*

La segmentation de l'image et la comparaison des résultats avec les probabilités d'appartenance aux divers organes permet d'établir, une fois l'identification *i(l)* faite entre le label $l_j$ et l'organe *i* correspondant, un critère global de qualité de segmentation: $\frac{1}{J} \times \left[ \sum_{j=1}^{J} \left( \frac{p_{i(l_j),j}}{\max\limits_{k}(p_{k,j})} \right) \right]$. Ce critère sera égal à 1 pour une segmentation parfaite et à 0 pour une segmentation catastrophique. On peut aussi définir un critère de détection des organes, correspondant au nombre d'organes pour lesquels une correspondance directe d'organe à organe est impossible, du fait d'une fusion ou d'une scission de deux organes dans l'image de segmentation, divisé par le nombre d'organes défini dans le fantôme.

**b) Projet « PNA » :**

Les « PNA » (« Peptidic nucleic acids ») ont montré une forte efficacité dans les stratégies anti-sens pour bloquer l'expression de gène par dégradation des ARN messagers intracellulaires. L'utilisation chez l'animal de telles molécules nécessite des connaissances sur leur comportement *in vivo.* Peu d'informations étaient disponibles sur les propriétés pharmacocinétiques de ces molécules. Les données disponibles indiquaient que ces composés étaient stables dans le plasma, excrétés dans l'urine, et que leur captation dans d'autres organes (fixation non spécifique) était très basse.

Par conséquent, ce type d'oligonucléotide semblait idéal pour développer des méthodes permettant de moduler leurs paramètres pharmacocinétiques de façon prévisible. La fonctionnalisation (modification chimique) du squelette peptidique des « PNA » a permis de synthétiser 12 dérivés possédant toujours leur activité anti-sens. C'est dérivés ont été marqué au fluor 18 et leurs pharmacocinétiques ont été analysées par imagerie TEP chez le rat. Pour chaque « PNA », quatre animaux ont été traités par injection simultanément. En appliquant la méthode de segmentation automatique d'image TEP à cette série de 12 « PNA » plus un « PNA » de contrôle, il a été possible d'isoler de façon automatique les organes et ainsi de d'obtenir les paramètres pharmacocinétiques par organe pour chaque type de « PNA ». Les cinétiques obtenues automatiquement ont montré une bonne concordance aux cinétiques obtenues par une segmentation manuelle des organes discernables sur l'image TEP.

Cette étude montre que les pharmacocinétiques et pharmaco-distributions d'oligonucléotides peuvent être fortement modifiées. Ces résultats présentent une avancée forte dans le développement de « drogues » de type « PNA » pour une utilisation *in vivo* à visée thérapeutique.

**2) Applications envisagées de la méthode de l'invention** :

La méthode de segmentation automatique d'images selon l'invention utilisant l'information pharmacocinétique peut être appliquée avantageusement à toute étude basée sur une mesure localisée dans l'espace, au sein d'un organisme, et présentant une variation au cours du temps. Les modalités concernées sont notamment la médecine nucléaire (techniques TEP et « SPECT », notamment), mais aussi l'IRM fonctionnelle et l'imagerie optique.

On peut notamment citer les applications suivantes.

**a) Pharmaco imagerie** :

> **Imagerie TEP corps entier** *:*

La présente méthode de segmentation permet d'obtenir une bonne segmentation, malgré les mouvements physiologiques du sujet imagé.

> **Pharmaco-imagerie sans** *a priori :*

La présente méthode de segmentation ne nécessite aucune connaissance préalable sur la réponse des divers organes au traceur.

➢ **Tri in vivo de molécules :**

Devant les capacités de la chimie combinatoire à générer de grandes banques de molécules, se pose le problème de la sélection de molécules d'intérêt. Cette sélection peut être opérée *in vitro* ou en fonction de caractéristiques chimiques, de polarité ou de caractéristiques géométriques de la molécule, mais rien n'assure alors le passage des barrières systèmes et l'efficacité *in vivo* de la molécule.

La bio-distribution *in vivo* renseigne sur les capacités de ciblage de la molécule injectée au sujet imagé. Il devient donc possible de sélectionner une molécule parmi un groupe de molécules d'intérêt. Cependant, le traitement manuel des données d'un grand nombre d'examens est une tâche longue et fastidieuse, ce qui limite dans les faits la quantité de molécules testées.

La présente méthode de segmentation permet, par son caractère automatique et sa rapidité, de traiter rapidement un grand nombre d'examens, permettant un tri *in vivo* dont le débit est désormais limité par l'acquisition des examens et non le traitement des données issues de ceux-ci.

➢ **Etude de nouveaux traceurs :**

La présente méthode ne requérant pas de connaissance a *priori* du comportement des traceurs - le modèle appliqué reste valable pour tout traceur -, il permet l'obtention rapide de renseignements sur la pharmacocinétique d'un nouveau traceur, aux propriétés inconnues.

➢ **Ciblage d'organes :**

Certaines molécules présentent un intérêt *in vitro* du fait de leur principe actif, mais n'entrent pas dans un organe cible. Elles ne présentent donc aucun intérêt *in vivo.* Cependant, par ajout d'étiquettes chimiques ne modifiant pas le principe actif d'une telle « drogue », ou en l'encapsulant dans un vecteur, il devient possible de modifier ses capacités de ciblage.

Une telle molécule ainsi déclinée génère une famille de molécules au principe actif supposé identique, mais dont les pharmacocinétiques seront différentes. Les molécules ainsi générées peuvent subir un tri *in vivo,* comme décrit ci-dessus, pour sélectionner celles qui présentent les capacités de ciblage requises.

**b) imagerie en physiologie ;**

**c) Détection d'analogie de réponse des organes à un traceur :**

La présente méthode de segmentation ne permet pas de distinguer deux pharmaco-organes aux fonctionnements identiques. Dans l'image résultant du procédé de segmentation, ces deux organes se verront attribuer le même label. Il est ainsi possible de repérer des analogies entre les fonctionnements de deux organes.

De façon analogue, certains organes sont présents dans l'organisme par paire (yeux, reins, poumons). Si le procédé de segmentation permet de les dissocier, il est fort probable que leur réponse au traceur soit différente, par exemple du fait du dysfonctionnement de l'un d'entre eux.

**d) Détermination de modèles de cinétiques** :

L'étape 40 de définition d'échantillons - voire même directement l'étape 30 qui extrait les voxels-modèles - permet d'estimer les modèles de cinétiques propres à chaque organe. Dans l'exemple de réalisation donné, les modèles des tous les organes, des droites dans l'espace des cinétiques, ont un unique point d'intersection : la cinétique artérielle. Celle-ci peut donc théoriquement être déterminée par un calcul de l'intersection des droites-modèles dans l'espace des cinétiques.

L'estimation de ces modèles étant biaisée par l'effet de volume partiel et le nombre d'échantillons relativement faible, les droites n'auront pas probablement pas de point d'intersection, mais passeront près de la cinétique artérielle. L'estimation des modèles peut donc permettre une estimation directe de la cinétique artérielle ou bien servir d'initialisation à une méthode permettant de le faire.

**e) Segmentation préalable à une reconstruction des cinétiques des structures :**

Parmi les utilisations de la segmentation, on compte désormais la reconstruction des cinétiques dans l'organisme, non pas élément de volume par élément de volume, mais organe par organe. En effet, le rapport signal sur bruit dépend de la finesse de l'échantillonnage temporel et de la résolution spatiale. En dégradant la résolution spatiale, il est possible d'affiner l'échantillonnage temporel. Plutôt que de dégrader la résolution spatiale de manière arbitraire, il paraît naturel de regrouper les éléments de volume ayant une réponse identique aux traceurs.

Plutôt que de segmenter des images anatomiques et de les recaler sur la modalité TEP afin de déterminer ces regroupements, le procédé de segmentation proposé permet de se passer de la modalité anatomique en utilisant comme regroupement les pharmaco-organes issus de la segmentation. En effet, le recalage entre modalité anatomique et fonctionnelle est rendu difficile dans le cas du corps entier, et la modalité anatomique n'est même pas toujours disponible.

**f) Utilisation des modèle de cinétique en reconstruction d'image :**

La méthode de segmentation selon l'invention permet d'accéder à un modèle de cinétique de chaque structure présente dans l'image. L'accès à ce modèle ne nécessite pas de segmenter entièrement l'image. Ce modèle de cinétique peut avoir de nombreuses applications, comme par exemple la reconstruction « 4D » en TEP [Nichols, 2002].

Plus généralement, l'information temporelle pourra être utilisée comme *a priori,* ou en information supplémentaire pour améliorer la qualité des images, notamment en terme de fluctuation statistique.

**g) Reconnaissance des structures en TEP** :

La méthode de segmentation selon l'invention permet de regrouper sous le même label les éléments de volume appartenant au même pharmaco-organe. Il reste alors à reconnaître les organes correspondant aux différents labels. La représentation hiérarchique proposée ici devrait faciliter cette étape de reconnaissance, en repérant le niveau de fusion permettant de faire apparaître un organe précis isolé des autres et en un seul morceau.

**h) Vérification de la cohérence fonctionnelle d'un organe anatomiquement segmenté** :

La corrélation entre les contours anatomiques d'un organe et l'identité des réponses de ses sous-parties à un traceur donné n'est pas assurée - lorsqu'une tumeur est présente par exemple. Le procédé proposé permet de séparer automatiquement un organe en sous-parties aux réponses distinctes au traceur.

**i) Séquences temporelles en imagerie optique** :

L'imagerie optique permet d'obtenir des images anatomiques, mais aussi fonctionnelles. Ces images présentent une très haute résolution spatiale et temporelle. La méthode selon l'invention permet de segmenter automatiquement de telles images.

**j) Images statiques :**

La présente méthode peut être appliquée à des images purement « 2D » ou « 3D », moyennant un modèle adéquat choisi lors de l'étape 10.

**Références bibliographiques citées**

- Ashburner et coll., A cluster analysis approach for the characterisation of dynamic PET data. Quantification of Brain Function using PET, pages 301-306, San Diego, Academic Press (1996).
- Acton, P.D., Pilowsky, L.S., Kung, H.F., Ell, P.J. Automatic segmentation of dynamic neuroreceptor single-photon emission tomography images using fuzzy clustering. European Journal of Nuclear Medecine and Molecular Imaging, Springer-Verlag, Heingelberg, 26(6):581-590 (1999).
- Brankov, J.G., Galatsanos, N.P., Yongyi Yang Wernick, M.N., Segmentation of dynamic PET or fMRI images based on a similarity metric. IEEE Transactions on Nuclear Science, 50(5):1410-1414 (2003).
- Guo, H., Renaut, R., Chen, K., Reiman, E. Clustering huge data sets for parametric PET imaging,. BioSystem 71, 81-92 (2003).
- Frouin, F., Boubacar, P., Frouin, V., De Cesare, A., Todd-Pokropek, A., Merlet, P., Herment, A. 3D regularisation and segmentation of factor volumes to process PET H215O myocardial perfusion studies. Functional Imaging and Modelling of the Heart (FIMH'2001). Lecture Notes in Computer Sciences, Springer Verlag, Heidelberg, 2230: 91-96 (2001).

- Frouin, V., Comtat, C., Reilhac, A., Grégoire, M.-C., Correction of Partial-Volume Effect for PET Striatal Imaging: Fast Implementation and Study of Robustness. Journal of Nuclear Medicine 43-12:1715-1726 (2002).
- Jain, A.K., Dubes, R.C., Algorithm for Clustering Data. Advanced Reference. Prentice-Hall, Englewood Cliffts, NJ (1988).
- Kimura, Y., Senda, M., Alpert, N. Fast formation of statistically reliable FDG parametric images based on clustering and principal components. Phys. Med. Biol. 47(3):455-468 (2002).
- Mangin, J.-F., Coulon, O., Frouin V., Robust Brain Segmentation Using Histogram Scale-Space Analysis and Mathematical Morphology. MICCAI, Springer Verlag, Heidelberg, LNCS 1496, pp. 1230-1241 (1998).
- Minka, T.P., Automatic choice of dimensionality for PCA. M.I.T. Media Laboratory Perceptual Computing Section Technical Report No 514 (2000).
- Nichols, T.E., Qi, J., Asma, E., Leahy, R.M., Spatiotemporal Reconstruction of List-Mode PET Data. IEEE Transactions on Medical Imaging, 21(4):396-404 (2002).
- Segars, W.P., Tsui, B.M.W., Frey, E.C., Johnson, G.A., Berr, S.S., Development of a 4D Digital Mouse Phantom for Molecular Imaging Research. Molecular Imaging and Biology (2004).
- Tipping, M.E., Bishop, C.M., Mixture of probabilistic principal component analysers. Neural Computation, 11(2):443-482 (1999).
- Wong, K.-P., Feng, D., Meikle, S.R., Fulham, M.J. 2001. IEEE Transactions on Nuclear Science 49:200-207 (2002)
- Zhou, Y., Huang, S.-C., Bergsneider, M., Wong, D.F. A non linear regression with spatial constraint for generation of parametric images in dynamic PET studies. Journal of Nuclear Medecine 42 (5), 100.

**ANNEXE A:**

**Allure des cinétiques dans un modèle compartimental à quatre paramètres.**

➢ *Modèle de cinétique à un seul traceur :*

Le modèle $M_{i,j}(t)$ du signal TEP au sein d'un élément de *volume j* donné compris dans l'organe *i* s'écrit, après correction de la décroissance radioactive :

$$M_{i,j}(t) = \left(\left(1 - Vb_{i,j}\right) \times Ct_i(t) + Vb_{i,j} \times Ca(t)\right) \times D$$

où D est la dose injectée et $Vb_{i,j}$ est le ratio de volume sanguin au sein de l'élément de volume *j* de l'organe *i*.
Lorsque *j* se déplace dans l'organe *i*, $M_{i,j}(t)$ décrit un segment de la droite *Ca(t)Ct_i(t)* dans l'espace des cinétiques.

➢ *Modèle de cinétique à plusieurs traceurs :*

Lorsque plusieurs traceurs $\{Tr_s\}_{1 \leq s \leq S}$ sont injectés au sein de l'organisme, l'activité obtenue au sein de l'élément de volume j au cours du temps est la somme des cinétiques des différents traceurs. Si les cinétiques tissulaires et plasmatiques dépendent du traceur, le ratio de volume plasmatique au sein de l'élément de volume *j*, lui, est indépendant de s. Le modèle $M_{i,s,j}(t)$ s'écrit, après correction de la décroissance radioactive :

$$M_{i,s,j}(t) = \sum_{s=1}^{S} \left[D_s \times \left(\left(1 - Vb_{i,j}\right) \times Ct_{i,s}(t) + Vb_{i,j} \times Ca_s(t)\right)\right]$$

$$M_{i,s,j}(t) = \left(\left(1 - Vb_{i,j}\right) \times \sum_{s=1}^{S} \left(D_s \times Ct_{i,s}(t)\right) + Vb_{i,j} \times \sum_{s=1}^{S} \left(D_s \times Ca_s(t)\right)\right)$$

$M_{i,s,j}(t)$ appartient toujours à une droite dans l'espace des cinétiques, cette fois la droite passant par $\sum_{s=1}^{S}\left(D_s \times Ct_{i,s}(t)\right)$ et $\sum_{s=1}^{S}\left(D_s \times Ca_s(t)\right)$.

➢ *Effet de volume partiel :*

L'effet de volume partiel peut être modélisé [Frouin, 2002] comme un lissage de l'image. Le signal $S_j$ mesuré dans l'image au sein d'un élément de *volume j* est donc une combinaison linéaire des modèles $M_{i,k}$ des pharmaco-organes des éléments de volume $k$ voisins. Considérons pour plus de clarté le cas de l'injection d'un seul traceur.

$$S_j(t) = \sum_{k \in Voisinage(j)} \left( \beta_{j,k} \times M_{i(k),k}(t) \right)$$

- Au sein d'un organe :

Lorsque le voisinage de $j$ sur lequel s'étend l'effet de volume partiel ne contient que l'organe $i$, le signal $S_j(t)$ s'écrit :

$$S_j(t) = \left[ \sum_{k \in Voisinage(j)} \left( \beta_{j,k} \times (1 - Vb_{i,k}) \right) \right] \times \sum_{s=1}^{S} \left( D_s \times Ct_{i,s}(t) \right) + \left[ \sum_{k \in Voisinage(j)} \left[ \beta_{j,k} \times Vb_{i,k} \right] \right] \times \sum_{s=1}^{S} \left( D_s \times Ca_s(t) \right)$$

$S_j(t)$ appartient toujours à la droite passant par $\displaystyle\sum_{s=1}^{S} \left( D_s \times Ct_{i,s}(t) \right)$ et $\displaystyle\sum_{s=1}^{S} \left( D_s \times Ca_s(t) \right)$.

**- En bordure d'organe :**

Lorsque, au contraire, ce voisinage de $j$ contient l ($l > 1$) organes dont les modèles sont différents, $C_j$ se calcule comme une combinaison linéaire des signaux appartenant chacun à une des droites $M_{i,j}$. L'ensemble des signaux contenus dans le voisinage de $j$ ne peut donc pas être décrit par une droite.

En effet :

$$S_j(t) = \sum_{i=1}^{I} \left( \left[ \sum_{k \in Voisinage(j) \cap i} \left( \beta_{j,k} \times (1 - Vb_{i,k}) \right) \right] \times \sum_{s=1}^{S} \left( D_s \times Ct_{i,s}(t) \right) + \left[ \sum_{k \in Voisinage(j) \cap i} \left[ \beta_{j,k} \times Vb_{i,k} \right] \right] \times \sum_{s=1}^{S} \left( D_s \times Ca_s(t) \right) \right)$$

$$S_j(t) = \sum_{i=1}^{I} \left( \left[ \sum_{k \in Voisinage(j) \cap i} \left( \beta_{j,k} \times (1 - Vb_{i,k}) \right) \right] \times \sum_{s=1}^{S} \left( D_s \times Ct_{i,s}(t) \right) \right) + \left[ \sum_{i=1}^{I} \left( \sum_{k \in Voisinage(j) \cap i} \left[ \beta_{j,k} \times Vb_{i,k} \right] \right) \right] \times \sum_{s=1}^{S} \left( D_s \times Ca_s(t) \right)$$

$$S_j(t) = \sum_{i=1}^{I} \left( a_{i,j,k} \times \sum_{s=1}^{S} \left( D_s \times Ct_{i,s}(t) \right) \right) + b \times \sum_{s=1}^{S} \left( D_s \times Ca_s(t) \right)$$

Les vecteurs $Ca_s(t)Ct_{i,s}(t)$ n'étant pas colinéaires pour $1 \leq i \leq l$, puisque les modèles sont supposés différents, $S_j(t)$ ne décrit donc pas une droite mais un sous espace de dimension $d$ telle que $1 < d \leq l$.

➢ *Mouvement physiologique :*

Dans les zones de l'espace affectées d'un mouvement physiologique non périodique ou dont la période est supérieure à la durée de l'acquisition d'une image de la série, un même élément de volume $j$ ne contient pas les mêmes organes pour toutes les images de la série. L'analyse de la cinétique du premier instant d'acquisition au dernier n'a donc plus de sens. C'est notamment le cas dans la vessie et les viscères.

Considérons une zone de l'image affectée par un mouvement périodique de période très inférieure à la durée d'acquisition. Le signal est accumulé pendant une durée au cours de laquelle un grand nombre de périodes sont achevées. Le temps d'acquisition $\Delta t$ d'une image peut être découpé en :

$$\Delta t = n \times P + \delta t,$$

ou $P$ est la période du mouvement physiologique. Si $P << \Delta t$, on a aussi $\delta t << \Delta t$. Si l'on suppose continue la cinétique des traceurs, le signal enregistré pendant la durée $\delta t$ est négligeable par rapport au signal enregistré pendant la durée $n \times P$. Ce sont donc, à peu de chose près, les mêmes sites physiologiques qui sont observés au sein d'un volume donné pour chaque image de la série étudiée. La démonstration du caractère linéaire du modèle à un organe malgré l'effet de volume partiel est aisément transposable au problème du mouvement physiologique périodique rapide.

Soit $k$ une unité de volume de centre $x$ se déplaçant avec les structures le long d'une courbe $\Omega$ et $j$ une unité de volume fixe dans le champ de vue.

**- Mouvement au sein du même organe $i$ :**

Le signal $S_j(t)$ à l'élément de volume $j$ s'écrit :

$$S_j(t) = \left[\oint_\Omega \left(\beta_{j,k(x)} \times \left(1 - Vb_{i,k(x)}\right)\right)dx\right] \times \sum_{s=1}^S \left(D_s \times Ct_{i,s}(t)\right) + \left[\oint_\Omega \left(\beta_{j,k(x)} \times Vb_{i,k(x)}\right)dx\right] \times \sum_{s=1}^S \left(D_s \times Ca_s(t)\right)$$

$S_j(t)$ appartient toujours à la droite passant par $\sum_{s=1}^S \left(D_s \times Ct_{i,s}(t)\right)$ et $\sum_{s=1}^S \left(D_s \times Ca_s(t)\right)$.

**- Mouvement passant par plusieurs organes :**

$$S_j(t) = \sum_{i=1}^I \left[\left(\int_{\Omega \cap i} \left(\beta_{j,k(x)} \times \left(1 - Vb_{i,k(x)}\right)\right)dx\right) \times \sum_{s=1}^S \left(D_s \times Ct_{i,s}(t)\right)\right] + \sum_{i=1}^I \left[\int_{\Omega \cap i} \left(\beta_{j,k(x)} \times Vb_{i,k(x)}\right)dx\right] \times \sum_{s=1}^S \left(D_s \times Ca_s(t)\right)$$

$S_j(t)$ ne décrit donc pas une droite, mais un sous espace de dimension $d$ telle que $1 < d \leq I$.

➢ *Mouvement physiologique et effet de volume partiel :*

On montre de manière connue que le modèle à un organe $i$ n'est conservé que si le voisinage de $k$ sur lequel s'étend le volume partiel, durant tout le trajet $\Omega$ de $k$ - de centre $x$ - dû au mouvement physiologique périodique, est inclus dans $i$.

Toujours avec l'hypothèse qu'il n'existe pas deux organes différents qui sont tels que leurs modèles de cinétiques sont identiques, le signal $S_j(t)$ à l'élément de *volume j* fixe dans le champ de vue appartient à la droite $Ca(t)Ct_i(t)$ pour tout $j$, si et seulement si le voisinage de $j$ sur lequel s'étend l'effet de volume partiel reste inclus dans l'organe $i$ durant le mouvement physiologique périodique.

➢ *Conclusion :*

Si l'on exclut les zones de mouvement physiologique non périodique ou à longue période, et si l'on suppose qu'aucun couple d'organes ne possède le même modèle de cinétique, le modèle à un organe $i$ est donc valide au sein d'un volume B, fixe dans le référentiel de la caméra, si et seulement si le voisinage *Vois(B)* de ce volume sur lequel s'étend l'effet de volume partiel reste inclus dans l'organe $i$ lors de la période du mouvement physiologique.

**ANNEXE B:**

**Choix de la mesure de non-validité locale du modèle à un organe.**

**1) <u>Vraisemblance du modèle de signal choisi</u> :**

Si $B_{j,r}$ est incluse dans un pharmaco-organe $i$,

$H_0$ : $C_j(t)$ peut s'écrire $C_j(t) = \mu_i(t) + \gamma_i j \times e_i(t) + \varepsilon_j(t)$

La vraisemblance de $H_0$ est un bon indicateur de la validité du modèle sur $B_{j,r}$. Lorsque la boule $B_{j,r}$ est incluse dans un organe, la dimension du signal est égale à 1. La sélection de modèle dans le cadre Bayésien, si $C_{j,r}$ est l'ensemble des cinétiques contenues dans la boule $B_{j,r}$ et $K$ est le nombre d'organes présent dans $B_{j,r}$, revient à maximiser :

$$p(C_{j,r} / k) = \int_{\theta} p(C_{j,r} / \theta) p(\theta / K) d\theta \text{ pour K = 1.}$$

Dans le cas d'une modélisation par ACP (analyse en composantes principales) avec un bruit additif gaussien, l'approximation de Laplace de l'évidence $p(C_{j,r} / k)$ pour le modèle à K vecteurs propres se révèle [Minka, 2000] être un excellent indicateur de la dimension réelle du modèle.

L'approximation de Laplace de la vraisemblance des données selon le modèle à K dimensions s'écrit :

$$p(\theta) \approx f(\Theta)(2\pi)^{rows(A)/2} |A|^{-1/2}$$

avec

$$\Theta = \arg\max_{\theta} \left( f(\theta) \right), \ A = -\left[ \frac{d^2 \log f(\theta)}{d\theta_i d\theta_j} \right]_{\theta=\Theta} \text{ et } \theta = (U, \Lambda, v).$$

$U$ est la matrice des vecteurs propres de la matrice de corrélation, $\Lambda$ la matrice des valeurs propres et $v$ une estimation de la variance du bruit et du signal non reconstruit par l'ACP au sein de la boule $B_{i,r}$. Soit $U'$, $L'$ et $v'$ les valeurs des paramètres $U, \Lambda$ et $v$ maximisant $f$.

$$v' = \frac{N \sum_{l=K+1}^{d} \lambda_l}{n(d-k) - 2}$$

Les valeurs diagonales de $\Lambda$ s'écrivent $\lambda'_l = \frac{N\lambda_l + \alpha}{N - 1 + \alpha}$ pour $l \leq K$ et $\lambda_l = V$ sinon, avec $\alpha$ représentant l'importance de l'a *priori.*

$$p(C_{j,r} / K) \approx p(U) \left( \prod_{k=1}^{K} \lambda_j \right)^{-N/2} V^{-N(d-k)/2} (2\pi)^{(m+k)/2} |A|^{-1/2} N^{-k/2}$$

➢ *Maxima locaux de la vraisemblance du modèle à un organe :*

Soit $j$ tel que la vraisemblance des données selon le modèle de l'organe $i$ soit maximale sur $B_{j,r}$

$j$ vérifie $p(C_{j,r} / K = 1) > p(C_{k,r} / K = 1)$ pour tout $k$ appartenant au voisinage de $j$.

Après un calcul de la carte spatiale des $p(C_{j,r} / K = 1)$, les maxima locaux sont extraits. Chaque maximum local situé dans un organe correspond à une vraisemblance maximale de la validité du modèle. Ces points sont donc très peu entachés de volume partiel, auquel cas la dimension du modèle nécessaire à la caractérisation du signal serait

supérieure à 1.

<u>Discussion :</u> Cette mesure s'avère en pratique extrêmement bruitée et peu sélective de l'intérieur des organes.

➢ ***Carte du nombre d'organes présents dans le voisinage :***

$D_j = \arg\max_K (p(C_{j,r} / K))$ est la dimension la plus vraisemblable des données au sein de $B_{j,r}$. Les zones $D_j$ est égale à 1 sont des zones situées à l'intérieur des organes.

<u>Discussion :</u> Cet indice présente des points de bruit impossibles à dissocier des réels éléments de volume $j$ tels que $B_{j,r}$ soit incluse dans un organe. De plus, les organes de petite taille ou de forme allongée ne présentant pas de points non entachés par le signal des pharmaco-organes voisins - du fait de l'effet de volume partiel - ils ne sont pas détectés.

## 2) Analyses en composantes principales locales :

Soit $\{e_{j,l}(t)\}_{1=l=T}$ les vecteurs propres d'une analyse en composante principale (ACP), centrée en $\mu_j(t)$, opérée sur les cinétiques des éléments de volume inclus dans la boule $B_{j,r}$. Soit $\sigma_{j,t}^2$ la variance au temps $t$ du bruit $\varepsilon_j(t)$ et $\{\lambda_{j,l}\}_{1 \leq l < T}$ les valeurs propres de l'ACP.

La variance moyenne de $\varepsilon_j(t)$ s'écrit : $\sigma_j^2 = \dfrac{1}{T} \times \sum_{t=1}^{T} \sigma_{j,t}^2 = \lambda_j^2$, avec $\lambda_j^2 = \dfrac{1}{T-1} \sum_{l=2}^{T} \lambda_{j,l}^2$.

Plaçons-nous pour commencer dans le cas d'une reconstruction par une méthode analytique. Le bruit étant stationnaire tant dans l'espace que dans le temps, sa variance est constante : $\sigma_{j,t}^2 = \sigma^2$. Au sein de l'organe,

$$\sigma^2 = \frac{1}{T-1} \sum_{l=2}^{T} \lambda_{j,l}^2 .$$

$\lambda_j^2$ semble donc un bon indicateur de la position de l'élément de volume $j$ par rapport aux frontières des organes.

En effet, lorsque l'élément de volume j contient, du fait de l'effet de volume partiel, les cinétiques de plusieurs pharmaco-organes, les valeurs propres de l'ACP non prises en compte pour modéliser le signal contiennent à la fois de l'information de pharmaco-cinétique et du bruit. On aura alors $\sigma^2 < \lambda_j^2$, avec $\lambda_j^2 = \dfrac{1}{T-1} \sum_{l=2}^{T} \lambda_{j,l}^2$. On en déduit donc que $\lambda_j^2 / \sigma^2 \sim 1$ lorsque $j$ se trouve au coeur d'un organe et $\lambda_j^2 / \sigma^2 \gg 1$ lorsque $j$ se trouve à la frontière entre deux organes.

Toutefois cette mesure ne permet pas de discriminer les points intérieurs à l'organisme des points extérieurs. En effet, lorsque $j$ est situé en dehors de l'organisme, l'extérieur de l'organisme ne contenant pas de traceur, $j$ ne contient que du bruit, d'où $\lambda_j^2 / \sigma^2 = 1$.

De même, entre deux organes $i$ et $o$, $i$ présentant un fort signal et $o$ un faible signal de l'ordre de la variance du bruit, $\lambda_j^2 / \sigma^2$ sera peu différent de 1.

**- Moyenne de la proportion de signal non reconstruit :**

Définissons la mesure suivante d'inhomogénéité locale des cinétiques relative au signal :

$$PNR_j = \frac{1}{N} \times \sum_{\{k \,/\, k \in B_{j,r}\}} \left[ \frac{\|\varepsilon_{j,k}\|^2}{\|C_k\|^2} \right]$$

Soit $j$ tel que $B_{j,r} \subset$ organe $i$, et $m$ tel que
$\#(B_{m,r} \cap B_{i,r}) \gg \#(B_{m,r} - B_{m,r} \cap B_{i,r})$ et que
$B_{m,r} - B_{m,r} \cap B_{i,r}$ sont constitué de points extérieurs à l'organe, dont les cinétiques ne peuvent être modélisées par les K premiers vecteurs de l'ACP calculée sur $i$. Supposons $\sigma_j \ll \|\mu_j\|^2$ :

1) Pour $k$ tel que $k \in B_{m,r} \cap i$, $\dfrac{\left\|\varepsilon_{j,k}\right\|^2}{\left\|C_k\right\|^2} \sim \dfrac{\sigma_i^2}{\left\|\mu_i\right\|^2}$, alors que $\dfrac{\left\|\varepsilon_{m,k}\right\|^2}{\left\|C_k\right\|^2} \sim \dfrac{\lambda_m^2}{\left\|\mu_i\right\|^2}$, avec $\lambda_m > \sigma_i$, comme nous l'avons vu plus haut.

2) Pour $k$ tel que $k \in B_{m,r} - B_{m,r} \cap i$, $\dfrac{\left\|\varepsilon_{m,k}\right\|^2}{\left\|C_k\right\|^2} \sim 1$, car $C_k$ est mal reconstruite par l'ACP caractérisant l'organe $i$.

3) Pour k tel que $k \in B_{j,r} - B_{m,r} \cap i$, $P_k$ appartient à l'organe $i$ :

$$\frac{\left\|\varepsilon_{j,k}\right\|^2}{\left\|C_k\right\|^2} \sim \frac{\sigma_i^2}{\left\|\mu_i\right\|^2} << 1.$$

$$PNR_m - PNR_j = \frac{1}{N} \times \left( \sum_{\{k/k \in B_{j,r}\}} \left[ \frac{\left\|\varepsilon_{j,k}\right\|^2}{\left\|C_k\right\|^2} \right] - \sum_{\{k/k \in B_{m,r}\}} \left[ \frac{\left\|\varepsilon_{m,k}\right\|^2}{\left\|C_k\right\|^2} \right] \right)$$

$$PNR_m - PNR_j = \begin{bmatrix} \frac{1}{N} \times \overbrace{\left( \sum_{\{k/k \in B_{j,r} \cap B_{m,r}\}} \left[ \frac{\left\|\varepsilon_{m,k}\right\|^2}{\left\|C_k\right\|^2} \right] - \sum_{\{k/k \in B_{j,r} \cap B_{m,r}\}} \left[ \frac{\left\|\varepsilon_{j,k}\right\|^2}{\left\|C_k\right\|^2} \right] \right)}^{A} \\ + \frac{1}{N} \times \underbrace{\left( \sum_{\{k/k \in B_{m,r} - B_{j,r} \cap B_{m,r}\}} \left[ \frac{\left\|\varepsilon_{m,k}\right\|^2}{\left\|C_k\right\|^2} \right] - \sum_{\{k/k \in B_{j,r} - B_{j,r} \cap B_{m,r}\}} \left[ \frac{\left\|\varepsilon_{j,k}\right\|^2}{\left\|C_k\right\|^2} \right] \right)}_{B} \end{bmatrix}$$

$$A \sim N_\cap \times \left( \frac{\lambda_m^2}{\left\|\mu_i\right\|^2} - \frac{\sigma_i^2}{\left\|\mu_i\right\|^2} \right) > 0.$$

$$B \sim 1 - \frac{\sigma_i^2}{\left\|\mu_i\right\|^2} > 0.$$

Pour tout élément de volume $k$ en bordure de l'organe $i$, et pour tout j tel que $B_{j,r}$ est incluse dans l'organe $i$, $PNR_k > PNR_j$. Avec les hypothèses que $\sigma_j << \left\|\mu_j\right\|^2$ et que les organes voisins de l'organe $i$ sont caractérisés par des cinétiques présentant une forte proportion de signal non reconstruite par le modèle défini sur $i$, il existe donc un minimum $PNR_j$ de $PNR$ au sein de l'organe $i$, tel que $B_{j,r}$ soit incluse dans l'organe $i$.

Cette mesure d'inhomogénéité nécessite le calcul d'une ACP pour chaque $B_{j,r}$ mais aussi celui de la projection de la cinétique de chaque élément de volume inclus dans $B_{j,r}$ sur le premier vecteur propre de cette ACP. Or, le critère étant calculé sur chaque élément de volume inclus dans l'organisme imagé, ce surcoût de calcul se révèle prohibitif.

**- Variance relative des résidus :**

Moyennant l'approximation qu'au sein et en bordure d'un organe, $\left\|C_k\right\|^2 \sim \left\|\mu_j\right\|^2$, définissons la mesure suivante d'inhomogénéité locale des cinétiques relative au signal :

$$IR_j = \lambda_j^2 / \left\| \mu_j \right\|^2 ,$$

où $\mu_j$ est la moyenne du signal sur la boule B$_{j,r}$, incluse dans l'organe, et $\lambda_j^2 = \dfrac{1}{T-1} \displaystyle\sum_{l=2}^{T} \lambda_{j,l}^2$ . $\lambda_j^2$ est la variance du signal non reconstruit par une ACP opérée sur les cinétiques des éléments de volume appartenant à la boule B$_{j,r}$ centrée en j et de rayon r, dont seules les K premiers vecteurs propres ont été conservés.

➢ **Variance relative non reconstruite corrigée pour les reconstructions itératives :**

Dans le cas d'une reconstruction itérative avec un nombre fixe d'itérations pour chaque image de la séquence reconstruite, la variance du bruit $\sigma_j^2(t)$ à l'élément de volume $j$ et à l'instant $t$ peut s'écrire :

$$\sigma_j^2(t) = \alpha^2 \times S_j(t) / \Delta_t.$$

$\sigma_j^2(t)$ est non stationnaire, tant d'un point de vue spatial que temporel, car proportionnel au signal. Afin de s'affranchir de la contribution du signal à la variance du bruit, interdisant toute comparaison de variance de bruit inter voxels, nous étudierons non pas $\sigma_j^2(t)$, mais $\alpha^2 = \sigma_j(t)^2 / S_j(t) \times \Delta_t$. En effet, $\alpha^2$ est une quantité indépendante du signal de la durée d'acquisition des images de la séquence, donc stationnaire dans l'espace et le temps.

Si l'on suppose une variation continue et faible du signal dans l'espace, $S_{j,k}(t)$, pour $k \in B_{j,r}$ peut être approché par la cinétique moyenne $\mu_{j,t}$ au sein de la boule $B_{j,r}$. De même, $S'_{j,k}(t) = S_{j,k}(t) \times \Delta_t$ peut être approché par $\mu'_i(t)$, la moyenne des cinétiques $C'_k(t)$ au sein de la boule $B_{j,r}$.

Soit l'analyse en composantes principales ($\mu'_j(t)$, $\{\lambda'_{j,l}\}$, $\{e'_{j,l}(t)\}$) calculée sur les cinétiques

$$C''_{i,k}(t) = C'_k(t) \Big/ \sqrt{\mu'_i(t)} = C_k(t) \times \Delta_t \Big/ \sqrt{\mu'_i(t)} .$$

$$C''_{i,k}(t) = S_{j,k}(t) \times \Delta_t \Big/ \sqrt{\mu'_i(t)} + N(0, \alpha'^2) = S''_{j,k}(t) + N(0, \alpha'^2) ,$$

avec $\alpha' \approx \alpha$, stationnaire dans l'espace. Quand $B_{j,r}$ est incluse dans un organe $i$, $S''_{j,k}(t) = M''_{i,k}(t)$ et décrit une droite dans l'espace des cinétiques lorsque $j$ se déplace au sein de $i$.

Le critère de variance relative non reconstruite devient $\Gamma'_{Vj} = \dfrac{\lambda'^2_j}{\left\| \mu_j \right\|^2}$, qui mesure la validité du modèle à un organe au sein de la boule $B_{j,r}$.

**ANNEXE C:**

**Variations de l'exemple de réalisation dans le cas d'images reconstruites par un algorithme itératif.**

Dans le cas d'un algorithme itératif, la variance du bruit est corrélée au signal. Afin de pouvoir comparer deux indices basés sur une mesure de l'erreur de reconstruction par le modèle de cinétique de pharmaco-organe, il semble donc essentiel de corriger de cette influence du signal à chaque fois que c'est possible.

Dans le cas d'une reconstruction itérative avec un nombre fixe d'itérations pour chaque image de la séquence reconstruite, la variance du bruit $\sigma_j^2(t)$ à l'élément de volume $j$ et à l'instant $t$ peut s'écrire :

$$\sigma_j^2(t) = \alpha^2 \times S_j(t) / \Delta_t.$$

$\sigma_j^2(t)$ est non stationnaire, tant d'un point de vue spatial que temporel, car proportionnel au signal. Afin de s'affranchir de la contribution du signal à la variance du bruit, interdisant toute comparaison de variance de bruit inter voxels, nous étudierons non pas $\sigma^2_j(t)$, mais $\alpha^2 = \sigma_j(t)^2 / S_j(t) \times \Delta_t$. En effet, $\alpha^2$ est une quantité indépendante du signal de la durée d'acquisition des images de la séquence, donc stationnaire dans l'espace et le temps.

➢ *Indicateur de l'intérieur des organes :*

Si l'on suppose une variation continue et faible du signal dans l'espace, $S_{j,k}(t)$, pour $k \in B_{j,r}$ peut être approché par la cinétique moyenne $\mu_{j,t}$ au sein de la boule $B_{j,r}$. De même, $S'_{j,k}(t) = S_{j,k}(t) \times \Delta_t$ peut être approché par $\mu'_i(t)$, la moyenne des cinétiques $C'_k(t)$ au sein de la boule $B_{j,r}$.

Soit l'analyse en composantes principales $(\mu'_j(t), \{\lambda'_{j,l}\}, \{e'_{j,l}(t)\})$ calculée sur les cinétiques

$$C''_{i,k}(t) = C'_k(t)\Big/\sqrt{\mu'_i(t)} = C_k(t) \times \Delta_t \Big/\sqrt{\mu'_i(t)} \,.$$

$$C''_{i,k}(t) = S_{j,k}(t) \times \Delta_t \Big/\sqrt{\mu'_i(t)} + N(0,\alpha'^2) = S''_{j,k}(t) + N(0,\alpha'^2) \,,$$

avec $\alpha' \approx \alpha$, stationnaire dans l'espace. Quand $B_{j,r}$ est incluse dans un organe $i$, $S''_{j,k}(t) = M''_{i,k}(t)$ et décrit une droite dans l'espace des cinétiques lorsque $j$ se déplace au sein de $i$.

Le critère de variance relative non reconstruite devient $\Gamma'_j = \dfrac{\lambda'^2_j}{\left\|\mu_j\right\|^2}$, qui mesure la validité du modèle à un organe au sein de la boule $B_{j,r}$.

➢ *Optimisation des échantillons :*

La proportion de signal non reconstruite calculée sur $B_{j,r}$ doit être corrigée de l'influence du signal sur le bruit. Elle devient $p''_{j,k} = \dfrac{\sum_{t=1}^{T}\left(\left|\varepsilon'_{j,k}(t)\right|^2 \Big/ S'_{j,k}(t)\right)}{\left\|C'_k\right\|^2}$ , où $S'_{j,k}(t)$ est le signal estimé en $k$ comme la reconstruction de $C'_k(t)$ par le modèle à un organe. $S'_{j,k}(t)$ s'écrit comme $S'_{j,k} = \mu'_j(t) - \left(e'_{j,1} \cdot C'_k\right) \times e'_{j,1}(t)$. $\mu'_j(t)$ est la moyenne des cinétiques $C'_k(t)$ au sein de la boule $B_{j,r}$ et $e'_{j,1}$ le vecteur propre associé à la première valeur propre d'une ACP calculée sur les cinétiques $C'_k(t)$ au sein de $B_{j,r}$ et centrée en $\mu'_j(t)$.

➢ *Classement des cinétiques :*

Soit le modèle $M''_{i,j}$ définit sur les cinétiques $C''_{i,j}(t) = C'_j(t)\Big/\sqrt{\mu'_i(t)} = C_j(t) \times \Delta_t \Big/\sqrt{\mu'_i(t)}$ au sein de l'échantillon $i$. $M''_{i,j}$ décrit toujours une droite dans l'espace des cinétiques lorsque $j$ varie au sein de $i$. L'erreur de reconstruction moyenne au sein de l'échantillon $i$ de la cinétique $C''_j(t)$ par le modèle $M''_{i,j}$ suit une loi stationnaire pour $j$ appartenant à $i$.

Une fois les modèles $M''_{i,j}$ calculés à partir des cinétiques $C''_{i,j}$ au sein de chaque échantillon, la segmentation de l'image revient à trouver, pour tout élément de volume $k$, l'échantillon $i$ qui minimise $\left\|C''_{i,k} - M''_{i,k}\right\|^2$.

On attribue donc à l'élément de volume $k$ le label $o$ tel que :

$$o = \arg\min_i \left\|C''_{i,k} - M''_{i,k}\right\|^2 \,.$$

➢ *Fusion des échantillons :*

La variance du bruit associé aux cinétiques $C''_{i,j}(t) = C_j(t) \times \Delta_t \big/ \sqrt{\mu'_i(t)}$ , pour l'élément de volume *j* inclus dans l'échantillon *i*, et égal à $\alpha'^2$, stationnaire dans l'espace comme dans le temps.

Soit $\lambda''^2_i = \dfrac{1}{T-1} \sum_{l=2}^{T} \lambda''^2_{i,l}$ la moyenne des T-1 plus petites valeurs propres d'une ACP calculée sur les cinétiques $C''_{i,k}(t)$ au sein de l'échantillon *i*. $\alpha'^2$ peut être estimée comme la moyenne des $\lambda''^2_i$ sur les échantillons :

$$\alpha'^2 = \frac{1}{I} \sum_{i=2}^{I} \lambda''^2_i \;.$$

La fonction de coût de fusion de deux échantillons a et *b* devient :

$$C_{a,b} = \beta \times \max\left[ \frac{1}{N} \times \sum_{k \in a} \frac{\|\varepsilon''_{b,k}\|^2}{\alpha'^2}, \frac{1}{N} \times \sum_{k \in b} \frac{\|\varepsilon''_{a,k}\|^2}{\alpha'^2} \right]$$

$$+ \delta \times \left(1 - \min\left[\|\mu_a\|_\infty / \|\mu_b\|_\infty, \|\mu_b\|_\infty / \|\mu_a\|_\infty\right]\right) + \gamma \times \left(1 - \min\left[\frac{t\max_b}{t\max_a}, \frac{t\max_a}{t\max_b}\right]\right)$$

## Revendications

**1.** Méthode de segmentation d'une image ou séquence d'images tridimensionnelles de départ à base de voxels pour l'obtention d'une image tridimensionnelle de segmentation comportant une partition en régions d'intérêt, ladite image ou séquence d'images comprenant des mesures, pour chaque voxel et au cours de n intervalle(s) de temps (n ≥ 1), de l'évolution réelle d'un signal représentatif d'au moins une variable de type physique, chimique ou biologique de ladite image ou séquence, **caractérisée en ce qu'**elle comprend les étapes suivantes :

a) une modélisation (10) du signal comprenant la définition d'un modèle paramétrique d'évolution spatio-temporelle dudit signal, ce modèle comprenant des jeux de paramètres homogènes de sorte que lesdits jeux soient respectivement propres à des structures correspondant auxdites régions d'intérêts et que chaque jeu de paramètres soit indépendant des coordonnées spatiales dans la structure correspondante ;
b) une extraction (30) d'échantillons de voxels de sorte que lesdits échantillons soient respectivement inclus dans lesdites structures, cette extraction comprenant :

(i) un calcul, pour chaque voxel de l'image ou séquence d'images de départ ou bien d'une zone d'intérêt de celle-ci, d'un critère de validité d'une hypothèse selon laquelle le modèle d'évolution de la variable au sein du voisinage de ce voxel est propre à une et une seule des structures,
(ii) une extraction de voxels-modèles qui sont définis comme réalisant les maxima locaux dudit critère,
(iii) une définition, pour chaque voxel-modèle, de l'un des échantillons de voxels inclus dans la structure correspondante, de manière à ce que cet échantillon présente une évolution de la variable qui soit conforme à celle du modèle de la structure à laquelle appartient le voxel-modèle, puis
(iv) une estimation, sur chaque échantillon ainsi défini, des paramètres dudit modèle d'évolution de la structure dans laquelle ledit échantillon est inclus ; puis

c) une fusion desdits échantillons regroupant les échantillons dont le modèle d'évolution est propre à la même structure, ladite fusion suivant, précédant ou incluant une classification de tous les voxels de ladite image ou séquence d'images ou d'une zone d'intérêt de celle-ci par agrégation à un groupe d'échantillons, de manière qu'une similarité maximale existe entre l'évolution de ladite variable pour ces voxels et l'évolution dudit modèle caractérisant ce groupe d'échantillons.

**2.** Méthode de segmentation selon la revendication 1, **caractérisée en ce que** l'on admet qu'il existe dans chaque image de départ de ladite séquence une partition de l'espace en un nombre fini desdites structures, lesquelles sont chacune connexes et présentent chacune en leur sein un comportement homogène en réponse à un phénomène étudié dont l'évolution de ladite variable est représentative.

**3.** Méthode de segmentation selon la revendication 2, **caractérisée en ce que** l'on détermine le nombre desdites structures a *posteriori.*

**4.** Méthode de segmentation selon une des revendications précédentes, **caractérisée en ce que** ledit modèle comprend en outre des paramètres hétérogènes dépendant des coordonnées spatiales des voxels au sein d'une même structure, et **en ce que** l'on admet pour l'étape a) que lesdits paramètres homogènes et hétérogènes peuvent être estimés sur un ou plusieurs éléments de volume inclus dans cette même structure.

**5.** Méthode de segmentation selon la revendication 4, **caractérisée en ce que** l'on admet en outre pour l'étape a) que lesdites structures présentent entre elles des réponses différentes à un phénomène étudié dont l'évolution de ladite variable est représentative, à moins qu'elles ne soient pas connexes.

**6.** Méthode de segmentation selon la revendication 5, **caractérisée en ce que** l'on admet en outre pour l'étape a) que le bruit, qui est inhérent auxdites mesures et qui est dû au mode d'acquisition de ladite séquence d'images de départ et de segmentation, est additif.

**7.** Méthode de segmentation selon la revendication 6 **caractérisée en ce que** l'on se fixe en outre pour l'étape a) les deux contraintes suivantes, pour prendre en compte des mélanges locaux de différentes évolutions temporelles dudit signal :

   (i) si la totalité des voxels qui sont voisins d'un voxel et qui contribuent à l'évolution de ladite variable relative à ce voxel est incluse dans la même structure, alors ledit signal correspondant est une réalisation du modèle de cette structure, et
   (ii) ledit jeu de paramètres homogènes pour ces voxels voisins est le même que celui dudit modèle propre à cette structure.

**8.** Méthode de segmentation selon la revendication 2, **caractérisée en ce que**, dans le cas où ladite image ou séquence d'images de départ est obtenue par une technique d'imagerie par injection de traceur, ledit modèle utilisé à l'étape a) est un modèle compartimental de type à un ou plusieurs traceurs indépendants et à plusieurs compartiments, tels que des compartiments biologiques.

**9.** Méthode de segmentation selon une des revendications précédentes, **caractérisée en ce que** l'étape b) d'extraction des voxels-modèles comprend une sélection préalable, dans un espace à n dimension(s) (n $\geq$ 1) correspondant à ladite image ou séquence d'images de départ, de voxels-modèles pour que chacune desdites structures d'intérêt contienne en son coeur au moins un voxel-modèle ainsi que le voisinage de celui-ci.

**10.** Méthode de segmentation selon la revendication 9, **caractérisée en ce que** l'étape b) comprend, suite à ladite sélection préalable, la définition d'une métrique prévue pour définir les distances dans ledit espace et ledit critère d'extraction local.

**11.** Méthode de segmentation selon la revendication 9, **caractérisée en ce que** l'on admet à l'étape b) que pour toute structure à segmenter, il existe un voxel dont le voisinage est compris au sein de la région d'intérêt correspondante.

**12.** Méthode de segmentation selon une des revendications précédentes, **caractérisée en ce que** l'étape b) comprend :

   - une détermination, pour chaque structure d'intérêt, d'un échantillon de voxels appartenant à cette structure, cette appartenance étant déterminée par une similitude de l'évolution de ladite variable avec le modèle relatif à cette structure, puis
   - une estimation, pour chaque échantillon, des paramètres homogènes du modèle qui correspondent à ladite structure.

**13.** Méthode de segmentation selon une des revendications précédentes, **caractérisée en ce qu'**il comprend en outre une étape de pré-segmentation (20) qui est mise en oeuvre avant ou après l'étape a) et qui consiste à séparer lesdites images de départ en une première partie contenant lesdites structures d'intérêt et en une seconde partie correspondant à un fond d'image exclu de la segmentation.

14. Méthode de segmentation selon une des revendications précédentes, **caractérisée en ce que** l'étape c) de fusion (50b) et de classification (50a) comprend une fusion desdits échantillons correspondant à une même structure d'intérêt, avant ou après ladite classification des voxels.

15. Méthode de segmentation selon la revendication 9, **caractérisée en ce que** la fusion desdits échantillons correspondant à une même structure d'intérêt est opérée par une classification ascendante hiérarchique.

16. Méthode de segmentation selon une des revendications précédentes, **caractérisée en ce qu'**elle comprend en outre une étape d'optimisation (40) des échantillons qui est mise en oeuvre suite à l'étape b) et avant l'étape c) et qui consiste à rechercher, pour chaque échantillon, une forme géométrique, paramétrique ou libre qui minimise en son sein le signal d'autres structures que celle dont le voxel-modèle a été extrait.

17. Méthode de segmentation selon une des revendications précédentes, **caractérisée en ce qu'**elle comprend en outre, suite à l'étape c), une étape de séparation (70) des structures non connexes qui présentent des évolutions similaires de ladite variable.

18. Méthode de segmentation selon la revendication 17, **caractérisée en ce qu'**elle comprend en outre une étape (60) de représentation hiérarchique desdites régions d'intérêt obtenues qui est mise en oeuvre suite à l'étape c) et avant ladite étape de séparation des structures non connexes.

19. Méthode de segmentation selon une des revendications précédentes, **caractérisée en ce qu'**elle exclue la bordure de chaque structure de l'extraction desdits échantillons mise en oeuvre à l'étape b).

20. Méthode de segmentation selon une des revendications précédentes, **caractérisée en ce que** ladite image ou séquence d'images est obtenue par une technique d'imagerie choisie dans le groupe constitué par la tomographie par émission de positons, l'imagerie par résonance magnétique, la tomographie d'émission de photons, les imageries optiques, le scanner aux rayons X, l'imagerie histologique, l'imagerie autoradiographique, l'imagerie satellitaire et l'imagerie photographique.

21. Méthode de segmentation selon une des revendications précédentes, **caractérisée en ce que** ladite image ou séquence d'images est obtenue par la technique de tomographie par émission de positons.

22. Méthode de segmentation selon la revendication 21, **caractérisée en ce que** l'on met en oeuvre à l'étape b) des analyses en composantes principales locales dans un espace à n dimensions pour déterminer, en fonction des valeurs propres et des vecteurs propres obtenus, les voxels qui présentent une évolution temporelle similaire de ladite variable.

23. Méthode de segmentation selon une des revendications précédentes, **caractérisée en ce que** lesdites images de départ sont des images d'un organisme entier, ladite méthode de segmentation segmentant ledit organisme selon une partition en pharmaco-organes.

24. Méthode de segmentation selon la revendication 23, **caractérisée en ce que** ledit corps est animé de mouvements physiologiques soit de type périodique, dont la période est réduite en comparaison de la durée d'acquisition de l'une des images de départ de ladite séquence, soit de type non périodique.

25. Méthode de segmentation selon la revendication 23 ou 24, **caractérisée en ce que** ladite variable représente la concentration radioactive en un instant donné $t_0$ à $t_n$ d'au moins un principe actif marqué et injecté dans ledit organisme, l'ensemble des voxels à l'intérieur de chaque pharmaco-organe présentant des cinétiques pharmacologiques de répartition dudit principe actif qui sont similaires.

**Patentansprüche**

1. Verfahren zur Segmentierung eines Bilds oder einer Ausgangssequenz dreidimensionaler Bilder auf der Basis von Voxeln zwecks Erhalts eines dreidimensionalen Segmentierungsbilds, umfassend eine Partition in Interessensregionen, wobei das Bild oder die Bildersequenz Messungen für jedes Voxel und während n Zeitintervallen (n ≥ 1) der tatsächlichen Entwicklung eines repräsentativen Signals mindestens einer Variablen physikalischen, chemischen oder biologischen Typs des Bilds oder der Sequenz umfasst, **dadurch gekennzeichnet, dass** sie die folgenden Schritte umfasst:

a) eine Modellierung (10) des Signals, umfassend die Definition eines parametrischen Modells der Raumzeitentwicklung des Signals, wobei dieses Modell Sets homogener Parameter derart umfasst, dass die Sets jeweils Strukturen zugeordnet sind, die den Interessensregionen entsprechen und dass jedes Parameterset unabhängig von den Raumkoordinaten in der entsprechenden Struktur ist,

b) eine Extraktion (30) von Proben von Voxeln derart, dass die Proben jeweils in den Strukturen integriert sind, wobei diese Extraktion umfasst:

(i) eine Berechnung für jedes Voxel des Bilds oder der Ausgangsbildersequenz oder einer Interessenszone derselben eines Gültigkeitskriteriums einer Hypothese, der gemäß das Evolutionsmodell der Variablen in der Nachbarschaft dieses Voxels zu einer und nur zu einer der Strukturen gehört,
(ii) eine Extraktion von Voxelmodellen, die als Realisator der lokalen Maxima des Kriteriums definiert sind,
(iii) eine Definition, für jedes Voxelmodell, einer der Voxelproben, die in der entsprechenden Struktur integriert sind, damit diese Probe eine Entwicklung der Variablen aufweist, die der des Modells der Struktur entspricht, der das Voxelmodell angehört, danach
(iv) eine Ermittlung, für jede derart definierte Probe, der Parameter des Evolutionsmodells der Struktur, in welche die Probe integriert ist; danach

c) eine Fusion der Proben, die die Proben zusammenführt, deren Evolutionsmodell derselben Struktur angehört, wobei der Fusion eine Klassifizierung aller Voxel des Bilds oder der Bildersequenz oder einer Interessenszone derselben durch Aggregation in eine Probengruppe derart folgt, vorausgeht oder integriert ist, dass eine maximale Ähnlichkeit zwischen der Entwicklung der Variablen für diese Voxel und der Entwicklung des Modells, das diese Probengruppe kennzeichnet, existiert.

2. Segmentierungsverfahren nach Anspruch 1, **dadurch gekennzeichnet, dass** davon ausgegangen wird, dass es in jedem Ausgangsbild der Sequenz eine Partition des Raums in eine endliche Zahl der Strukturen gibt, die alle verbunden sind und alle ein gleichartiges Verhalten als Antwort auf ein untersuchtes Phänomen aufweisen, für dessen Entwicklung die Variable repräsentativ ist.

3. Segmentierungsverfahren nach Anspruch 2, **dadurch gekennzeichnet, dass** die Anzahl der Strukturen im Nachhinein bestimmt wird.

4. Segmentierungsverfahren nach einem der vorangehenden Ansprüche, **dadurch gekennzeichnet, dass** das Modell ferner heterogene Parameter umfasst, die von den Raumkoordinaten der Voxel innerhalb einer selben Struktur abhängen, und dass für Schritt a) davon ausgegangen wird, dass die gleichartigen und verschiedenartigen Parameter für ein oder mehrere Volumenelemente bestimmbar sind, die in dieser selben Struktur integriert sind.

5. Segmentierungsverfahren nach Anspruch 4, **dadurch gekennzeichnet, dass** ferner für Schritt a) davon ausgegangen wird, dass die Strukturen untereinander unterschiedliche Antworten auf ein untersuchtes Phänomen aufweisen, für dessen Entwicklung die Variable repräsentativ ist, sofern sie nicht verbunden sind.

6. Segmentierungsverfahren nach Anspruch 5, **dadurch gekennzeichnet, dass** ferner für Schritt a) davon ausgegangen wird, dass das Rauschen, das den Messungen inhärent ist und das dem Erfassungsmodus der Ausgangsbilder- und Segmentierungssequenz geschuldet ist, additiv ist.

7. Segmentierungsverfahren nach Anspruch 6 **dadurch gekennzeichnet, dass** ferner für Schritt a) die beiden folgenden Bedingungen festgelegt werden, um die lokalen Mischungen verschiedener temporärer Entwicklungen des Signals zu berücksichtigen:

(i) wenn die Gesamtheit der Voxel, die einem Voxel benachbart sind und die zur Entwicklung der Variablen relativ zu diesem Voxel beitragen, in dieselbe Struktur integriert ist, ist das entsprechende Signal eine Realisierung des Modells dieser Struktur, und
(ii) das Set homogener Parameter für diese benachbarten Voxel ist dasselbe wie das des Modells, das zu dieser Struktur gehört.

**8.** Segmentierungsverfahren nach Anspruch 2, **dadurch gekennzeichnet, dass**, wenn das Bild oder die Ausgangsbildersequenz anhand einer Bildgebungstechnologie mit Tracerinjektion erhalten wird, das in Schritt a) verwendete Modell ein Kompartimentsmodell vom Typ mit einem oder mehreren unabhängigen Tracern und mit mehreren Kompartimenten, wie beispielsweise biologischen Kompartimenten, ist.

**9.** Segmentierungsverfahren nach einem der vorangehenden Ansprüche, **dadurch gekennzeichnet, dass** Schritt b) der Extraktion von Voxelmodellen eine vorherige Selektion in einem Raum mit n Dimensionen ($n \geq 1$) entsprechend dem Bild oder der Ausgangsbildersequenz von Voxelmodellen umfasst, damit jede der Interessensstrukturen in ihrem Kern mindestens ein Voxelmodell sowie die Nachbarschaft desselben enthält.

**10.** Segmentierungsverfahren nach Anspruch 9, **dadurch gekennzeichnet, dass** Schritt b) nach der vorherigen Selektion die Definition einer Metrik umfasst, die vorgesehen ist, um die Abstände im Raum und das lokale Extraktionskriterium zu definieren.

**11.** Segmentierungsverfahren nach Anspruch 9, **dadurch gekennzeichnet, dass** für Schritt b) davon ausgegangen wird, dass es für jede zu segmentierende Struktur ein Voxel gibt, dessen Nachbarschaft in der entsprechenden Interessensregion integriert ist.

**12.** Segmentierungsverfahren nach einem der vorangehenden Ansprüche, **dadurch gekennzeichnet, dass** Schritt b) umfasst:

- eine Bestimmung, für jede Interessensstruktur, einer Voxelprobe, die zu dieser Struktur gehört, wobei diese Zugehörigkeit mittels einer Ähnlichkeit der Entwicklung der Variablen mit dem Modell relativ zu dieser Struktur bestimmt wird, danach
- eine Ermittlung, für jede Probe, der gleichartigen Parameter des Modells, die der Struktur entsprechen.

**13.** Segmentierungsverfahren nach einem der vorangehenden Ansprüche, **dadurch gekennzeichnet, dass** es ferner einen Vorsegmentierungsschritt (20) umfasst, der vor oder nach Schritt a) durchgeführt wird und der darin besteht, die Ausgangsbilder in einen ersten Teil, der die Interessensstrukturen enthält, und in einen zweiten Teil, der einem von der Segmentierung ausgeschlossenen Bildhintergrund entspricht, zu separieren.

**14.** Segmentierungsverfahren nach einem der vorangehenden Ansprüche, **dadurch gekennzeichnet, dass** Schritt c) der Fusion (50b) und der Klassifizierung (50a) eine Fusion der Proben umfasst, die einer selben Interessensstruktur angehören, vor oder nach der Klassifizierung der Voxel.

**15.** Segmentierungsverfahren nach Anspruch 9, **dadurch gekennzeichnet, dass** die Fusion der Proben, die einer selben Interessensstruktur angehören, mittels einer aufsteigenden hierarchischen Klassifizierung erfolgt.

**16.** Segmentierungsverfahren nach einem der vorangehenden Ansprüche, **dadurch gekennzeichnet, dass** es ferner einen Optimierungsschritt (40) der Proben umfasst, der nach Schritt b) und vor Schritt c) durchgeführt wird und der darin besteht, für jede Probe eine geometrische, parametrische oder freie Form zu suchen, die in ihr das Signal anderer Strukturen als der, aus der das Voxelmodell extrahiert wurde, minimiert.

**17.** Segmentierungsverfahren nach einem der vorangehenden Ansprüche, **dadurch gekennzeichnet, dass** es ferner nach Schritt c) eine Separationsschritt (70) der nicht verbundenen Strukturen umfasst, die ähnliche Entwicklungen der Variablen aufweisen.

**18.** Segmentierungsverfahren nach Anspruch 17, **dadurch gekennzeichnet, dass** es ferner einen hierarchischen Repräsentationsschritt (60) der erhaltenen Interessensregionen umfasst, der nach Schritt c) und vor dem Separationsschritt der nicht verbundenen Strukturen durchgeführt wird.

**19.** Segmentierungsverfahren nach einem der vorangehenden Ansprüche, **dadurch gekennzeichnet, dass** es den Rand jeder Struktur von der Extraktion der Proben, umgesetzt in Schritt b), ausschließt.

**20.** Segmentierungsverfahren nach einem der vorangehenden Ansprüche, **dadurch gekennzeichnet, dass** das Bild oder die Bildersequenz mittels einer Bildgebungstechnik erhalten wird, die aus der Gruppe ausgewählt ist, die von der Positonen-Emissions-Tomographie, der Magnetresonanztomographie, der Photonen-Emissions-Tomographie, den optischen Bildgebungen, dem Röntgenstrahlenscanner, der histologischen Bildgebung, der autoradiographischen Bildgebung, der Satellitenbildgebung und der fotografischen Bildgebung gebildet ist.

21. Segmentierungsverfahren nach einem der vorangehenden Ansprüche, **dadurch gekennzeichnet, dass** das Bild oder die Bildersequenz mittels der Positonen-Emissions-Tomographietechnik erhalten wird.

22. Segmentierungsverfahren nach Anspruch 21, **dadurch gekennzeichnet, dass** in Schritt b) Analysen lokaler Hauptkomponenten in einem Raum mit n Dimensionen durchgeführt werden, um in Abhängigkeit der Eigenwerte und der erhaltenen Eigenwerte die Voxel zu bestimmen, die eine temporäre Entwicklung aufweisen, die der Variablen ähnlich ist.

23. Segmentierungsverfahren nach einem der vorangehenden Ansprüche, **dadurch gekennzeichnet, dass** die Ausgangsbilder Bilder eines gesamten Organismus sind, wobei das Segmentierungsverfahren den Organismus gemäß einer Partition in Pharmakoorgane segmentiert.

24. Segmentierungsverfahren nach Anspruch 23, **dadurch gekennzeichnet, dass** der Körper von physiologischen Bewegungen entweder periodischen Typs, wobei die Periode im Vergleich mit der Dauer der Erfassung eines der Ausgangsbilder der Sequenz reduziert ist, oder nicht periodischen Typs animiert ist.

25. Segmentierungsverfahren nach Anspruch 23 oder 24, **dadurch gekennzeichnet, dass** die Variable die radioaktive Konzentration zu einem bestimmten Moment $t_0$ bis $t_n$ mindestens eines markierten und in den Organismus injizierten Wirkstoffs darstellt, wobei die Gesamtheit der Voxel im Innern jedes Pharmakoorgans pharmakologische Verteilungskinetiken des Wirkstoffs aufweist, die ähnlich sind.

**Claims**

1. A method of segmenting a starting image or sequence of tridimensional images based on voxels for obtaining a tridimensional segmentation image comprising a partition into regions of interest, said image or sequence of images comprising measurements, for each voxel and in the course of n time interval(s) (n ≥ 1), of the real evolution of a signal representative of at least one variable of physical, chemical or biological type of said image or sequence, which comprises the following steps:

   a) a modeling (10) of the signal comprising the definition of a parametric model of spatio-temporal evolution of said signal, this model comprising sets of homogeneous parameters so that said sets are respectively specific to structures corresponding to said regions of interest and that each set of parameters is independent of the spatial coordinates in the corresponding structure;
   b) an extraction (30) of samples of voxels so that said samples are respectively included in said structures, this extraction comprising:

      (i) a calculation, for each voxel of the starting image or sequence of images or else of a zone of interest of the latter, of a criterion of validity of a hypothesis according to which the model of evolution of the variable within the neighborhood of this voxel is specific to one and only one of the structures,
      (ii) an extraction of model-voxels which are defined as realizing the local maxima of said criterion,
      (iii) a definition, for each model-voxel, of one of the samples of voxels included in the corresponding structure, in such a way that this sample exhibits an evolution of the variable which complies with that of the model of the structure to which the model-voxel belongs, then
      (iv) an estimation, on each sample thus defined, of the parameters of said model of evolution of the structure in which said sample is included; then

   c) a merging of said samples grouping together the samples whose evolution model is specific to the same structure, said merging following, preceding or including a classification of all the voxels of said image or sequence of images or of a zone of interest of the latter by aggregation with a group of samples, in such a manner that a maximum similarity exists between the evolution of said variable for these voxels and the evolution of said model characterizing this group of samples.

2. The segmentation method as claimed in claim 1, wherein it is granted that there exists in each starting image of said sequence a partition of the space into a finite number of said structures, which are each connected and each exhibit within them a homogeneous behavior in response to a phenomenon studied of which the evolution of said variable is representative.

3. The segmentation method as claimed in claim 2, wherein the number of said structures is determined *a posteriori.*

4. The segmentation method as claimed in one of the preceding claims, wherein said model furthermore comprises heterogeneous parameters dependent on the spatial coordinates of the voxels within one and the same structure, and wherein it is granted for step a) that said homogeneous and heterogeneous parameters can be estimated on one or more volume elements included in this same structure.

5. The segmentation method as claimed in claim 4, wherein it is furthermore granted for step a) that said structures together exhibit different responses to a phenomenon studied of which the evolution of said variable is representative, unless they are not connected.

6. The segmentation method as claimed in claim 5, wherein it is furthermore granted for step a) that the noise, which is inherent in said measurements and which is due to the mode of acquisition of said sequence of starting and segmentation images, is additive.

7. The segmentation method as claimed in claim 6 wherein for step a) the following two constraints are furthermore fixed, so as to take into account local mixtures of various temporal evolutions of said signal:

(i) if the totality of the voxels which are neighbors of a voxel and which contribute to the evolution of said variable relating to this voxel is included in the same structure, then said corresponding signal is a realization of the model of this structure, and
(ii) said set of homogeneous parameters for these neighbor voxels is the same as that of said model specific to this structure.

8. The segmentation method as claimed in claim 2, wherein, in the case where said starting image or sequence of images is obtained by an imaging technique by tracer injection, said model used in step a) is a compartmental model of type with one or more independent tracers and with several compartments, such as biological compartments.

9. The segmentation method as claimed in one of the preceding claims, wherein step b) of extraction of the model-voxels comprises a prior selection, in a space with n dimensions ($n \geq 1$) corresponding to said starting image or sequence of images, of model-voxels so that each of said structures of interest contains at its heart at least one model-voxel as well as the neighborhood of the latter.

10. The segmentation method as claimed in claim 9, wherein step b) comprises, following said prior selection, the definition of a metric designed to define the distances in said space and said local extraction criterion.

11. The segmentation method as claimed in claim 9, wherein it is granted in step b) that for any structure to be segmented, there exists a voxel whose neighborhood is included within the corresponding region of interest.

12. The segmentation method as claimed in one of the preceding claims, wherein step b) comprises:

- a determination, for each structure of interest, of a sample of member voxels belonging to this structure, this membership being determined by a similitude of the evolution of said variable with the model relating to this structure, then
- an estimation, for each sample, of the homogeneous parameters of the model which correspond to said structure.

13. The segmentation method as claimed in one of the preceding claims, wherein it furthermore comprises a presegmentation step (20) which is implemented before or after step a) and which consists in separating said starting images into a first part containing said structures of interest and into a second part corresponding to an image background excluded from the segmentation.

14. The segmentation method as claimed in one of the preceding claims, wherein step c) of merging (50b) and of classification (50a) comprises a merging of said samples corresponding to one and the same structure of interest, before or after said classification of the voxels.

**15.** The segmentation method as claimed in claim 9, wherein the merging of said samples corresponding to one and the same structure of interest is operated by a hierarchical ascending classification.

**16.** The segmentation method as claimed in one of the preceding claims, wherein it furthermore comprises a step of optimization (40) of the samples which is implemented following step b) and before step c) and which consists in seeking, for each sample, a geometric, parametric or free form which minimizes within it the signal of structures other than that whose model-voxel has been extracted.

**17.** The segmentation method as claimed in one of the preceding claims, wherein it furthermore comprises, following step c), a step of separation (70) of the nonconnected structures which exhibit similar evolutions of said variable.

**18.** The segmentation method as claimed in claim 17, wherein it furthermore comprises a step (60) of hierarchical representation of said regions of interest obtained which is implemented following step c) and before said step of separation of the nonconnected structures.

**19.** The segmentation method as claimed in one of the preceding claims, wherein it excludes the boundary of each structure from the extraction of said samples implemented in step b).

**20.** The segmentation method as claimed in one of the preceding claims, wherein said image or sequence of images is obtained by an imaging technique chosen from the group consisting of positon emission tomography, magnetic resonance imaging, photon emission tomography, optical imagings, X-ray scanner, histological imaging, autoradiographic imaging, satellite imaging and photographic imaging.

**21.** The segmentation method as claimed in one of the preceding claims, wherein said image or sequence of images is obtained by the technique of positon emission tomography.

**22.** The segmentation method as claimed in claim 21, wherein in step b), local principal component analyses in a space with n dimensions are implemented to determine, as a function of the eigenvalues and of the eigenvectors obtained, the voxels which exhibit a similar temporal evolution of said variable.

**23.** The segmentation method as claimed in one of the preceding claims, wherein said starting images are images of a whole organism, said segmentation method segmenting said organism according to a partition into pharmaco-organs.

**24.** The segmentation method as claimed in claim 23, wherein said body is imbued with physiological motions either of periodic type, whose period is reduced in comparison with the duration of acquisition of one of the starting images of said sequence, or of nonperiodic type.

**25.** The segmentation method as claimed in claim 23 or 24, wherein said variable represents the radioactive concentration at a given instant $t_0$ to $t_n$ of at least one marked active principle injected into said organism, the whole set of voxels inside each pharmaco-organ exhibiting pharmacological kinetics of distribution of said active principle which are similar.

| 10 |
|----|

| 20 |
|----|

| 30 |
|----|

| 40 |
|----|

| 50a | | 50b |

| 60 |
|----|

| 70 |
|----|

# Fig. 1

— Cinétique tissulaire vessie

˙˙ Famille cinétiques non physiologiques vessie

···· Cinétique tissulaire muscle

# Fig. 2

# Fig. 3a

# Fig. 3b

Temps

# Fig. 4

Temps

# Fig. 5

EP 1 899 914 B1

**Fig. 6a**

**Fig. 6b**

**Fig. 7a**

**Fig. 7b**

**Fig. 8a**

**Fig. 8b**

**Fig. 9a**

**Fig. 9b**

Fig. 10a            Fig. 10b

**Fig. 11a**          **Fig. 11b**

coût de fusion

échantillons

## Fig. 12

## Fig. 13a   Fig. 13b   Fig. 13c   Fig. 13d

**Fig. 14a**  **Fig. 14b**  **Fig. 14c**

**Fig. 15**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1365356 A

**Littérature non-brevet citée dans la description**

- A cluster analysis approach for the characterisation of dynamic PET data. **ASHBURNER.** Quantification of Brain Function using PET. Academic Press, 1996, 301-306
- Automatic segmentation of dynamic neuroreceptor single-photon emission tomography images using fuzzy clustering. **ACTON, P.D. ; PILOWSKY, L.S. ; KUNG, H.F. ; ELL, P.J.** European Journal of Nuclear Medecine and Molecular Imaging. Springer-Verlag, 1999, vol. 26, 581-590
- **BRANKOV, J.G. ; GALATSANOS, N.P. ; YONGYI YANG ; WERNICK, M.N.** Segmentation of dynamic PET or fMRI images based on a similarity metric. *IEEE Transactions on Nuclear Science,* 2003, vol. 50 (5), 1410-1414
- **GUO, H. ; RENAUT, R. ; CHEN, K. ; REIMAN, E.** Clustering huge data sets for parametric PET imaging. *BioSystem,* 2003, vol. 71, 81-92
- 3D regularisation and segmentation of factor volumes to process PET H215O myocardial perfusion studies. Functional Imaging and Modelling of the Heart (FIMH'2001). **FROUIN, F. ; BOUBACAR, P. ; FROUIN, V. ; DE CESARE, A. ; TODD-POKROPEK, A. ; MERLET, P. ; HERMENT, A.** Lecture Notes in Computer Sciences. Springer Verlag, 2001, vol. 2230, 91-96
- **FROUIN, V. ; COMTAT, C. ; REILHAC, A. ; GRÉGOIRE, M.-C.** Correction of Partial-Volume Effect for PET Striatal Imaging: Fast Implementation and Study of Robustness. *Journal of Nuclear Medicine,* 2002, vol. 43-12, 1715-1726
- **JAIN, A.K. ; DUBES, R.C.** Algorithm for Clustering Data. Advanced Reference. Prentice-Hall, 1988

- **KIMURA, Y. ; SENDA, M. ; ALPERT, N.** Fast formation of statistically reliable FDG parametric images based on clustering and principal components. *Phys. Med. Biol.,* 2002, vol. 47 (3), 455-468
- Robust Brain Segmentation Using Histogram Scale-Space Analysis and Mathematical Morphology. **MANGIN, J.-F. ; COULON, O. ; FROUIN V.** MICCAI. Springer Verlag, 1998, vol. 1496, 1230-1241
- **MINKA, T.P.** Automatic choice of dimensionality for PCA. *M.I.T. Media Laboratory Perceptual Computing Section Technical Report,* 2000, vol. 514
- **NICHOLS, T.E. ; QI, J. ; ASMA, E. ; LEAHY, R.M.** Spatiotemporal Reconstruction of List-Mode PET Data. *IEEE Transactions on Medical Imaging,* 2002, vol. 21 (4), 396-404
- **SEGARS, W.P. ; TSUI, B.M.W. ; FREY, E.C. ; JOHNSON, G.A. ; BERR, S.S.** Development of a 4D Digital Mouse Phantom for Molecular Imaging Research. *Molecular Imaging and Biology,* 2004
- **TIPPING, M.E. ; BISHOP, C.M.** Mixture of probabilistic principal component analysers. *Neural Computation,* 1999, vol. 11 (2), 443-482
- **WONG, K.-P. ; FENG, D. ; MEIKLE, S.R. ; FULHAM, M.J.** *2001. IEEE Transactions on Nuclear Science,* 2002, vol. 49, 200-207
- **ZHOU, Y. ; HUANG, S.-C. ; BERGSNEIDER, M. ; WONG, D.F.** A non linear regression with spatial constraint for generation of parametric images in dynamic PET studies. *Journal of Nuclear Medecine,* vol. 42 (5), 100